# EUROPÄISCHE PATENTSCHRIFT

(11) **EP 0 282 452 B1**
(45) Veröffentlichungstag und Bekanntmachung des Hinweises auf die Patenterteilung: **08.07.1992**
(21) Anmeldenummer: 88810133.4
(22) Anmeldetag: 04.03.1988
(51) Int. Cl.: C07C 335/18, C07C 335/32, C07C 331/28, C07C 267/00, C07C 217/90, A01N 47/30, A01N 47/40, A01N 47/42

(54) **Phenoxyphenyl-thioharnstoffe, -isothioharnstoffe und -carbodiimide und ihre Verwendung bei der Kontrolle von Schädlingen**
Phenoxyphenyl thioureas, ditto isothioureas and ditto carbodiimides and their use in pest control
Phénoxyphényl-thiourées, isothiourées et carbodiimides et leur utilisation comme pesticide

(30) Priorität: 10.03.1987 CH 878/87; 11.01.1988 CH 65/88
(43) Veröffentlichungstag der Anmeldung: 14.09.1988
(73) Patentinhaber: CIBA-GEIGY AG, 4002 Basel (CH)
(72) Erfinder: Ehrenfreund, Josef, Dr., CH-4123 Allschwil (CH)

(56) Entgegenhaltungen:
- EP-A- 0 025 010
- EP-A- 0 175 649
- EP-A- 0 296 120
- DE-A- 3 801 395
- GB-A- 2 060 626

## Beschreibung

Die vorliegende Erfindung betrifft neue substituierte Phenoxyphenylthioharnstoffe, -isothioharnstoffe und -carbodiimide, ihre Salze mit organischen oder anorganischen Säuren, Verfahren und Zwischenprodukte zu ihrer Herstellung, Schädlingsbekämpfungsmittel, die diese Verbindungen enthalten, sowie ihre Verwendung in der Schädlingsbekämpfung.

Die erfindungsgemässen Verbindungen entsprechen der Formel I 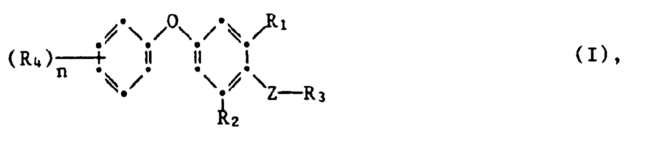
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle können geradkettig oder verzweigt sein. Als Beispiele solcherAlkyle seien Methyl, Aethyl, Propyl, Isopropyl sowie Butyl und Pentyl und ihre Isomeren genannt. Diese Ausführungen gelten sinngemäss auch für die Alkoxy-Substituenten.

Bei den als Substituenten in Betracht kommenden Cycloalkylen und Cycloalkenylen handelt es sich beispielsweise um Cyclopropyl, Cyclobutyl, Cyclopentyl, Cyclopentenyl, Cyclohexyl, Cyclohexenyl oder Cycloheptyl. Diese können auch ein- oder zweifach mit einem C₁-C₃-Alkylrest subsitutiert sein.

Die als Substituenten in Betracht kommenden Alkenyle können geradkettig oder verzweigt sein und eine oder mehrere Doppelbindungen aufweisen. Beispiele solcher Alkenyle sind u.a. Vinyl, Allyl, 1-Propenyl, Isopropenyl, Allenyl, Butenyle, Butadienyle oder Pentenyle.

Die Verbindungen der Formel in denen Z für -N=C(SR₅)-NH- steht, können auch in Form vom Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, lodwasserstoffsäure, Salpetersäure, verschiedene Phosphorsäuren, Schwefelsäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäuren oder Salicylsäure.

Verbindungen der Formel I, in denen Z für -N=C(SR₅)-NH- steht, können in den tautomeren Formen 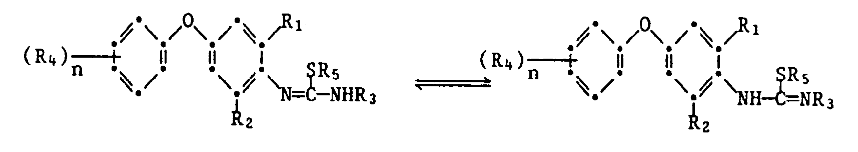 vorliegen. Die Erfindung umfasst sowohl die einzelnen Tautomeren, als auch Tautomerengemische.

Unter den Verbindungen der Formel stehen diejenigen im Vordergrund, in denen R₁ für C₃-C₇-Cycloalkyl, R₂ für C₁-C₄-Alkyl oder Cyclopentyl, R₃ für C₁-C₄-Alkyl oder C₃-C₅-Cycloalkyl, R₄ für Wasserstoff, Halogen oder C₁-C₃-Alkyl, n für die Zahlen 1 oder 2, Z für -NH-CS-NH, -N=C(SR₅)-NH- oder -N=C=N- und R₅ für C₁-C₃-Alkyl stehen.

Dabei sind diejenigen Verbindungen der Formel bevorzugt, in denen
a) R₁ für C₅-Cₛ-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für -NH-CS-NH- stehen oder
b) R₁ für C₅-Cₛ-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2, Z für -N=C(SR₅)-NH- und R₅ für Methyl oder Aethyl stehen oder
c) R₁ für C₅-Cₛ-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2, Z für -N=C=N- stehen.

Die erfindungsgemässen Verbindungen der Formel können nach im Prinzip bekannten Verfahren hergestellt werden, indem man z.B.

A) ein Isothiocyanat der Formel 11 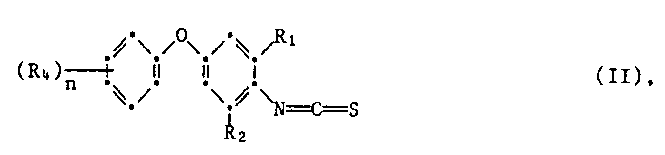 mit einem Amin der Formel III 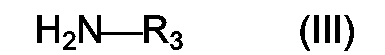 zum Thioharnstoff umsetzt und gegebenenfalls
B) den erhaltenen Thioharnstoff mit einer Verbindung der Formel IV 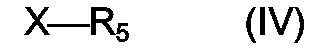 zum Isothioharnstoff umsetzt oder
C) den erhaltenen Thioharnstoff durch Abspalten von Schwefelwasserstoff in das Carbodiimid überführt. R_{1'} R₂, R₃, R₄, R₅ und n haben dabei die angegebene Bedeutung und X steht für eine geeignete Abgangsgruppe wie z.B. ein Halogenatom, insbesondere Chlor, Brom oder Jod, oder Alkylsulfat.

Das Verfahren Awird üblicherweise unter normalem Druck, und in Gegenwart eines organischen Lösungs-oder Verdünnungsmittels durchgeführt. Die Temperatur beträgt dabei zwischen 0 und +150_{°}C, vorzugsweise +10 bis 70°C. Als Lösungs-oder Verdünnungsmittel eignen sich z.B. Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Das Verfahren B wird zweckmässigerweise in einem inerten organischen Lösungsmittel und unter leicht erhöhtem oder normalem Druck durchgeführt. Die Temperatur beträgt dabei zwischen +10 und 250°C, vorzugsweise Siedetemperatur des verwendeten Lösungsmittels oder +50 bis 150°C. Geeignete Lösungs- oder Verdünnungsmittel sind z.B. Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid.

Das Verfahren C wird zweckmässigerweise in einem aprotischen organischen Lösungs- oder Verdünnungsmittel unter normalem Druck durchgeführt. Die Temperatur beträgt dabei zwischen 0 und +150_{°}C, vorzugsweise +10 bis 50°C. Geeignete Lösungs- oder Verdünnungsmittel sind z.B. Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon oder Ccylohexanon. Die Abspaltung von Schwefelwasserstoff erfolgt nach in der Literatur beschriebenen Arbeitsweisen (T. Shibanuma, Chemistry Letters 1977, p. 575-76; S. Kim, Tetrahedron Letters 1985, p. 1661-64; W. Weith, B. 6,1873, p. 1398; G. Amiard, Bull. Soc. chim. 1956, p. 1360). Als Abspaltungs-Reagenzien werden dabei u.a. HgO, bestimmte Pyridinium Salze, Chloressigsäureester, Cyanursäurechlorid, p-Toluolsulfochlorid oder bestimmte Phosphorsäureester-Derivate verwendet.

Die Isothiocyanate der Formel können nach im Prinzip bekannten Methoden hergestellt werden, z.B. indem man ein Phenoxyanilin der Formel V 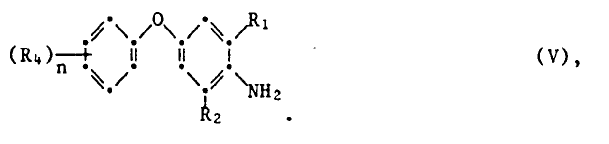 mit Thiophosgen umsetzt, wobei R_{1'} R₂, R₄ und n die für Formel angegebene Bedeutung haben.

Das Verfahren zur Herstellung der Verbindungen der Formel II wird zweckmässigerweise in Gegenwart einer organischen oder anorganischen Base, wie z.B. Triäthylamin oder Calciumcarbonat, und einem gegenüber den Reaktionsteilnehmern inerten Lösungs- oder Verdünnungsmittels unter normalem Druck durchgeführt. Die Temperatur beträgt dabei zwischen 0 und +100_{°}C, vorzugsweise Siedetemperatur des verwendeten Lösungs- oder Verdünnungsmittels oder +20 bis 80°C. Geeignete Lösungs- und Verdünnungsmittel sind u.a. Aether oder ätherartige Verbindungen, wie z.B. Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon; oder chlorierte Kohlenwasserstoffe wie z.B. Dichlormethan. Die Herstellung kann auch in Anwesenheit von Wasser im 2-Phasensystem erfolgen.

Die Phenoxyaniline der Formel V können nach im Prinzip bekannten Methoden hergestellt werden, z.B. indem man ein Anilin der Formel VI 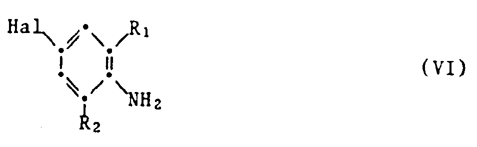 mit einem Phenol der Formel VII 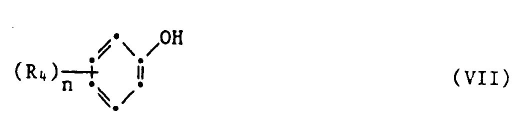 umsetzt, wobei R_{1'} R₂, R₄ und n die für Formel angegebene Bedeutung haben und Hal für Halogen, insbesondere für Chlor und Brom steht.

Das Verfahren zur Herstellung der Verbindungen der Formel V wird zweckmässigerweise in Gegenwart einer organischen oder insbesondere anorganischen Base, wie z.B. einem Alkalihydroxid oder -carbonat, und einem gegenüber den Reaktionsteilnehmern inerten, vorzugsweise polaren Lösungs- oder Verdünnungsmittels unter normalem Druck durchgeführt. Die Temperatur beträgt dabei 0 bis +200_{°}C, vorzugsweise Siedetemperaturdes verwendeten Lösungs-oder Verdünnungsmittels oder +50 bis 170°C. Als vorteilhaft kann sich auch der Zusatz eines Schwermetall-Katalysators wie z.B. Kupferpulver oder basisches Kupfer-II-carbonat erweisen. Geeignete Lösungs- und Verdünnungsmittel sind u.a. Amide wie Dimethylformamid, Dimethylsulfoxid, N-Methylpyrrolidon und andere aprotisch dipolare Lösungsmittel.

Die Verbindungen der Formeln II und V sind neu und bilden ebenfalls einen Gegenstand der vorliegenden Erfindung. Die Verbindungen der Formeln III, IV, VI (vgl. DE-OS 27 27 529; Synth. Communications Vol. 16/809, 1986) und VII sind dagegen bekannt oder können nach im Prinzip bekannten Methoden hergestellt werden.

Aus den DE-OS 26 39 748, 27 02 235, 27 27 416, 27 27 529 und 27 30 620 sind Phenylthio- und Phenylisothioharnstoffe bekannt, die in 2,6-Stellung des N-Phenylringes zwar durch Cycloalkyle substituiert sein können jedoch keine Phenoxygruppe aufweisen. Aus der DE-OS 30 34 905 und den EP-OS 025 010 und 175 649 sind Phenoxyphenylcarbodiimide, Phenoxyphenylthio- und Phenoxyphenylisothioharnstoffe bekannt, die in den Stellungen 2- und/oder 6- des N-Phenylringes keine Cycloalkylgruppen aufweisen. Alle diese bekannten Verbindungen sind pestizid wirksam zeichnen sich aber durch eine unbefreidigende Pflanzenverträglichkeit aus.

Ueberraschenderweise wurde gefunden, dass die erfindungsgemässen Verbindungen der Formel 1 bei günstigerWarmblüter-und Pflanzenverträglichkeitwertvolle Wirkstoffe in der Schädlingsbekämpfung sind. So eignen sich die Verbindungen der Formel z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina,wiez.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus-und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel bzw. Kombinationen dieser Wirkstoffe mit anderen Insektiziden oder Akariziden, und gegenbenenfalls einen festen oderflüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage Kommen: Aromatische Kohlenwasserstoffe, bevorzugt die Fraktionen C₈ bis C₁₂, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren (C_{1O}-C₂₂), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979;

Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel oder Kombinationen dieserWirkstoffe mitandern Insektiziden oderAkariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zu Erzielung spezieller Effekte enthalten.

### Beispiel 1: Herstellung

### 1.1. Zwischenprodukte

### 1.1.1. 4-Phenoxyaniline

### 1.1.1.1. 2-Cyclohexyl-4-phenoxy-6-isopropylanilin

5,7 g Phenol werden in 30 ml Toluol gelöst und mit 0,8 g gemahlenem Kaliumcarbonat und 6,8 g einer 50%igen wässrigen Lösung von Kaliumhydroxid versetzt. Während 5 Stunden wird das Wasser ausgekreist und dann das Toluol abdestilliert. Zum Rückstand werden 30 ml Dimethylformamid und 0,25 g basisches Kupfercarbonat gegeben, worauf so viel Lösungsmittel abdestilliert wird, bis die Innentemperatur 140°C erreicht hat. Bei dieser Temperatur werden 11,9 g 2-Cyclohexyl-4-brom-6-isopropylanilin zum Reaktionsgemisch zugetropft und 16 Stunden lang bei gleichbleibender Temperatur gerührt. Anschliessend wird das Lösungsmittle im Vacuum abdestilliert und der Rückstand mitAether verdünnt und filtriert. Die organische Phase wird zweimal mit 10%iger wässriger Natriumhydroxid-Lösung und Wasser gewaschen und über Natriumsulfat getrocknet. Das Lösungsmittel wird am Vacuum entfernt, worauf die Titelverbindung der Formel 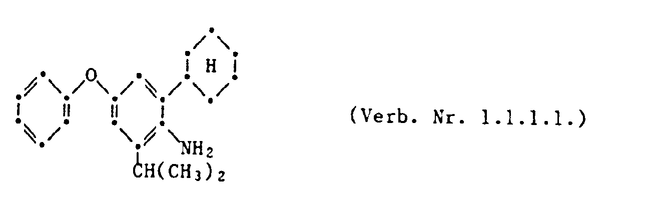 als dunkelbrauner Feststoff vorliegt. Nach Umkristallisation aus Hexan: Smp. 108-110°C.

Auf analoge Weise werden die folgenden Verbindungen hergestellt.

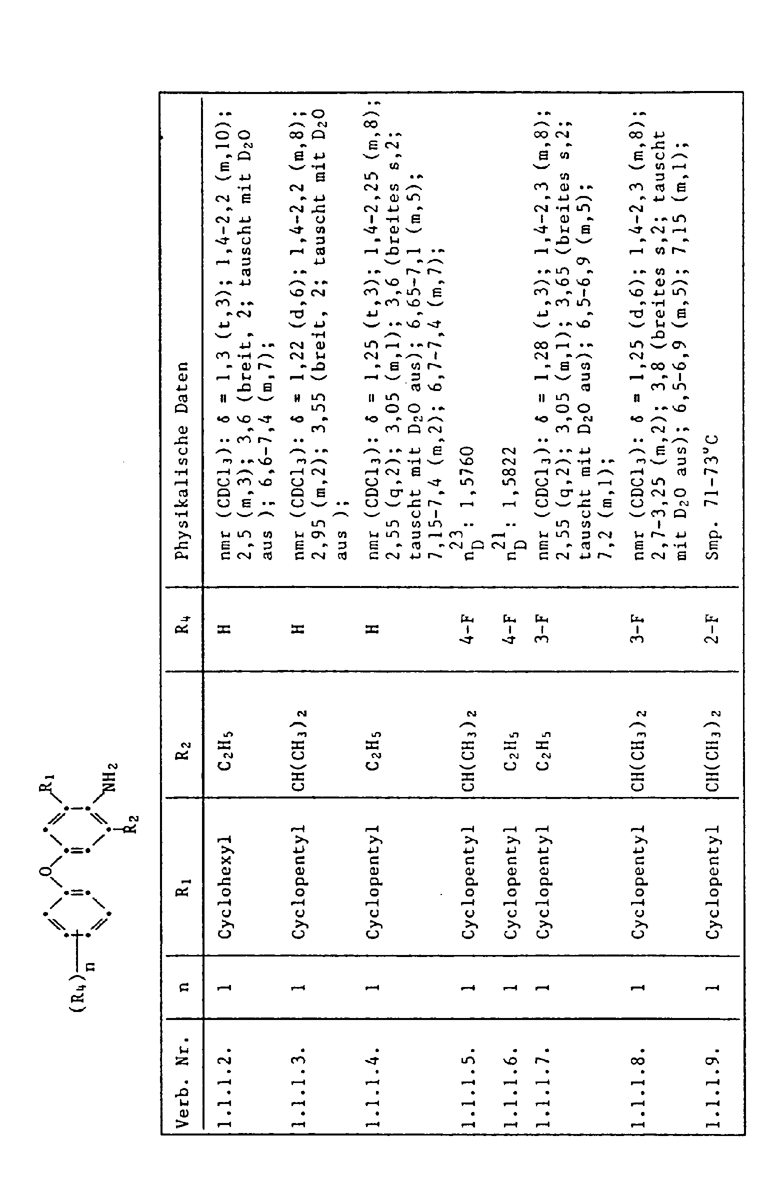

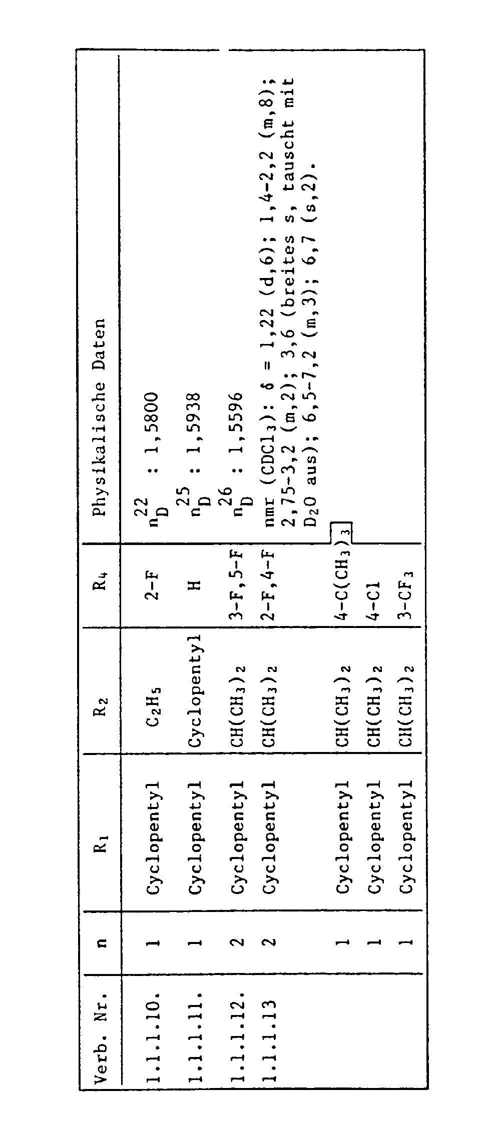

### 1.1.2. Phenylisothiocyanate

### 1.1.2.1. 2-Cyclohexyl-4-phenoxy-6-isopropyl-phenylisothiocyanat

7,0 g 2-Cyclohexyl-4-phenoxy-6-isopropylanilin, gelöst in 20 ml Dichlormethan werden unter starkem Rühren tropfenweise zu 3,1 g Thiophosgen, 40 ml Dichlormethan, 20 ml Wasser und 5,0 g gemahlenem Calciumcarbonat gegeben. Das Reaktionsgemisch wird 5 Stunden lang unter Rückfluss gerührt, dann abgekühlt und über Kieselgur filtriert. Die organische Phase wird abgetrennt, mit Wasser gewaschen, über Natriumsulfat getrocknet und am Vacuum vom Lösungsmittel befreit. Die Titelverbindung der Formel 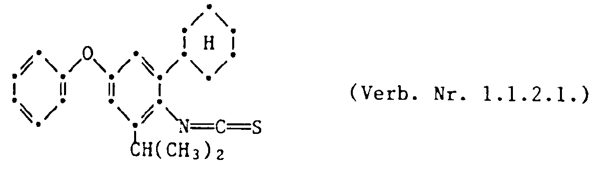 liegt in Form von gelben Kristallen vor. Smp. 58-60,5°C.

Auf analoge Weise werden die folgenden Verbindungen herstellt:

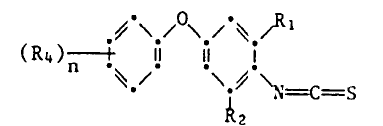

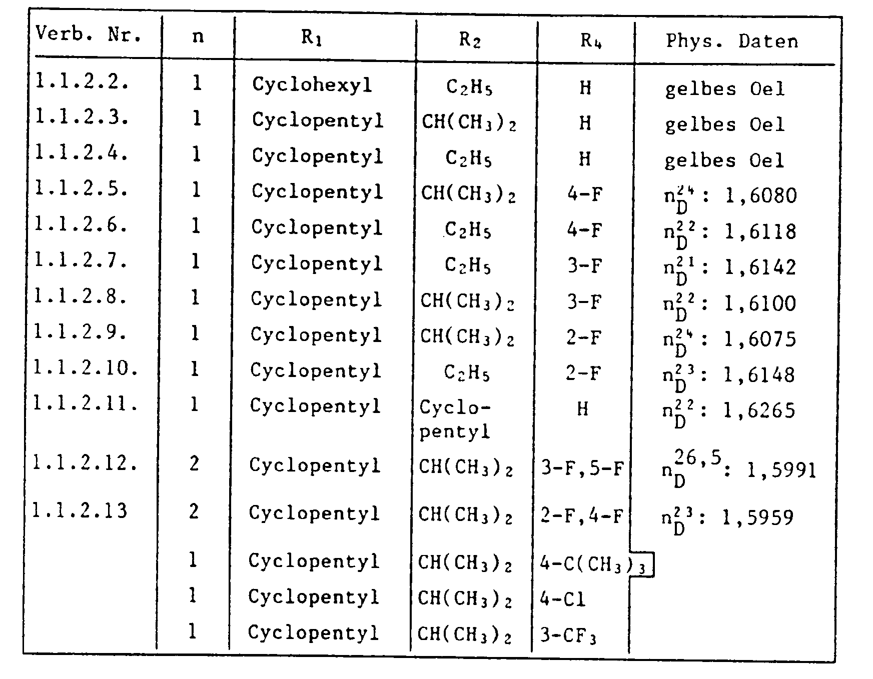

### 1.2. Endprodukte

### 1.2.1. Phenoxyphenylthioharnstoffe

### 1.2.1.1. N-(2-Cyclohexyl-4-phenoxy-6-isopropyl)-phenyl-N'-tert.butyl-thioharnstoff

7.3 g 2-Cyclohexyl-4-phenoxy-6-isopropyl-phenylisothiocyanatwerden in 15 ml Tetrahydrofuran gelöst und mit 3,3 g tert.Butylamin versetzt. Das Reaktionsgemisch wird 20 Stunden lang bei Raumtemperatur stehengelassen, dann auf Wassergegossen und anschliessend dreimal mit Hexan extrahiert. Die Hexanauszüge werden über Natriumsulfat getrocknet, und schliesslich wird das Hexan abdestilliert. Die Titelverbindung der Formel 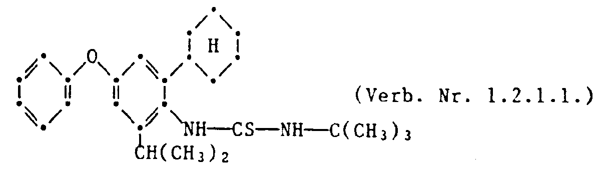 wird aus Hexan umkristallisiert. Smp. 134-136°C.

Auf analoge Weise werden die folgenden Verbindungen herstellt:

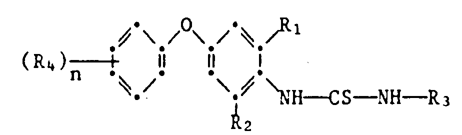

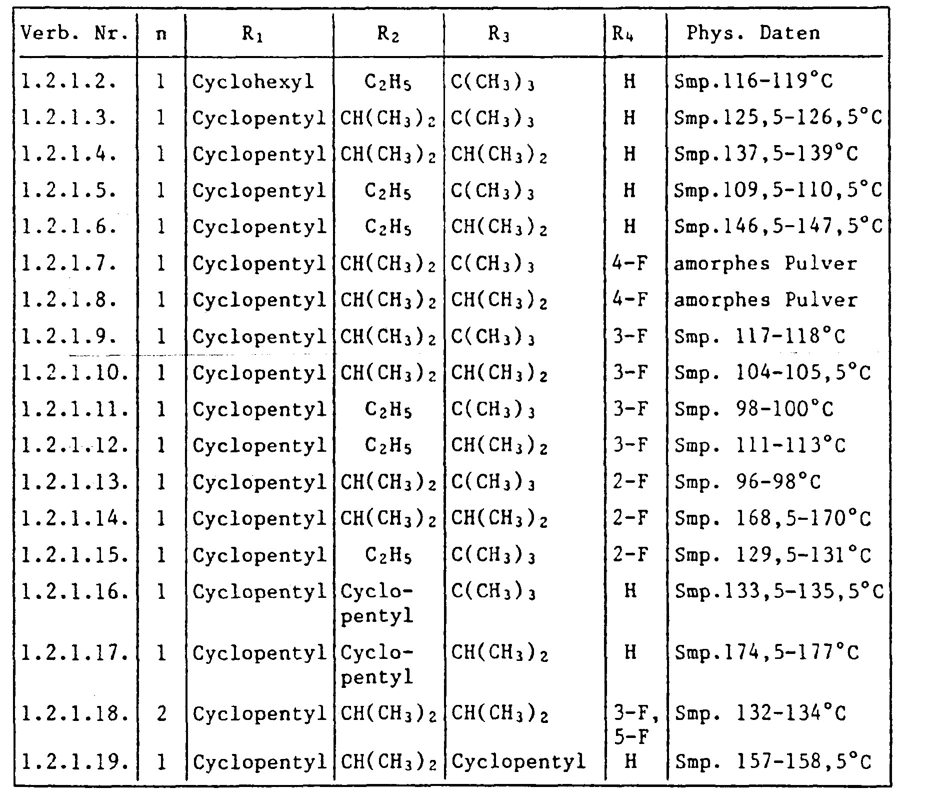

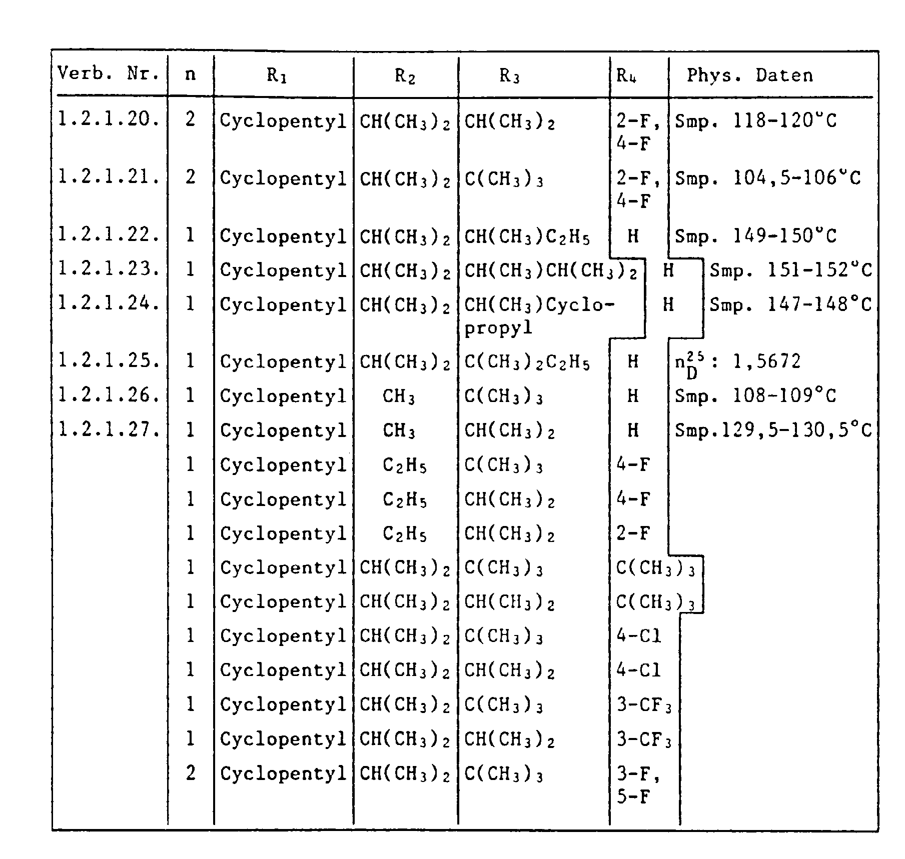

### 1.2.2. Phenoxyphenylisothioharnstoffe

### 1.2.2.1. N-(2-Cyclohexyl-4-phenoxy-6-isopropyl)-phenyl-N'-tert.butyl-S-methyl-isothioharnstoff

2,6 g N-(2-Cyclohexyl-4-phenoxy-6-isopropyl)-phenyl-N'-tert.butyl-thioharnstoffwerden in 35 ml Aethanol vorgelegt, bei Raumtemperatur mit 1,3 g Methyljodid versetzt und während 6 Stunden bei 40°C gerührt. Anschliessend wird die Reaktionslösung abgekühlt, auf 500 ml Wasser gegossen und dreimal mit Methylenchlorid extrahiert.

Die organischen Phasen werden je dreimal mit 5%iger wässriger Sodalösung und mit Wasser gewaschen, anschliessend über Natriumsulfat getrocknet, und schliesslich wird das Lösungsmittel abgedampft. Der Rückstand wird auf Kieselgel mit Hexan:Aether (4:1) als Laufmittel chromatographiert. Die Titelverbindung der Formel 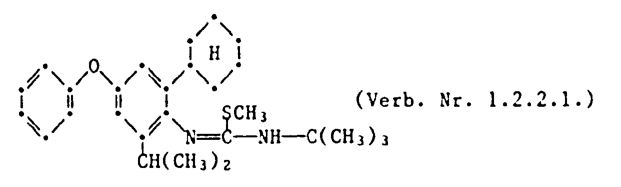 liegt in Form farbloser Kristalle vor. Smp. 91-99°C.

Das HJ-Salz von N-(2-Cyclopentyl-4-phenoxy-6-isopropyl)-phenyl-N'-isopropyl-S-methyl-isothioharnstoff (Verb. 1.2.2.3.) wird hergestellt, indem man 5,0 g N-(2-Cyclopentyl-4-phenoxy-6-isopropyl)-phenyl-N'-isopropyl-thioharnstoff in 50 ml Aethanol vorlegt, bei Raumtemperatur mit 2,7 g Methyljodid versetzt und während 6 Stunden bei 40°C rührt. Anschliessend wird die Reaktionslösung abgekühlt, auf 400 ml Wasser gegossen und der entstandene gelbe Niederschlag abfiltriert. Der getrocknete Rückstand wird aus Aethanol:Wasser (1:2) umkristallisiert und weist einen Smp. von 175,5-177°C auf.

Auf analoge Weise werden die folgenden Verbindungen herstellt:

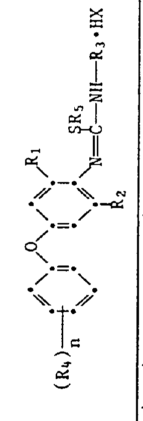

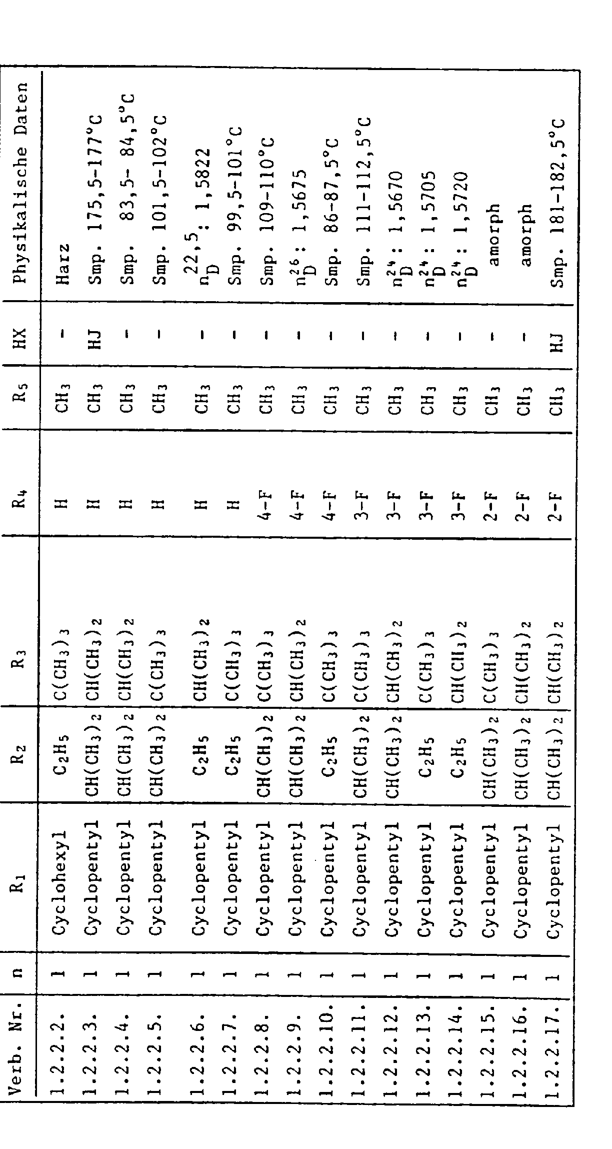

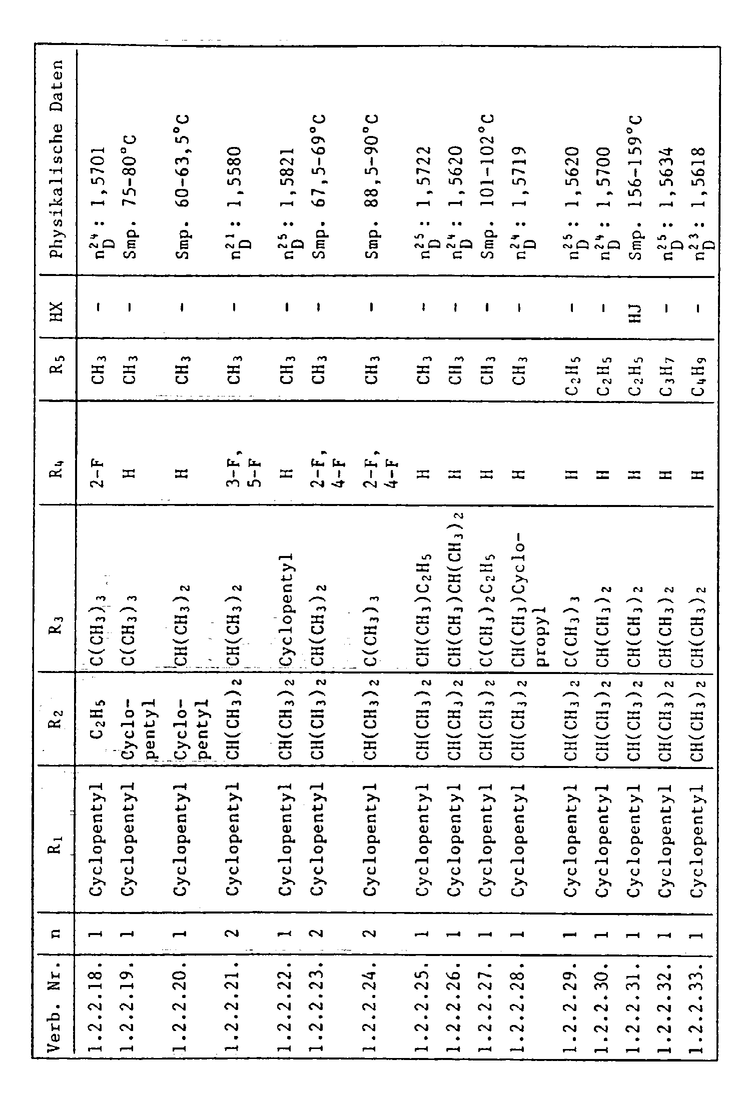

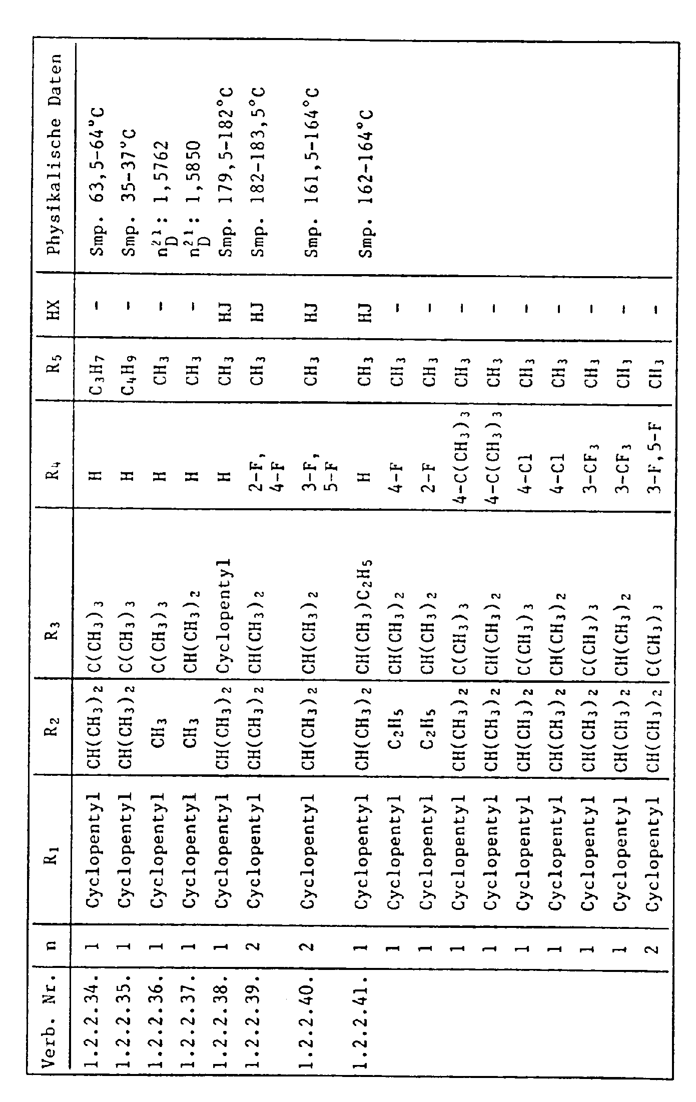

### 1.2.3. Phenoxyphenylcarbodiimide

### 1.2.3.1. N-(2-Cyclohexyl-4-phenoxy-6-isopropyl)-phenyl-N'-tert.butyl-carbodiimid

2,6 g N-(2-Cyclohexyl-4-phenoxy-6-isopropyl)-phenyl-N'-tert.butyl-thioharnstoff und 1,9 g 2-Chlor-1-me- thylpyridiniumjodid werden in 20 ml trockenem Acetonitril vorgelegt, bei Raumtemperatur tropfenweise unter Rühren mit 1,5 g Triäthylamin in 20 ml Acetonitril versetzt und während 2 ¹/₂ Stunden unter Rückfluss erhitzt. Anschliessend wird das Reaktionsgemisch eingedampft und der Rückstand mehrmals mit Hexan digeriert. Die Hexanphasen werden gut mit Wassergewaschen, über Natriumsulfat getrocknet und schliesslich eingedampft. Der Rückstand wird in Hexan gelöst und mit Kieselgel entfärbt. Die Titelverbindung der Formel 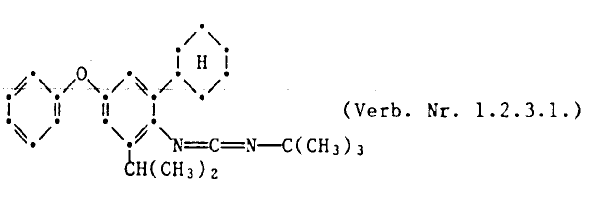 liegt als gelbes Oel mit einem Brechungsindex von 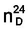 : 1,5640 vor.

Auf analoge Weise werden die folgenden Verbindungen hergestellt:

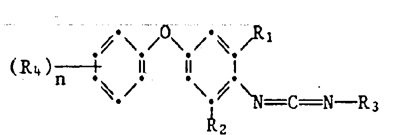

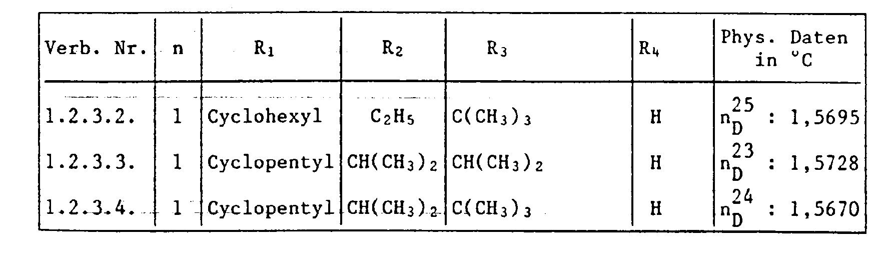

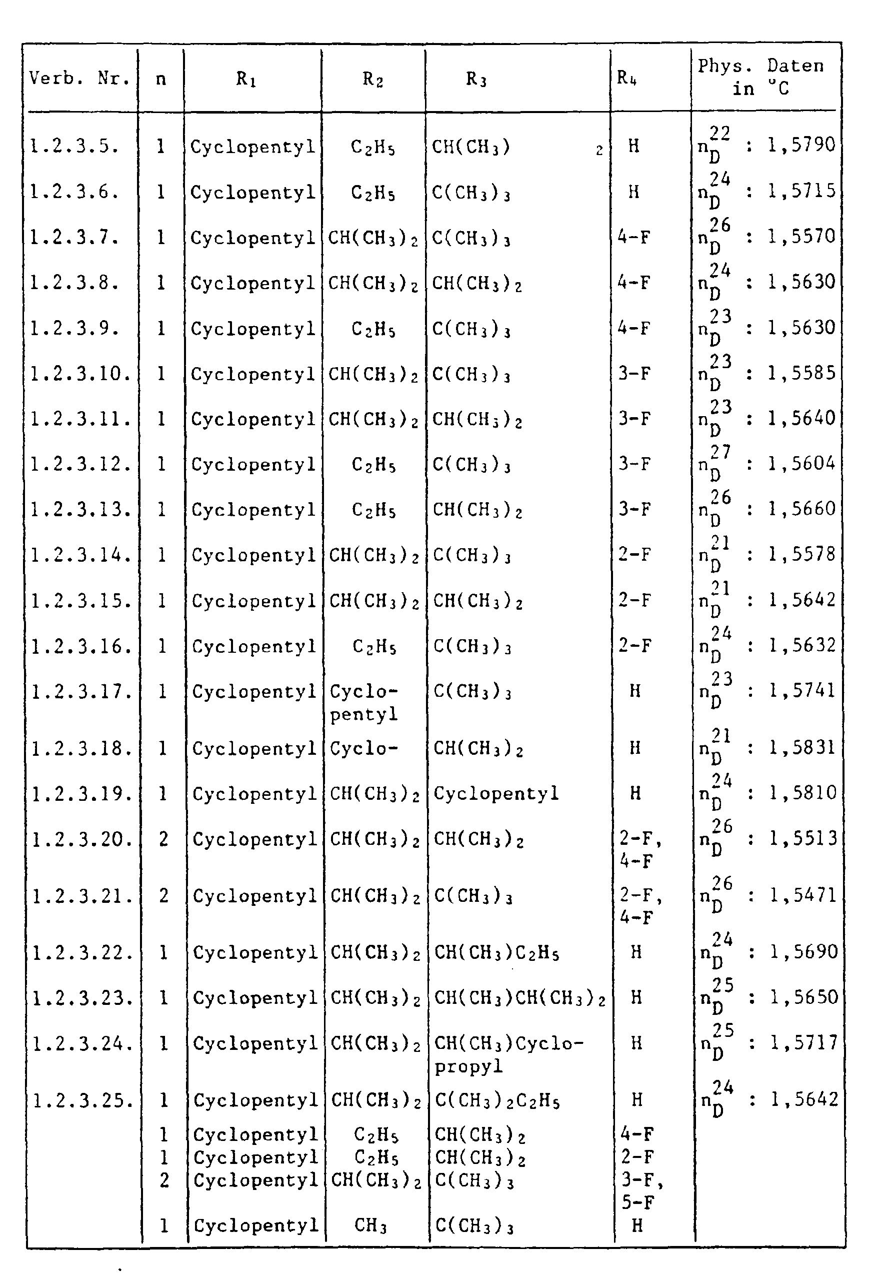

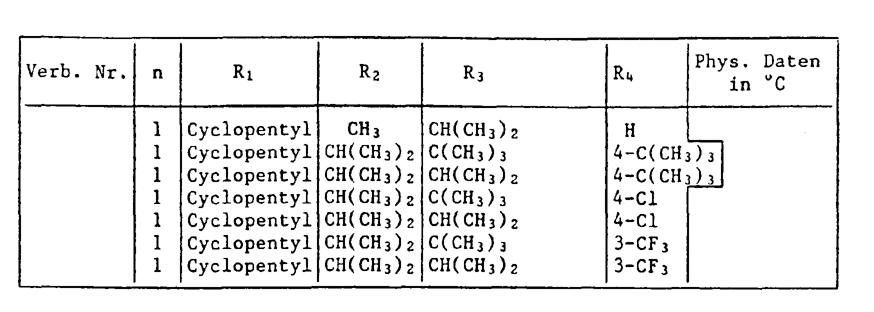

Beispiel 2: Formulierungen von Wirkstoffen der Formel gemäss den Herstellungsbeispielen 1.2.

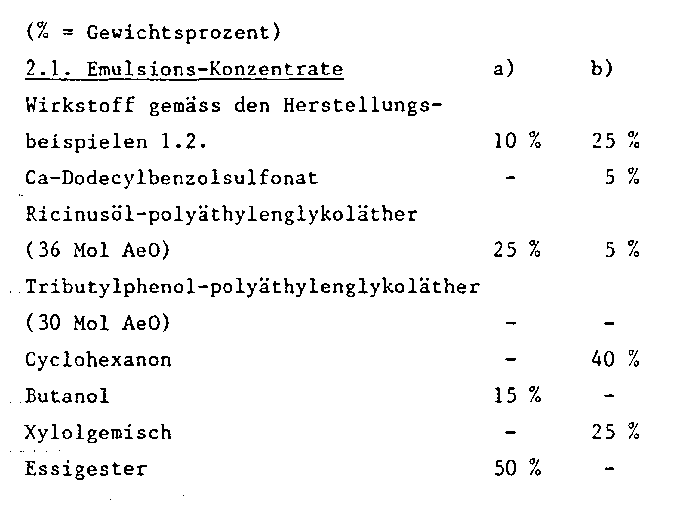

Aus solchen Konzentraten können durch Verdünnen mit Wasser Emulsionen jeder gewünschten Konzentration hergestellt werden.

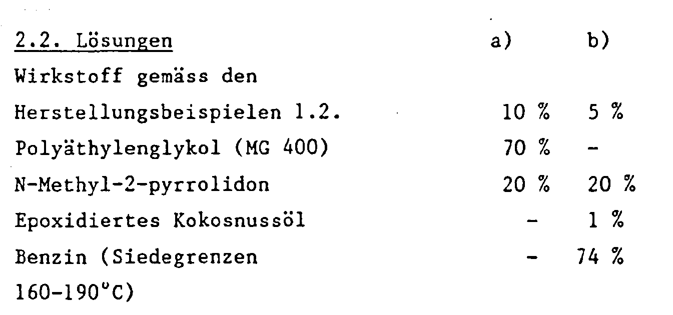

Die Lösungen sind zur Anwendung in Form kleinster Tropfen geeignet.

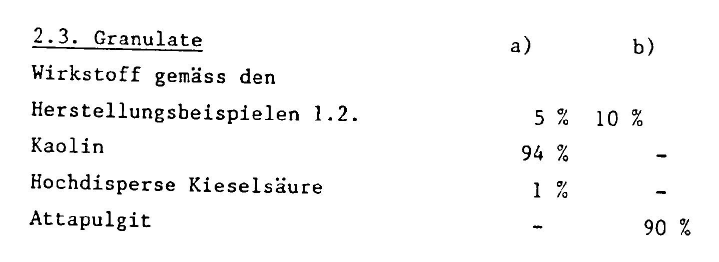

Der Wirkstoff wird in Methylenchlorid gelöst, auf den Träger aufgesprüht und das Lösungsmittel anschliessend im Vakuum abgedampft.

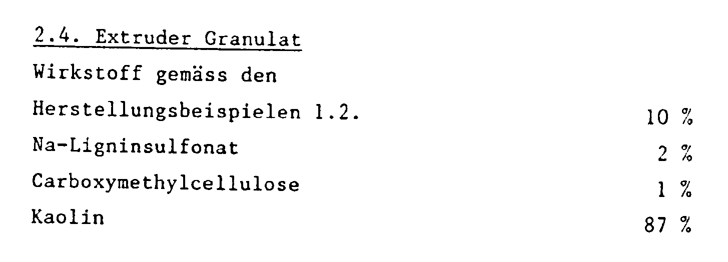

Der Wirkstoff wird mit den Zusatzstoffen vermischt, vermahlen und mit Wasser angefeuchtet. Dieses Gemisch wird extrudiert und anschliessend im Luftstrom getrocknet.

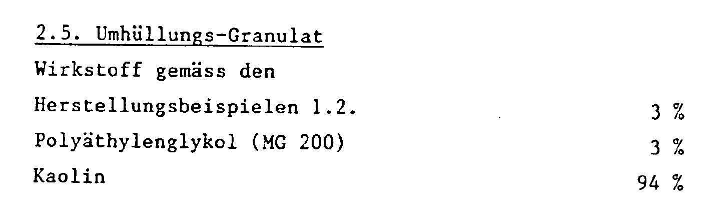

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

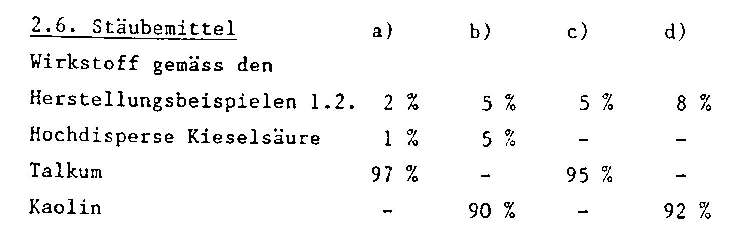

Durch inniges Vermischen der Trägerstoffe mit dem Wirkstoff und gegebenenfalls Vermahlen auf einer geeigneten Mühle erhält man gebrauchsfertige Stäubemittel.

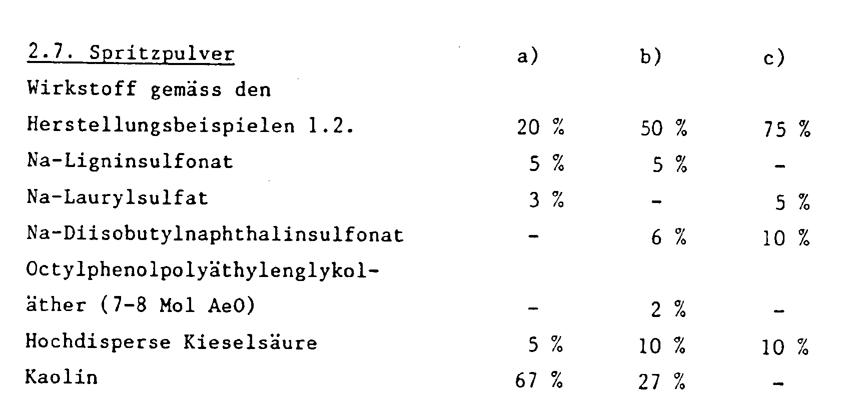

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

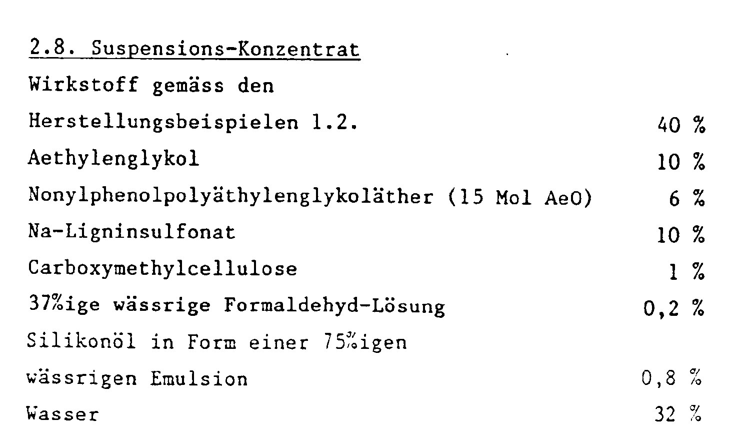

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Beispiel 3: Biologische Prüfungen

### 3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung derAbtötungsrate festgestellt.

Verbindungen gemäss den Beispielen 1.2.1., 1.2.2. und 1.2.3. zeigen in diesem Test gute Wirkung.

### 3.2. Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss den Beispielen 1.2.1., 1,2,2. und 1.2.3. zeigen in diesem Test gute Wirkung gegen Lucilia sericata.

### 3.3. Wirkung gegen ektoparasitäre Zecken

10 frische, vollgesogene Boophilus microplus Weibchen werden in einer Reihe dorsal auf einer PVC Platte aufgeklebt und mit einem Wattebausch überdeckt. Danach werden die Testtiere mit 10 ml der wässrigen Testlösung übergossen. Eine Stunde später wird der Wattebausch entfernt und die Zecken über Nacht bei 24°C getrocknet. Nach dem Trocknen werden die Zecken 4 Wochen lang bis zum Ende der Eiablage und Beginn des Larvenschlupfes bei 28°C und 80 % Luftfeuchtigkeit gehalten.

Jede Testsubstanz wird in einer Konzentration von 500 ppm getestet. Die akarizide Wirkung zeigt sich entweder beim Weibchen als Mortalität oder Sterilität oder im Eigelege durch Blockieren der Embryogenese oder des Schlüpfaktes. Alle Substanzen werden gegen zwei verschiedene Zeckenstämme, den OP-resistenten Stamm BIARRA und den Amidin-resistenten Stamm ULAM geprüft.

Verbindungen gemäss den Beispielen 1.2.1., 1.2.2. und 1.2.3. zeigen gute Wirkung im obigen Test.

### 3.4. Frassgift-Wirkung auf Spodoptera littoralis-Larven (L₁)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26°C und ca. 50 % relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss den Beispielen 1.2.1., 1.2.2. und 1.2.3. zeigen gute Wirkung in diesem Test.

### 3.5. Frassgift-Wirkung auf Spodoptera littoralis- und Heliothis virescens-Larven (L₃)

Eingetopfte Sojapflanzen (Topfgrösse 10 cm Durchmesser) im 4-Blatt-Stadium werden mit wässrigen Wirkstoffemulsionen besprüht, die den Wirkstoff in Konzentrationen von 50 bis 400 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2.1., 1,2,2. und 1.2.3. zeigen 80-100%ige Wirkung (Mortalität).

### 3.6. Frassgift-Wirkung auf Plutella xylostella-Larven (L₂)

Eingetopfte Chinakohlpflanzen (Topfgrösse 10 cm Durchmesser) im 4-Blatt-Stadium werden mit wässrigen Wirkstoffemulsionen besprüht, die den Wirkstoff in Konzentrationen von 50 bis 400 ppm enthalten.

Nach zwei Tagen werden die behandelten Chinakohlpflanzen mit 10 Plutella xylostella-Larven im zweiten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2.1., 1.2.2. und 1.2.3. zeigen 80-100%ige Wirkung (Mortalität).

### 3.7. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 40 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 6 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Beleuchtungsperiode von 16 Stunden durchgeführt.

Verbindungen gemäss den Beispielen 1.2.1., 1.2.2. und 1.2.3 zeigen in diesem Test gute Wirkung.

### 3.8. Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N₂- bis N₃- Stadium besiedelt und mit einem Plastikzylinder abgedeckt. Der Versuch wird bei 26°C und ca. 60 % relativer Luftfeuchtigkeit mit einer Beleuchtungsperiode von 16 Stunden durchgeführt. Nach zwei und fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen gemäss den Beispielen 1.2.1., 1.2.2. und 1.2.3. zeigen im obigen Test 80-100%ige Wirkung (Mortalität) gegen Nilaparvata lugens.

### 3.9. Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Durchmeservon ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 24°C und einer relativen Luftfeuchtigkeit von ca. 50 % gehalten. Sechs Tage nach dem Ansatz wird die in den Bechern enthaltene Erde abgesiebt und die zurückbleibenden Larven werden auf ihre Mortalität geprüft.

Verbindungen gemäss den Beispielen 1.2.1., 1.2.2. und 1.2.3. zeigen gute Wirkung im obigen Test.

### 3.10. Kontaktwirkung auf Aphis craccivora

In Töpfen angezogene 4-5 Tage alte Erbsenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach 3 und 5 Tagen. Der Versuch wird bei ca. 21 °C und einer relativen Luftfeuchtigkeit von etwa 55% durchgeführt.

Verbindungen gemäss den Beispielen 1.2.1., 1.2.2. und 1.2.3. zeigen gute Wirkung in diesem Test.

### 3.11. Kontaktwirkung auf Myzus persicae

Etwa 4 bis 5 Tage alte, in Wasser angezogene Erbsenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend 100 ppm der zu prüfenden Verbindung bis zur Tropfnässe direkt besprüht. Man verwendet pro Testsubstanz zwei Pflanzen. Eine Auswertung der erzielten Abtötungsrate erfolgt 3 und 5 Tage nach Applikation. Der Versuch wird bei ca. 21°C und etwa 60 % relativer Luftfeuchtigkeit durchgeführt.

Verbindungen gemäss den Beispielen 1.2.1, 1.2.2. und 1.2.3. zeigen gute Wirkung in diesem Test.

### 3.12. Wirkung gegen Tetranychus urticae (OP-sensibel)

Die Primärblättervon Phaseolus vulgaris-Pflanzen werden 24 Stunden vor dem Versuch auf akarizide Wirkung mit einem infestierten Blattstück aus einer Massenzucht von Tetranychus urticae (OP-sens.) belegt (Mischpopulation). Die Toleranz bezieht sich auf die Verträglichkeit gegenüber Diazinon.

Die so behandelten infestierten Pflanzen werden mit einer Versuchslösung in Emulsionsform enthaltend 400 ppm der zu prüfenden Verbindung bis zur Tropfnässe besprüht. Während des Versuchsverlaufs stehen die Pflanzen in Gewächshauskabinen bei ca. 25°C mit etwa 50 % rel. Luftfeuchtigkeit.

Nach 6 Tagen werden Imagines und Larven (alle beweglichen Stadien) sowie abgelegte Eier unter dem Binokular auf lebende und tote Individuen ausgewertet.

Verbindungen gemäss den Beispielen 1.2.1., 1.2.2. und 1.2.3. zeigen in diesem Versuch gute Wirkung.

### 3.13. Wirkung gegen Panonychus ulmi (OP- und Carbamat-resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 100 ppm derzu prüfenden Verbindung bis zur Tropfnässe besprüht. Die behandelten Pflanzen werden dann während 14 Tagen bei ca. 25°C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von dem entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen gemäss den Beispielen 1.2.1., 1.2.2. und 1.2.3. zeigen gute Wirkung im obigen Test.

### 3.14. Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss den Beispielen 1.2.1., 1.2.2. und 1.2.3. zeigen gute Wirkung in diesem Test.

### 3.15. Wirkung gegen sensible und resistente Adulte von Bemisia tabaci

Baumwollblätter werden in eine Testlösung enthaltend 400 ppm Wirkstoff eingetaucht. Auf die behandelten trockenen Blätter werden dann in zugedeckten Petri-Schalen 20-50 sensible resp. resistente Adulte von Bemisia tabaci gegeben. Nach 24 Stunden wird die Mortalität durch Auszählen bestimmt.

Verbindungen gemäss den Beispielen 1.2.1., 1.2.2. und 1.2.3. zeigen gute Wirung im obigen Test.

## Patentansprüche (Patentansprüche für folgende(n) Vertragsstaat(en) : AT, BE, CH, DE, FR, GB, GR, IT, LI, NL.)

1. Verbindungen der Formel 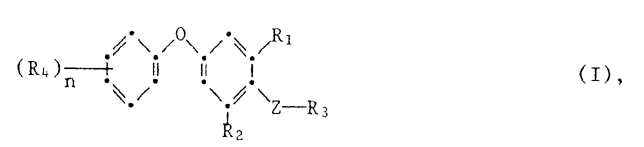
worin
R₁ für C₃-C₇-Cycloalkyl oder C₅-Cₛ-Cycloalkenyl,
R₂ für C₁-C₆-Alkyl, C₅-Cₛ-Cycloalkyl oder C₅-Cₛ-Cycloalkenyl,
R₃ für Cₗ-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-Cₐ-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, sowie deren Salze mit organischen oder anorganischen Säuren.

2. Verbindungen der Formel gemäss Anspruch 1, worin R₁ für C₃-C₇-Cycloalkyl, R₂ für C₁-C₄-Alkyl oder Cyclopentyl, R₃ für C₁-C₄-Alkyl oder C₃-C₅-Cycloalkyl, R₄ für Wasserstoff, Halogen oder C₁-C₃-Alkyl, n für die Zahlen 1 oder 2, Z für -NH-CS-NH-, -N=C(SR₅)-NH- oder -N=C=N- und R₅ für C₁-C₃-Alkyl stehen.

3. Verbindungen der Formel gemäss Anspruch 2, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für -NH-CS-NH- stehen.

4. Verbindungen der Formel gemäss Anspruch 2, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2, Z für -N=C(SR₅)-NH- und R₅ für Methyl oder Aethyl stehen.

5. Verbindungen der Formel gemäss Anspruch 2, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für -N=C=N- stehen.

6. Verbindungen gemäss einem der Ansprüche 1 oder 3 der Formeln

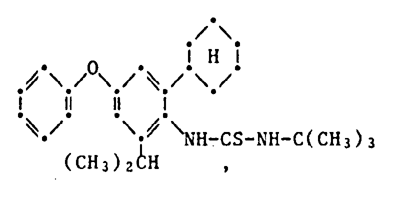

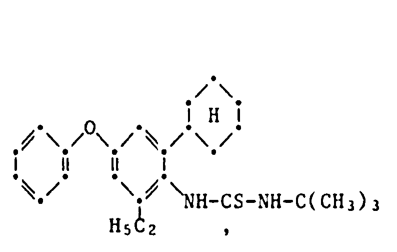

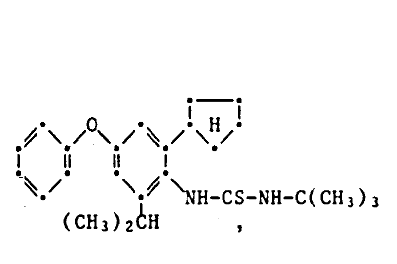

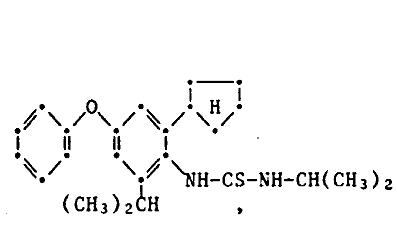

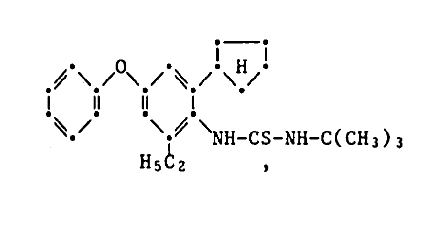

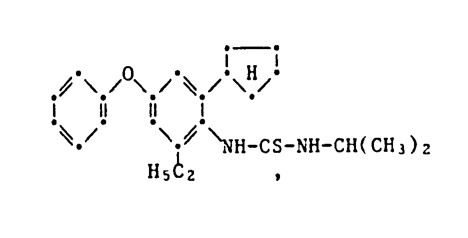

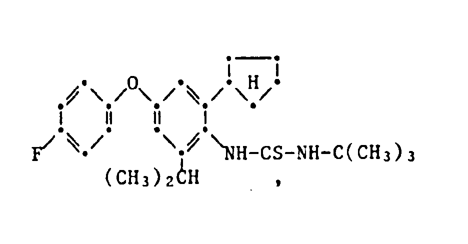

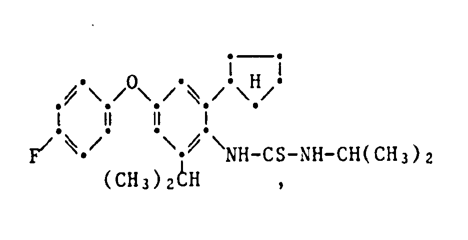

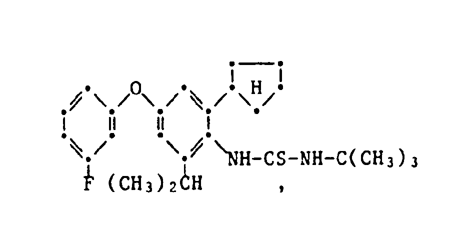

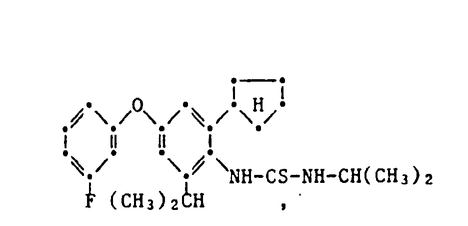

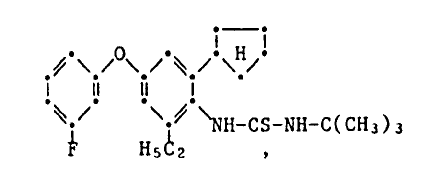

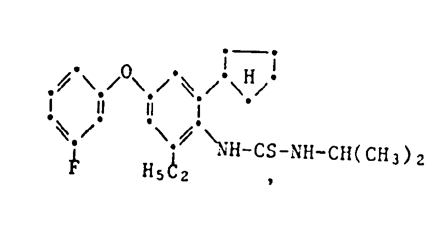

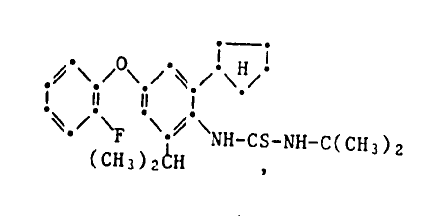

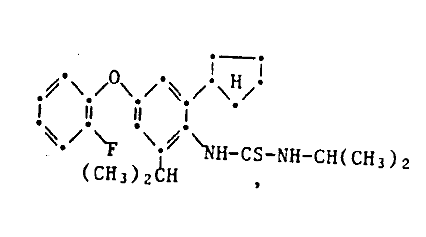

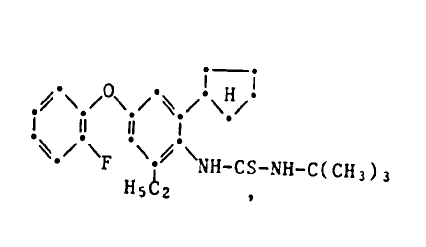

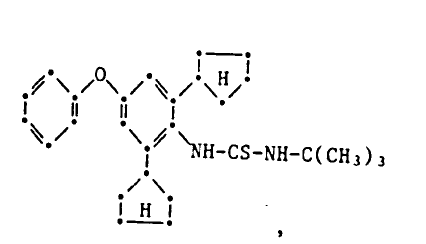

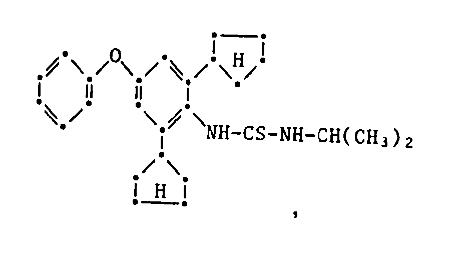

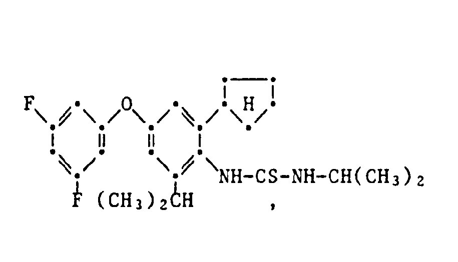

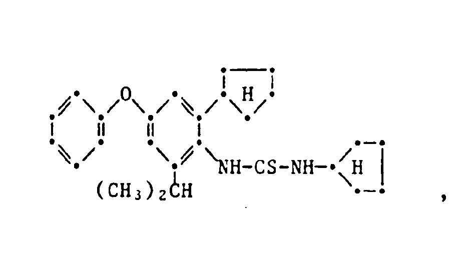

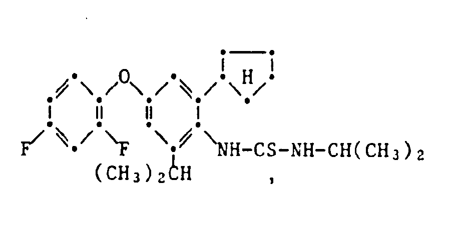

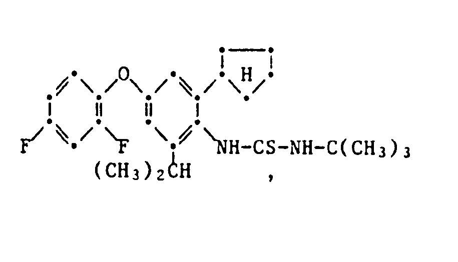

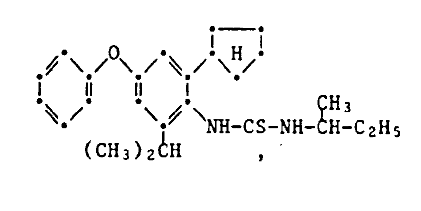

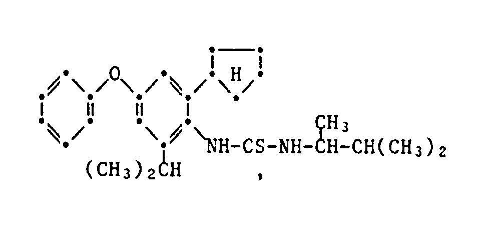

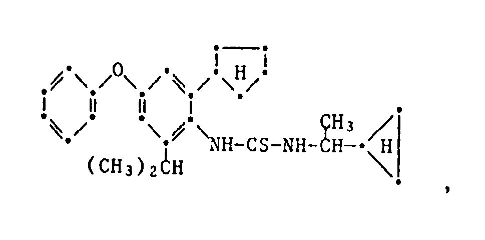

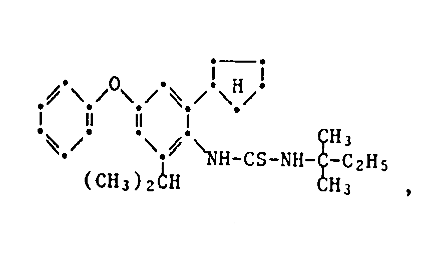

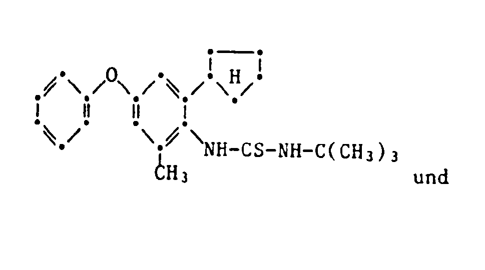

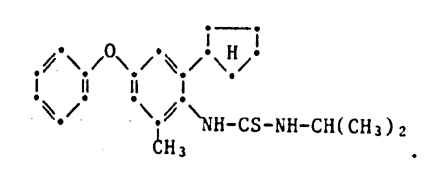

7. Verbindungen gemäss einem der Ansprüche 1 oder 4 der Formeln

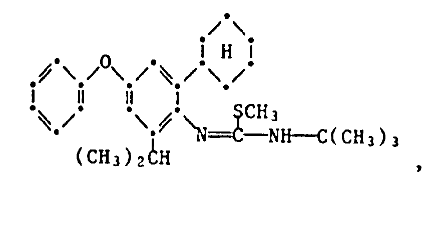

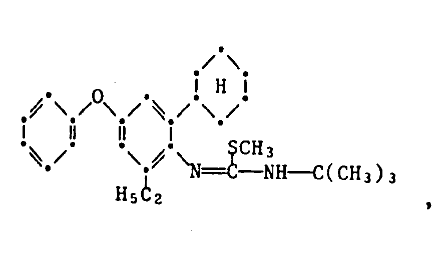

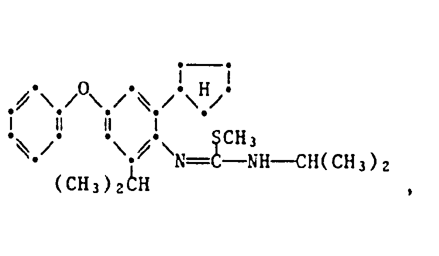

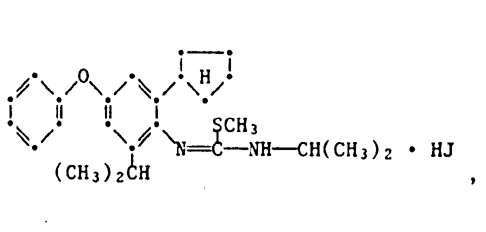

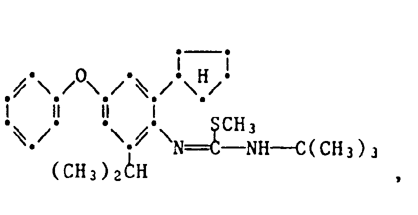

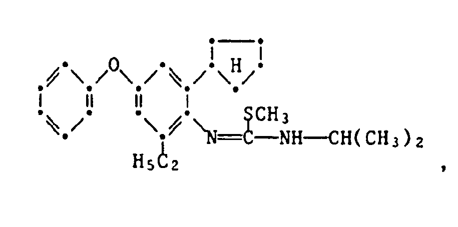

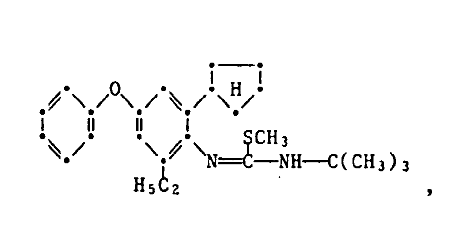

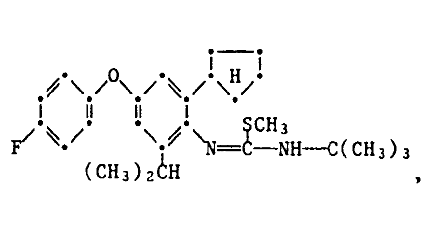

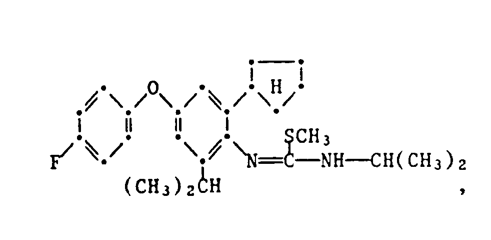

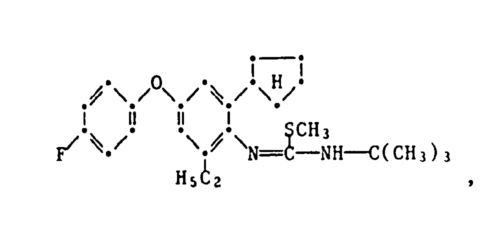

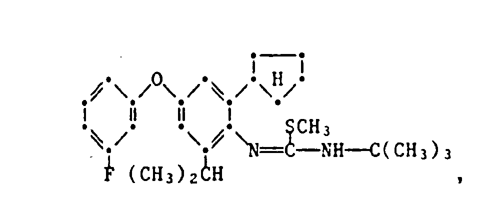

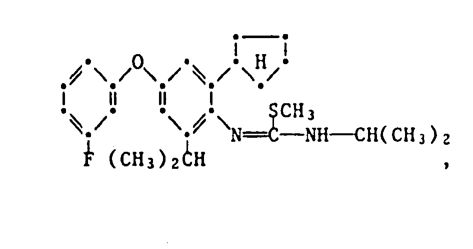

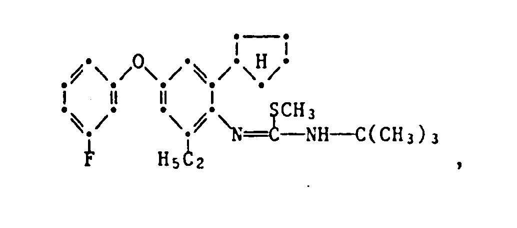

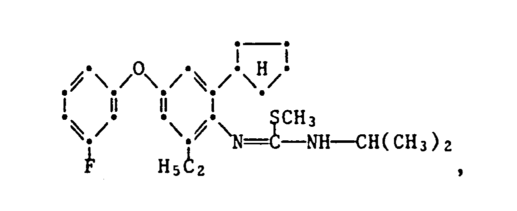

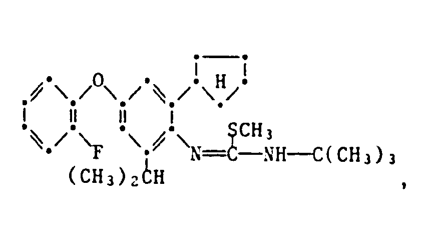

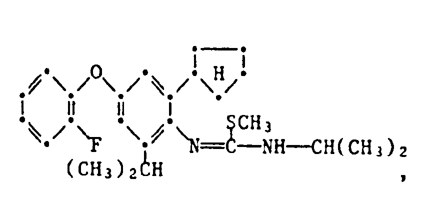

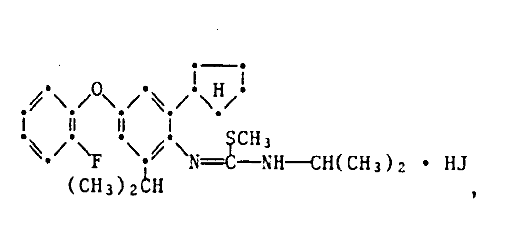

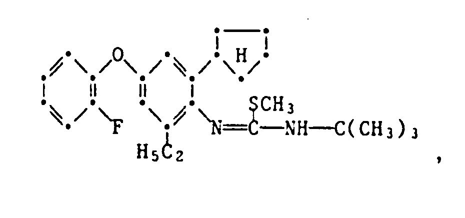

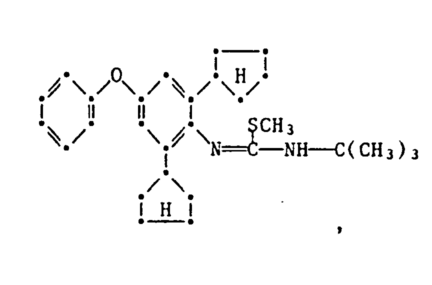

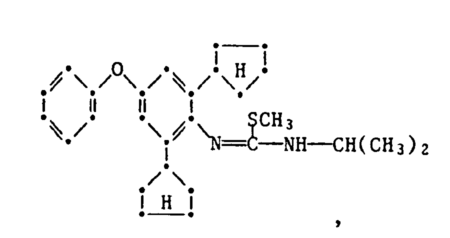

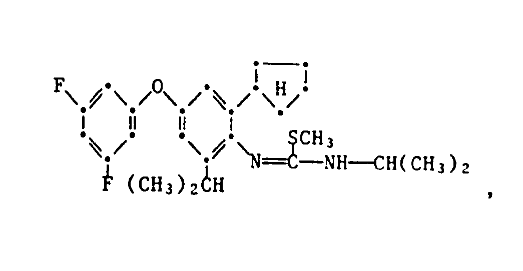

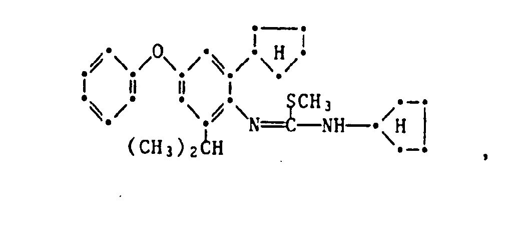

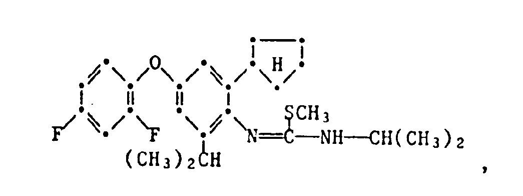

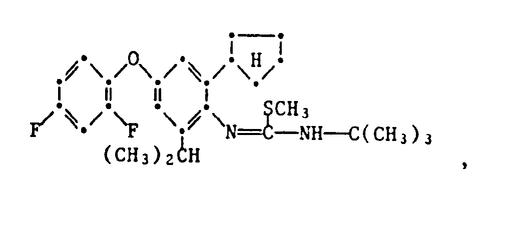

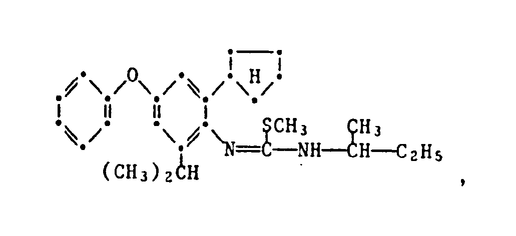

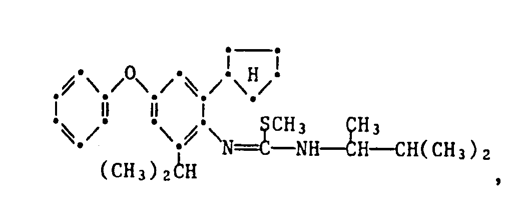

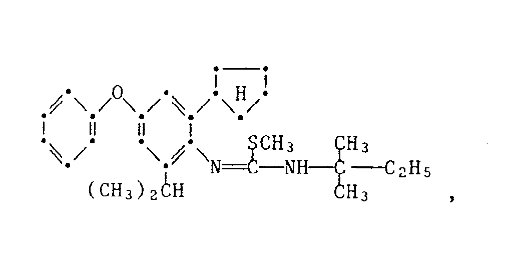

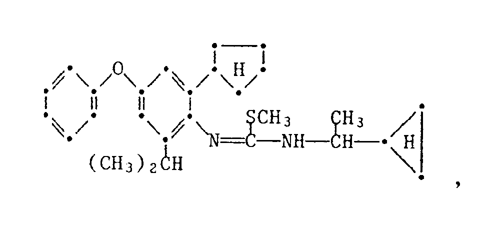

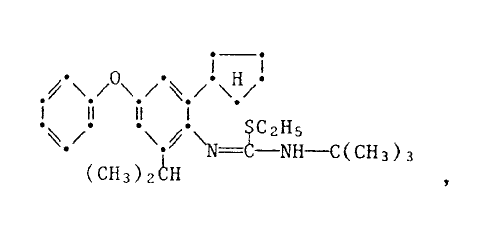

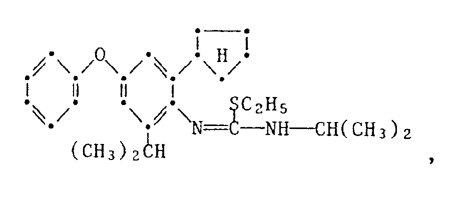

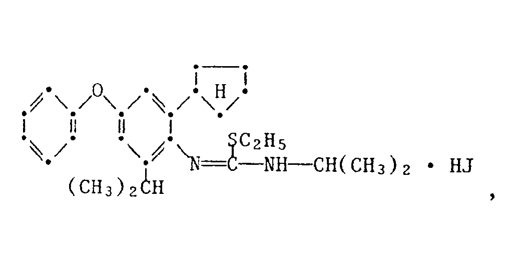

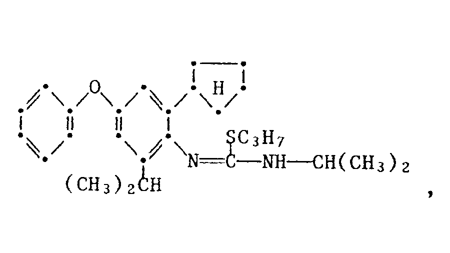

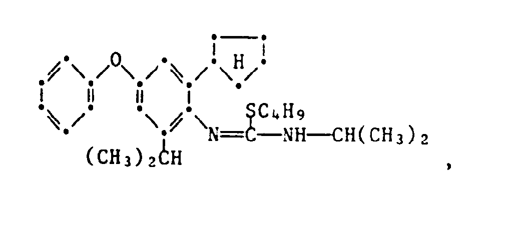

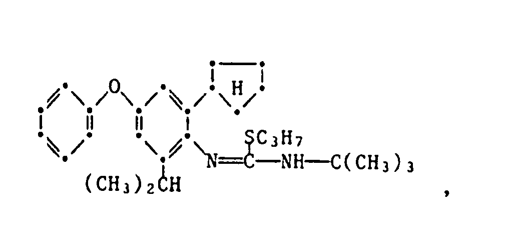

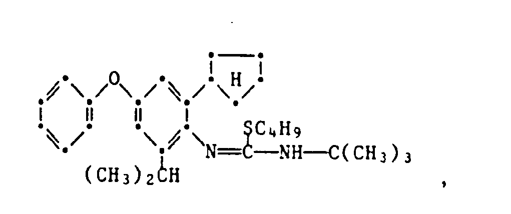

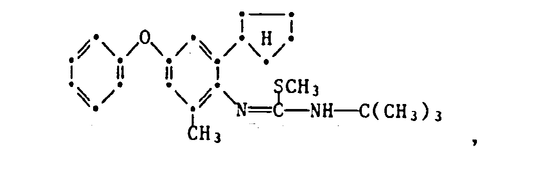

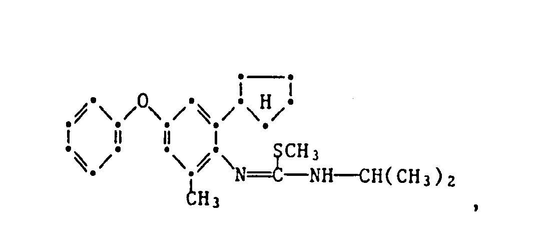

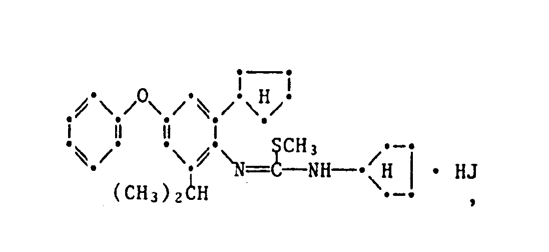

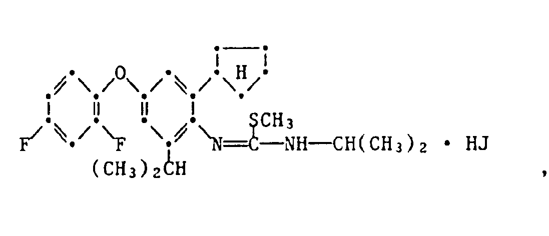

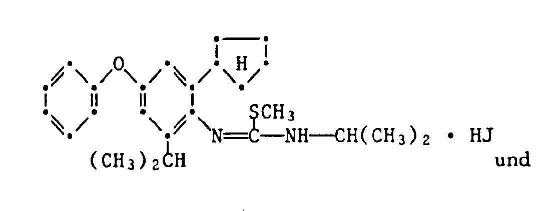

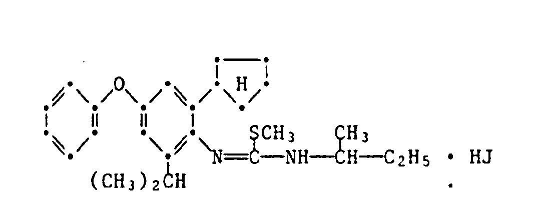

8. Verbindungen gemäss einem der Ansprüche 1 oder 5 der Formeln

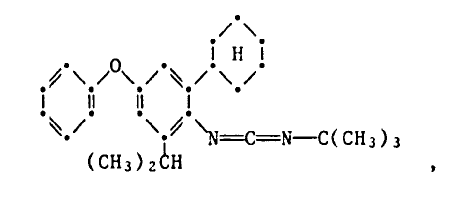

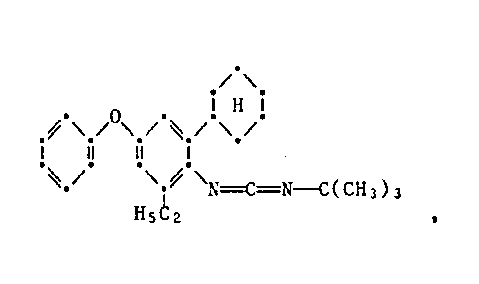

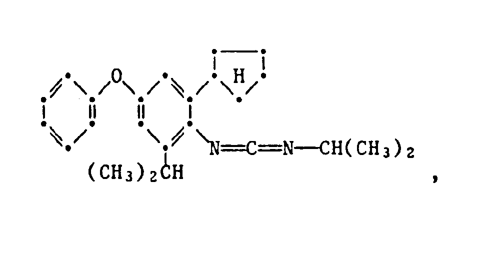

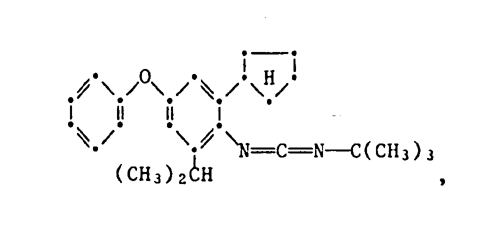

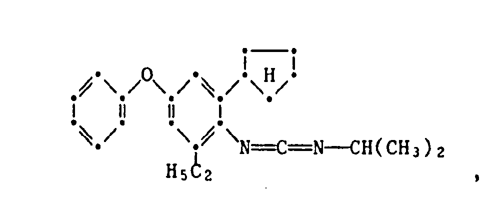

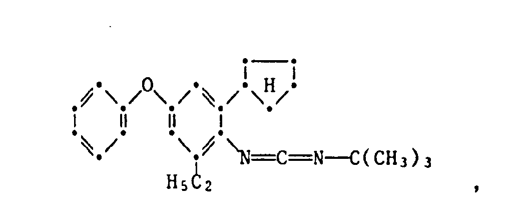

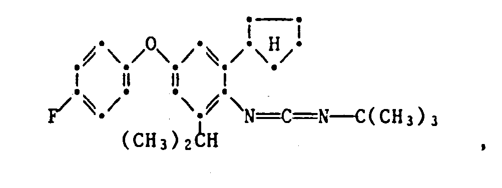

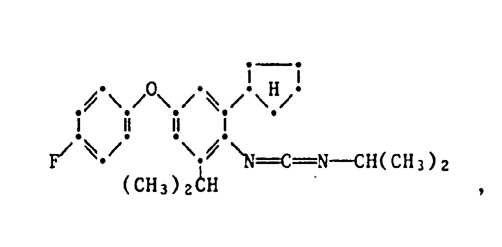

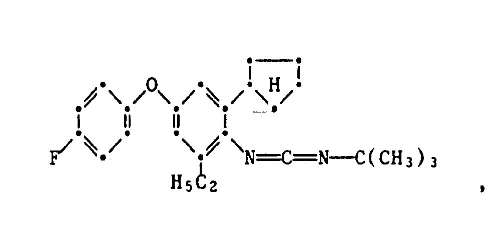

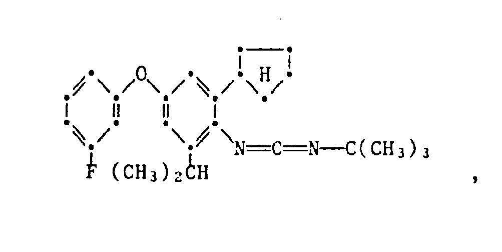

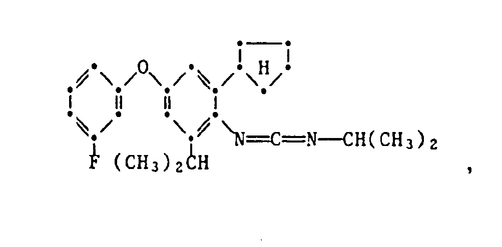

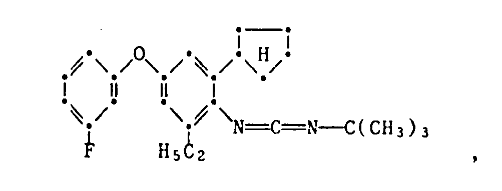

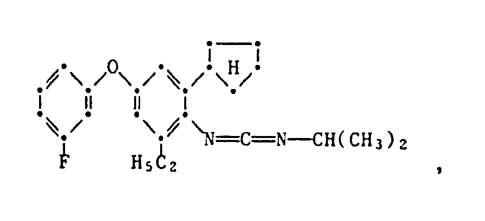

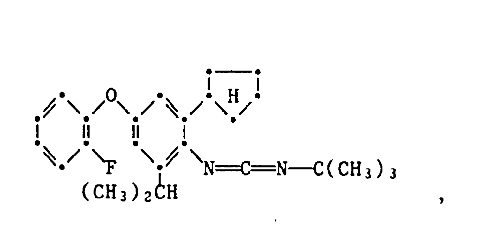

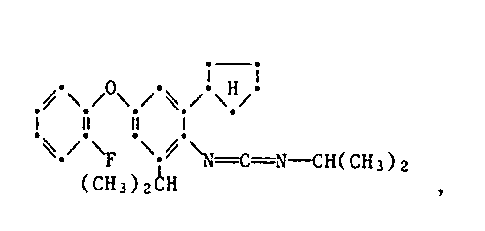

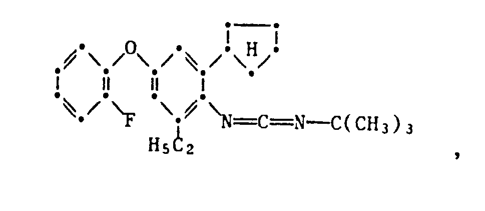

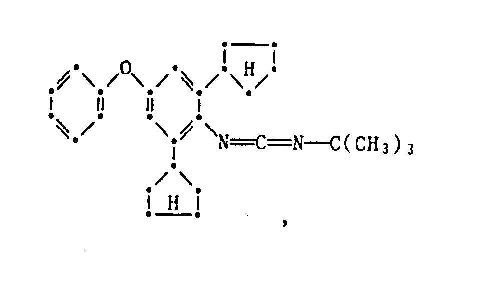

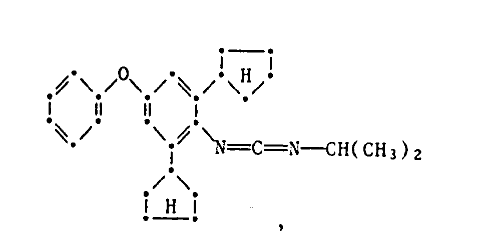

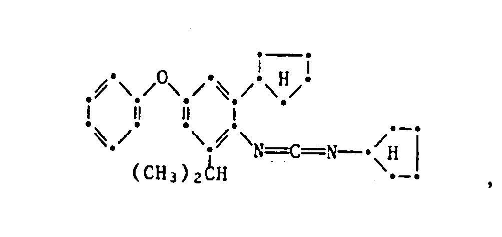

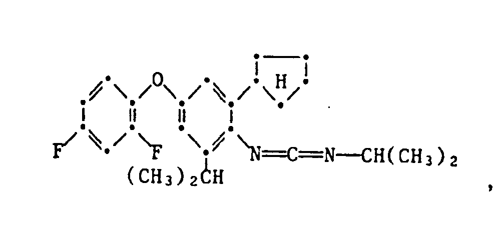

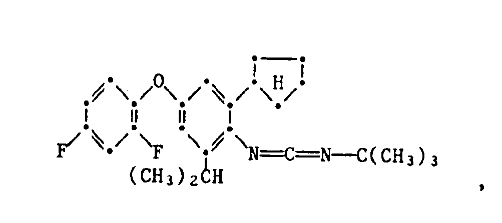

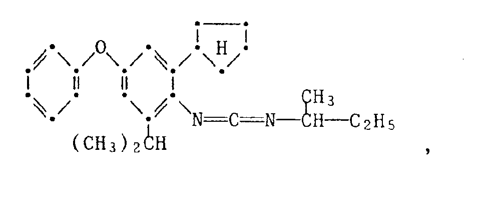

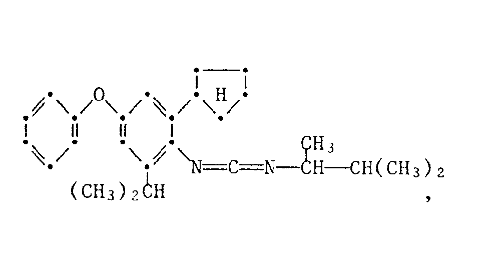

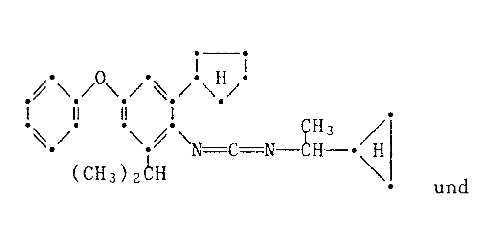

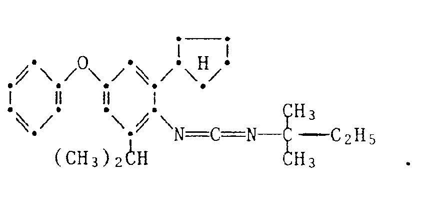

9. Verbindungen der Formel V 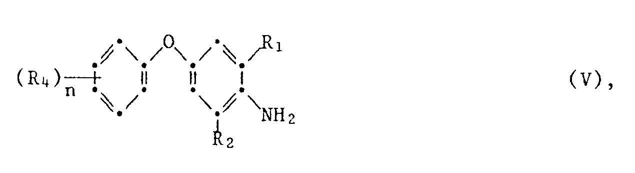
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₄ je für Wasserstoff, Halogen, C₁-C₄-Alkyl, C₁-C₃-Alkoxy oder CF₃ und
n für die Zahlen 1 bis 3
stehen.

10. Verbindungen der Formel V gemäss Anspruch 9, worin R₁ für C₃-C₇-Cycloalkyl, R₂ für C₁-C₄-Alkyl oder Cyclopentyl, R₄ für Wasserstoff, Halogen oder C₁-C₃-Alkyl und n für die Zahlen 1 oder 2 stehen.

11. Verbindungen der Formel V gemäss Anspruch 10, worin R₁ für C₅-C₆-Cycloalkyl, R₂ für Aethyl oder Isopropyl, R₄ für Wasserstoff oder Fluor und n für die Zahlen 1 oder 2 stehen.

12. Verbindungen gemäss einem der Ansprüche 10 oder 11 der Formeln

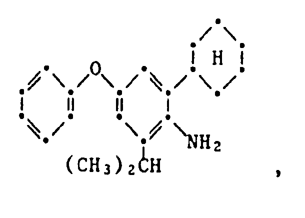

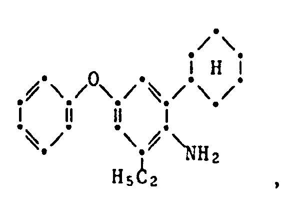

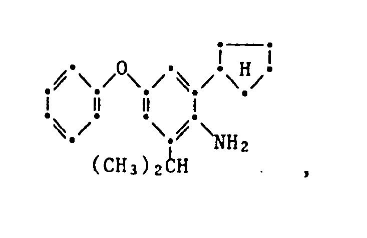

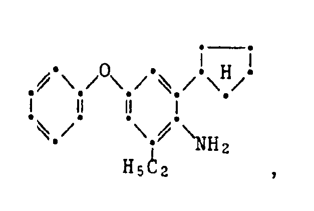

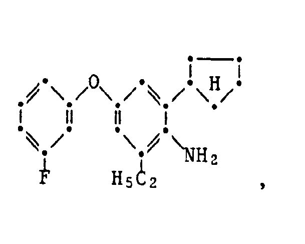

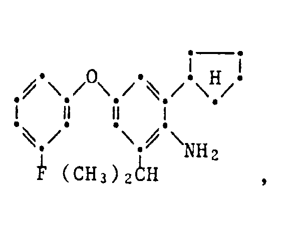

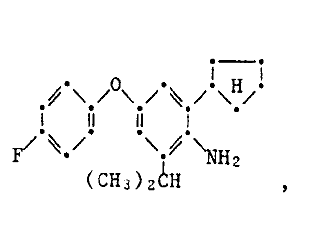

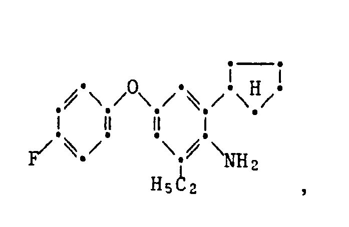

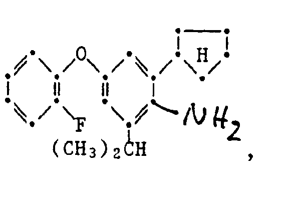

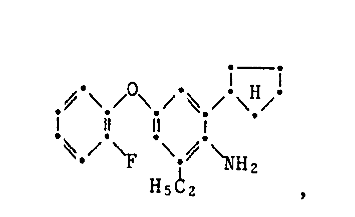

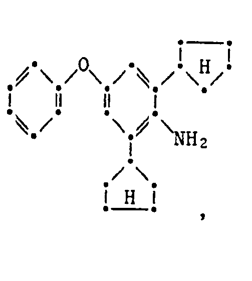

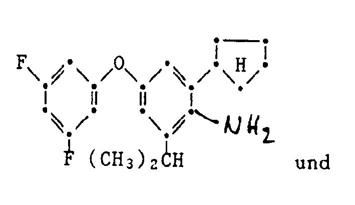

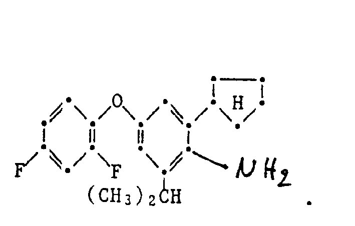

13. Verbindungen der Formel II 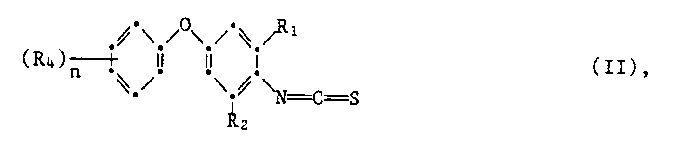
worin
R₁ für C₃-C₇-Cycloalkyl oder C₅-C₆-Cycloalkenyl,
R₂ für C₁-C₄-Alkyl oder C₅-Cₛ-Cycloalkyl,
R₄ je für Wasserstoff, Halogen, C₁-C₄-Alkyl, C₁-C₃-Alkoxy oder CF₃, und
n für die Zahlen 1 bis 3 stehen.

14. Verbindungen der Formel II gemäss Anspruch 13, worin R₁ für C₃-C₇-Cycloalkyl, R₂ für C₁-C₄-Alkyl, R₄ für Wasserstoff, Halogen oder C₁-C₃-Alkyl und n für die Zahlen 1 oder 2 stehen.

15. Verbindungen der Formel II gemäss Anspruch 14, worin R₁ für C₅-C₆-Cycloalkyl, R₂ für Aethyl oder Isopropyl, R₄ für Wasserstoff oder Fluor und n für die Zahlen 1 oder 2 stehen.

16. Verbindungen gemäss einem der Ansprüche 14 oder 15 der Formeln

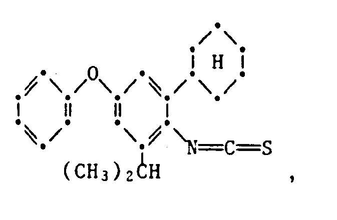

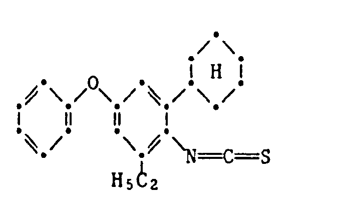

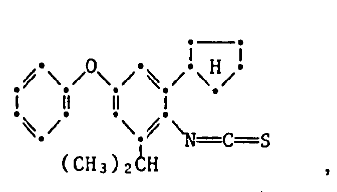

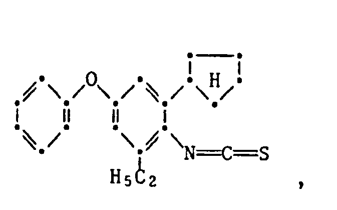

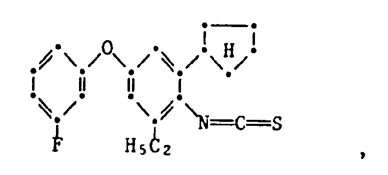

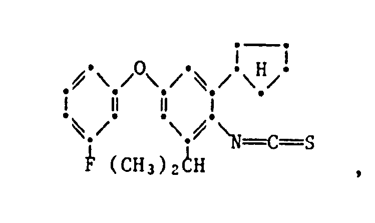

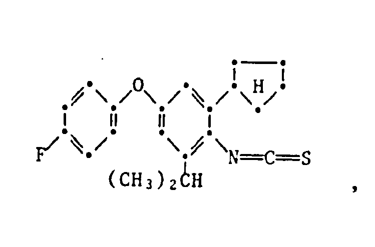

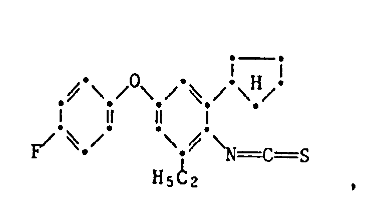

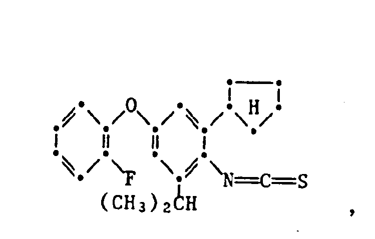

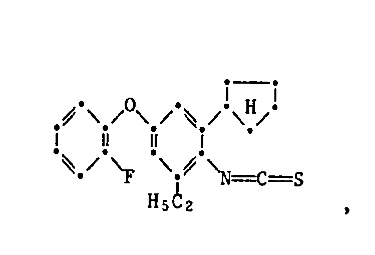

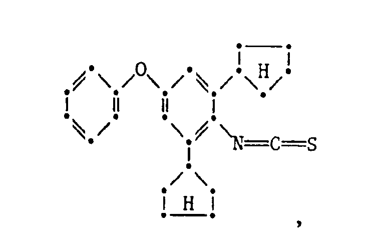

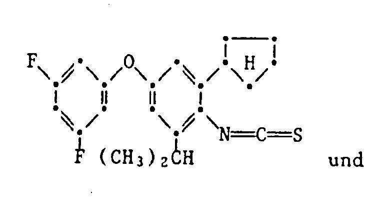

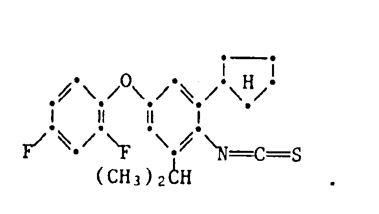

17. Verfahren zur Herstellung einer Verbindung der Formel 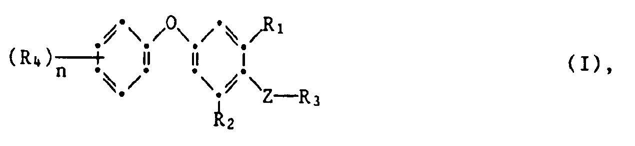
worin
R₁ für C₃-C₇-Cycloalkyl oder C₅-Cₛ-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl, R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl stehen, dadurch gekennzeichnet, dass man
A) ein Isothiocyanat der Formel II 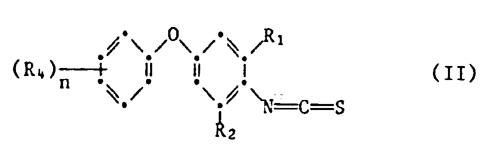 mit einem Amin der Formel III 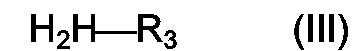 in einem organischen Lösungs- oder Verdünnungsmittel bei Normaldruck und einer Temperatur von 0 bis +150_{°}C zum Thioharnstoff umsetzt und gegebenenfalls
B) den erhaltenen Thioharnstoff mit einer Verbindung der Formel IV 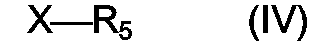 in einem inerten organischen Lösungsmittel unter leicht erhöhtem oder normalem Druck und bei einer Temperatur von +10 bis 250°C zum Isothioharnstoff umsetzt oder
C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150_{°}C in das Carbodiimid überführt, worin R_{1'} R₂, R₃, R₄, R₅ und n die angegebene Bedeutung haben und X für eine Abgangsgruppe steht.

18. Verfahren zur Herstellung einer Verbindung der Formel V 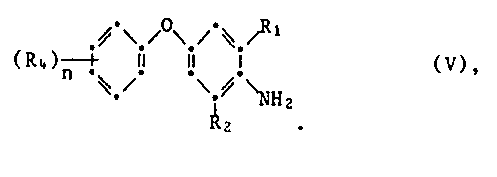
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₄ je für Wasserstoff, Halogen, C₁-C₄-Alkyl, C₁-C₃-Alkoxy oder CF₃ und
n für die Zahlen 1 bis 3 stehen, dadurch gekennzeichnet, dass man ein Phenol der Formel VII 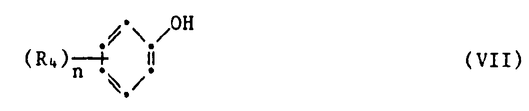
in Gegenwart einer organischen oder anorganischen Base, unter normalem Druck und bei einer Temperatur von 0 bis +100_{°}C in einem inerten Lösungs- oder Verdünnungsmittel mit einem Anilin der Formel VI 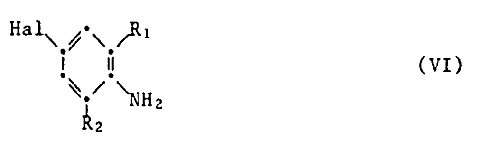
umsetzt, worin R_{1'} R₂, R₄ und n die angegebene Bedeutung haben und Hal für ein Halogenatom steht.

19. Verfahren zur Herstellung einer Verbindung der Formel II 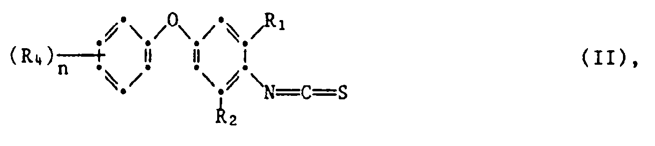
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₄ je für Wasserstoff, Halogen, C₁-C₄-Alkyl, C₁-C₃-Alkoxy oder CF₃ und
n für die Zahlen 1 bis 3 stehen, dadurch gekennzeichnet, dass man ein Phenoxyanilin der Formel V 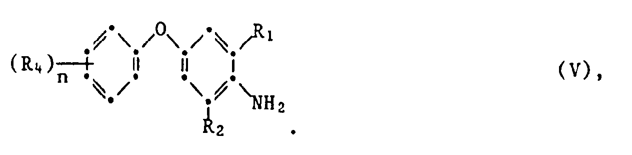 in Gegenwart einer organischen oder anorganischen Base, unter normalem Druck und bei einer Temperatur von 0 bis +100_{°}C in einem inerten Lösungs- oder Verdünnungsmittel mit Thiophosgen umsetzt, worin R_{1'} R₂, R₄ und n die angegebene Bedeutung haben.

20. Schädlingsbekämpfungsmittel, welches als aktive Komponente mindestens eine Verbindung der Formel 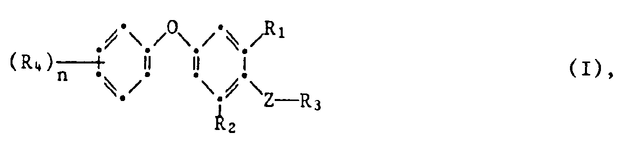
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, oder eines ihrer Salze mit einer organischen oder anorganischen Säure zusammen mit geeigneten Trägern und/oder Zuschlagstoffen enthält.

21. Schädlingsbekämpfungsmittel gemäss Anspruch 20, welches als aktive Komponente mindestens eine Verbindung der Formel enthält, worin R₁ für C₃-C₇-Cycloalkyl, R₂ für C₁-C₄-Alkyl oder Cyclopentyl, R₃ für C₁-C₄-Alkyl oder C₃-C₅-Cycloalkyl, R₄ für Wasserstoff, Halogen oder C₁-C₃-Alkyl, n für die Zahlen 1 oder 2, Z für -NH-CS-NH-, -N=C(SR₅)-NH- oder -N=C=N- und R₅ für C₁-C₃-Alkyl stehen.

22. Schädlingsbekämpfungsmittel gemäss Anspruch 21, welches als aktive Komponente mindestens eine Verbindung der Formel enthält, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für -NH-CS-NH- stehen.

23. Schädlingsbekämpfungsmittel gemäss Anspruch 21, welches als aktive Komponente mindestens eine Verbindung der Formel enthält, worin R₁ für C₅-C₆-Cycloalkyl, R₂ für Aethyl oder Isopropyl, R₃ für Isopropyl, tert.Butyl odre Cyclopentyl, R₄ für Wasserstoff oder Fluor, n für die Zahlen 1 oder 2, Z für -N=C(SR5)-NH- und R₅ für Methyl oder Aethyl stehen.

24. Schädlingsbekämpfungsmittel gemäss Anspruch 21, welches als aktive Komponente mindestens eine Verbindung der Formel enthält, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für-N=C=N- stehen.

25. Verwendung einer Verbindung der Formel I 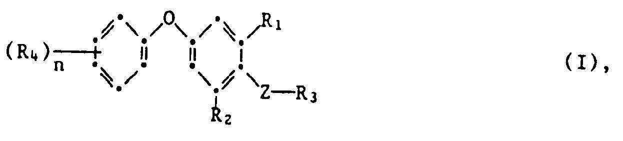
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, oder eines ihrer Salze mit einer organischen oder anorganischen Säure zur Bekämpfung von Schädlingen an Tieren und Pflanzen.

26. Verwendung gemäss Anspruch 25 einer Verbindung der Formel zur Bekämpfung von Insekten und Arachniden.

27. Verfahren zur Bekämpfung von Schädlingen an Tieren und Pflanzen, dadurch gekennzeichnet, dass man die Schädlinge in ihren verschiedenen Entwicklungsstadien mit einer Verbindung der Formel I 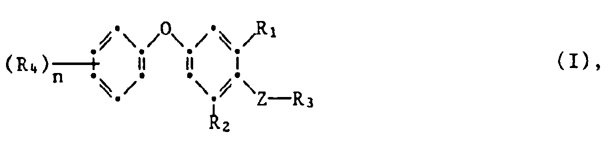
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, oder einem ihrer Salze mit einer organischen oder anorganischen Säure in Kontakt bringt.

## Patentansprüche (Patentansprüche für folgende(n) Vertragsstaat(en) : ES)

1. Verfahren zur Herstellung einer Verbindung der Formel I 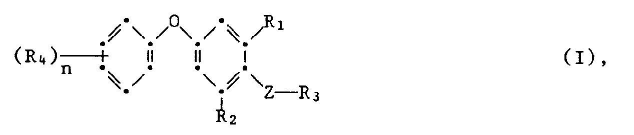
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl, R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, dadurch gekennzeichnet, dass man
A) ein Isothiocyanat der Formel II 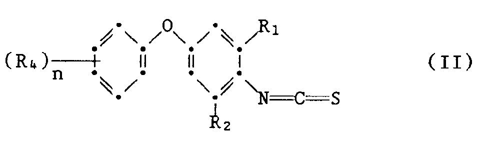 mit einem Amin der Formel III 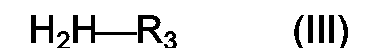
in einem organischen Lösungs- oder Verdünnungsmittel bei Normaldruck und einer Temperatur von 0 bis +150_{°}C zum Thioharnstoff umsetzt und gegebenenfalls
B) den erhaltenen Thioharnstoff mit einer Verbindung der Formel IV 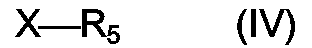
in einem inerten organischen Lösungsmittel unter leicht erhöhtem oder normalem Druck und bei einer Temperatur von +10 bis 250°C zum Isothioharnstoff umsetzt oder
C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150_{°}C in das Carbodiimid überführt, worin R_{1'} R₂, R₃, R₄, R₅ und n die angegebene Bedeutung haben und X für eine Abgangsgruppe steht.

2. Verfahren gemäss Anspruch 1, worin R₁ für C₃-C₇-Cycloalkyl, R₂ für C₁-C₄-Alkyl oder Cyclopentyl, R₃ für C₁-C₄-Alkyl oder C₃-C₅-Cycloalkyl, R₄ für Wasserstoff, Halogen oder C₁-C₃-Alkyl, n für die Zahlen 1 oder 2, Z für -NH-CS-NH-, -N=C(SR₅)-NH- oder -N=C=N- und R₅ für C₁-C₃-Alkyl stehen.

3. Verfahren gemäss Anspruch 2, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für -NH-CS-NH- stehen.

4. Verfahren gemäss Anspruch 2, worin R₁ für C₅-C₆-Cycloalkyl, R₂ für Aethyl oder Isopropyl, R₃ für Isopropyl, tert.Butyl oder cyclopentyl, R₄ für Wasserstoff oder Fluor, n fürdie Zahlen 1 oder 2,Zfür-N=C(SR₅)-NH-und R₅ für Methyl oder Aethyl stehen.

5. Verfahren gemäss Anspruch 2, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für -N=C=N-stehen.

6. Verfahren gemäss einem der Ansprüche 1 oder 3 zur Herstellung der Verbindungen der Formeln

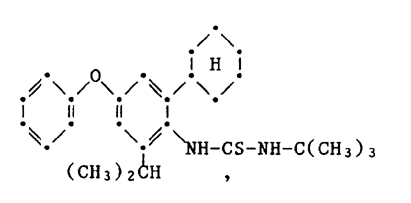

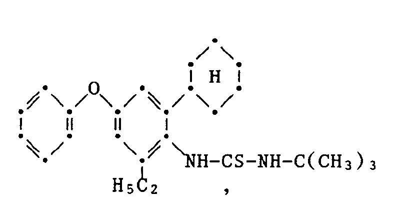

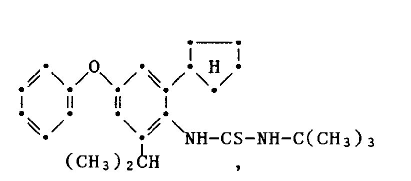

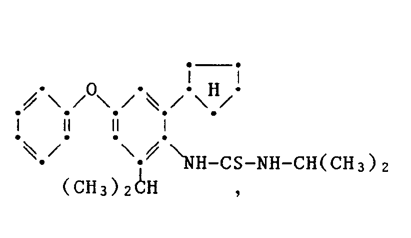

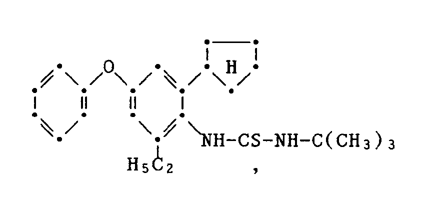

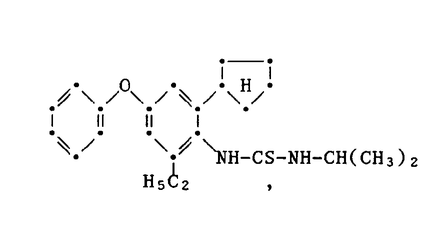

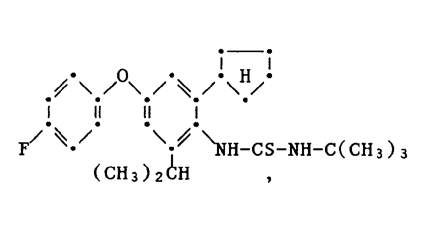

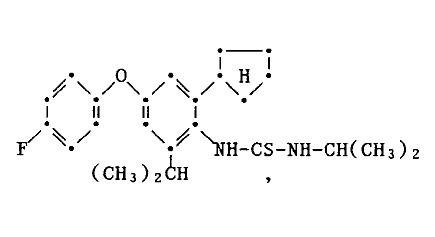

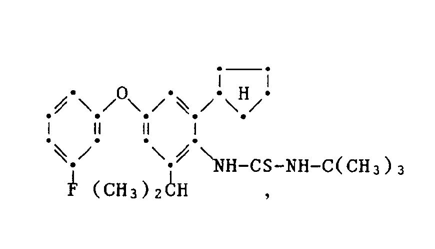

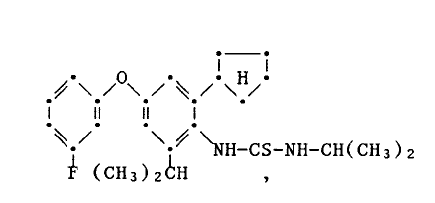

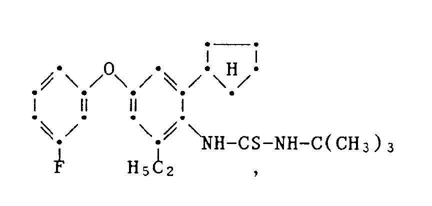

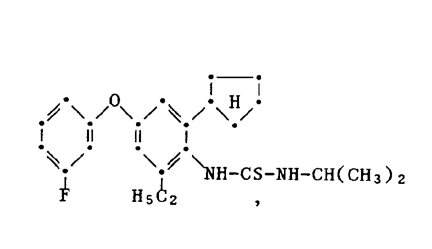

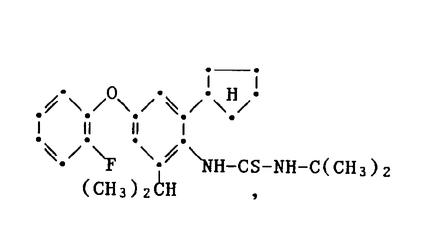

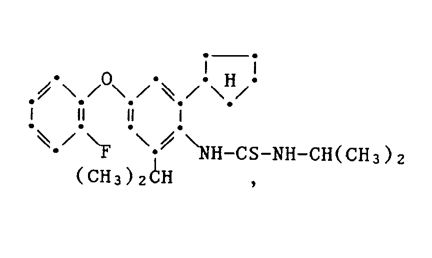

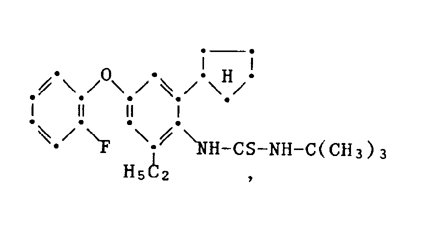

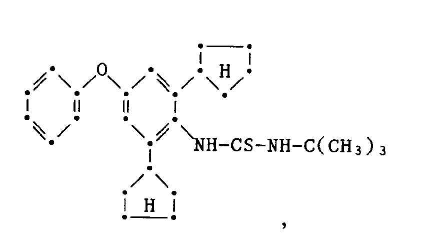

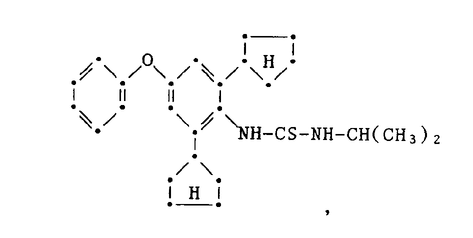

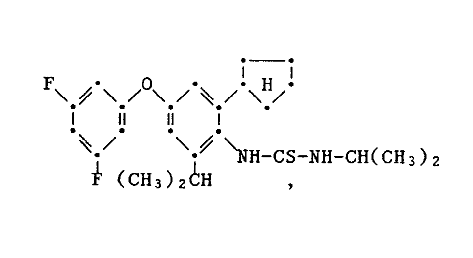

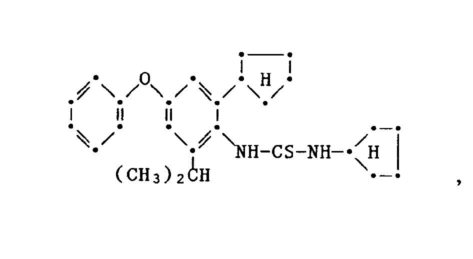

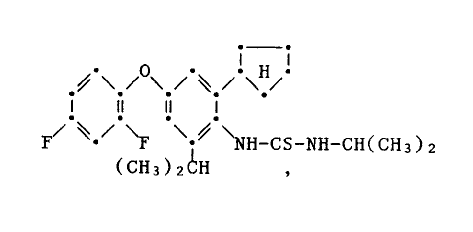

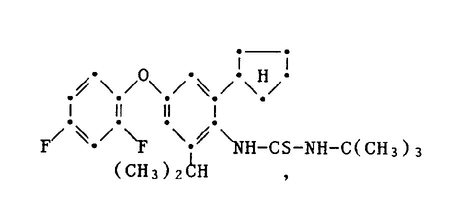

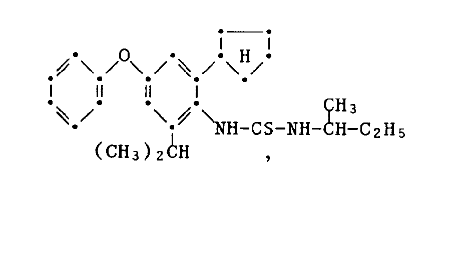

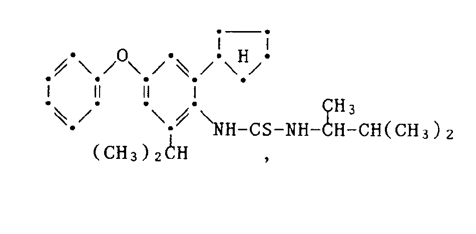

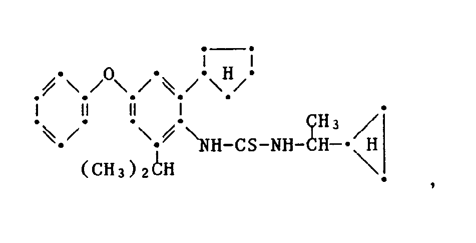

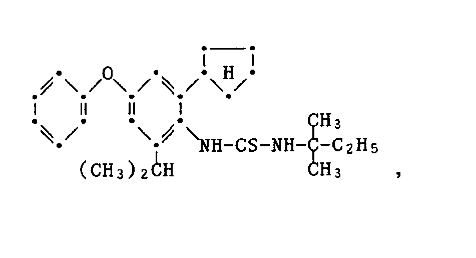

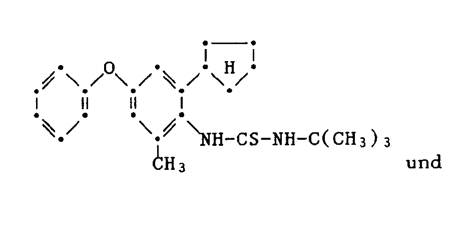

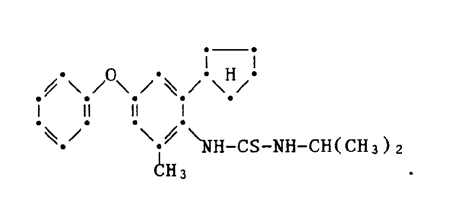

7. Verfahren gemäss einem der Ansprüche 1 oder 4 zur Herstellung der Verbindungen der Formeln

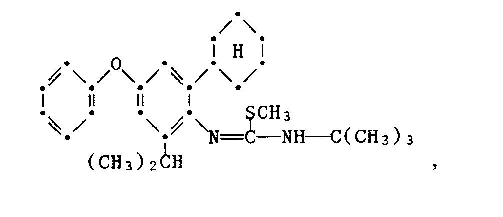

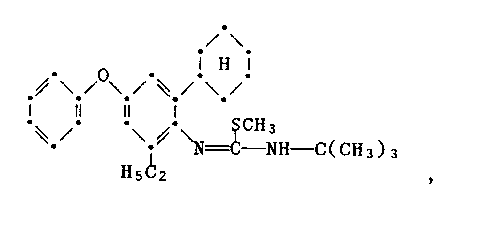

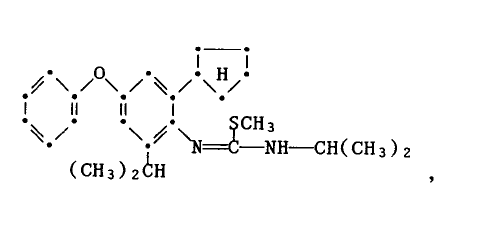

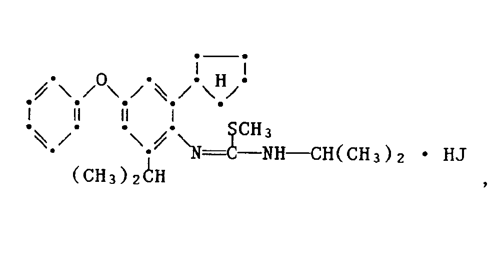

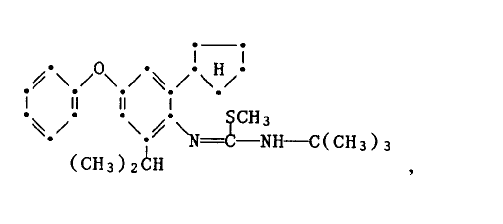

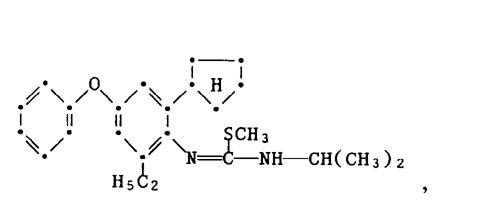

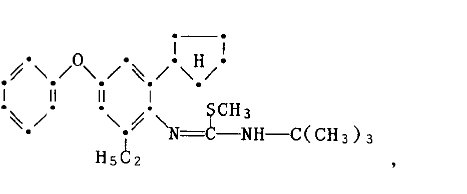

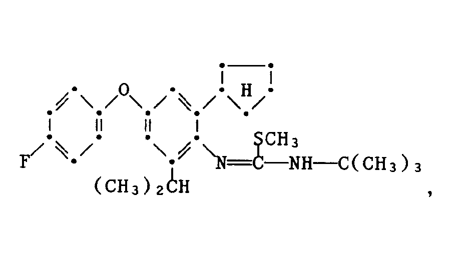

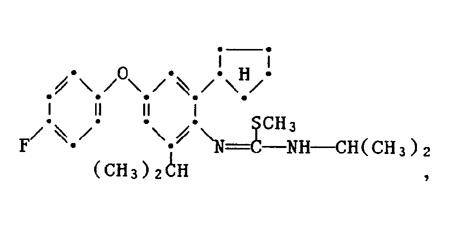

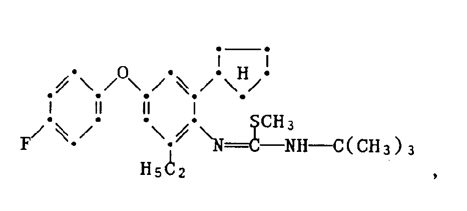

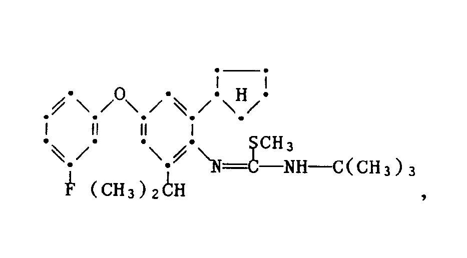

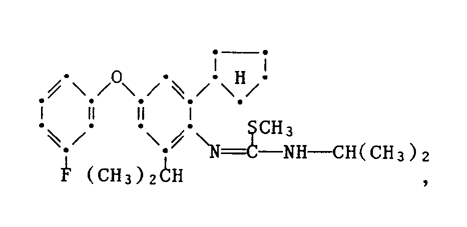

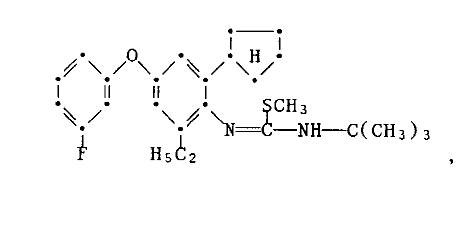

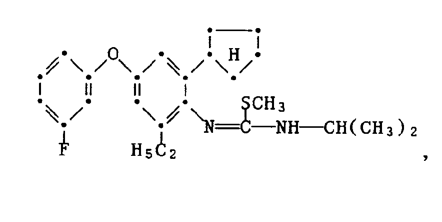

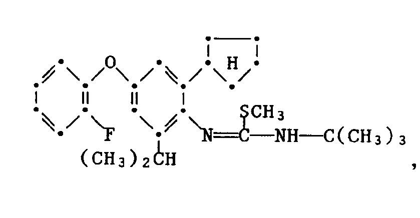

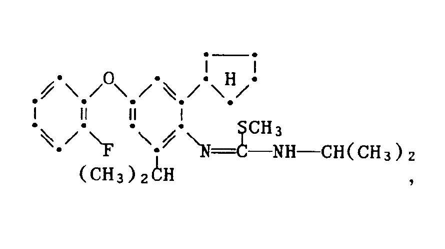

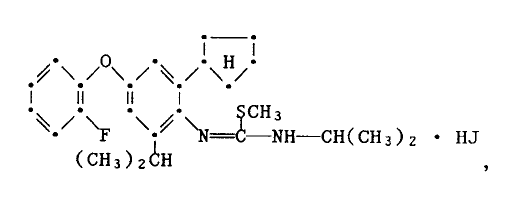

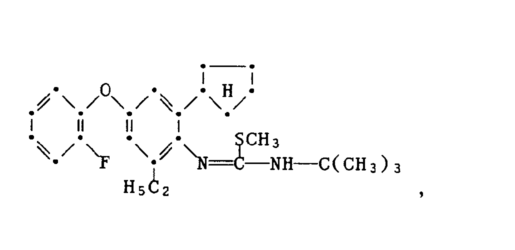

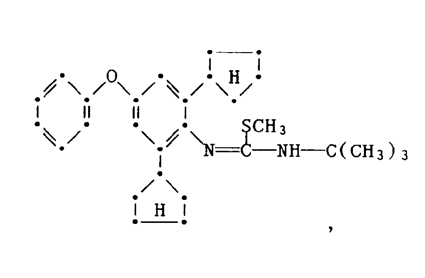

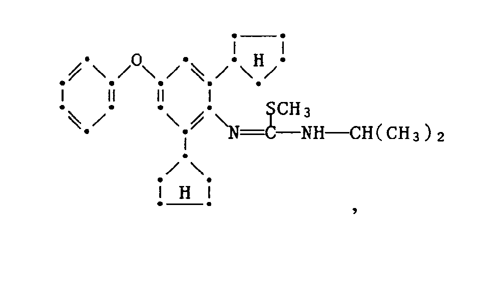

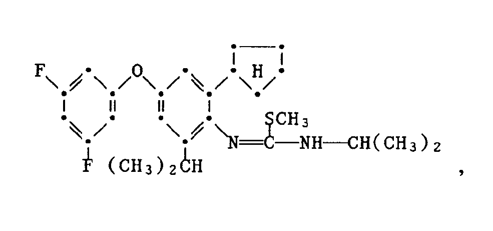

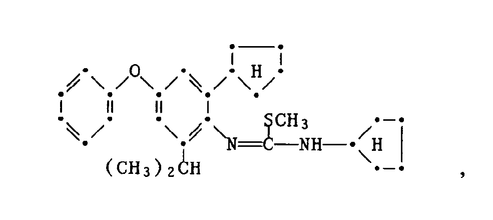

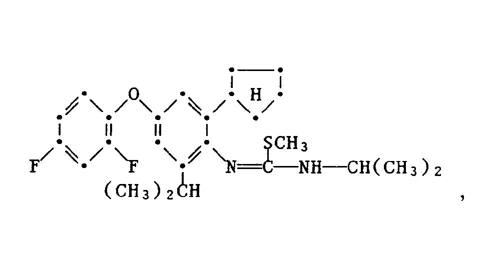

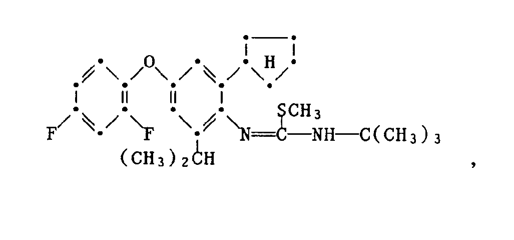

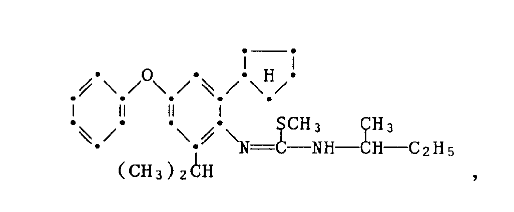

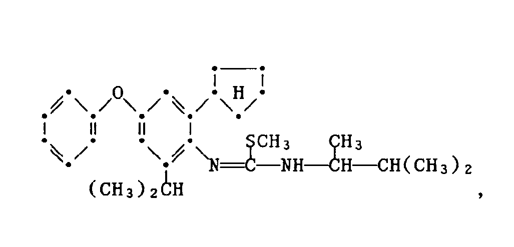

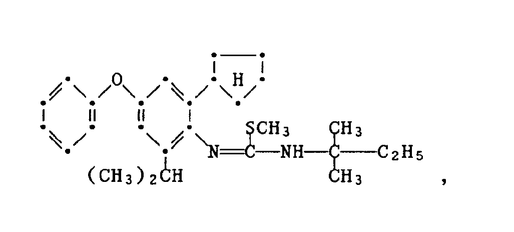

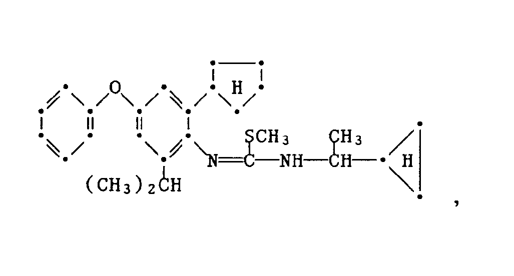

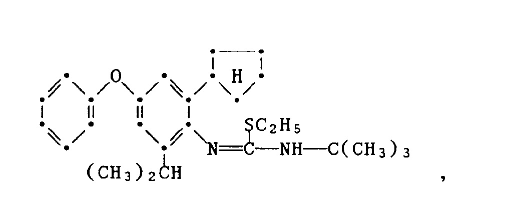

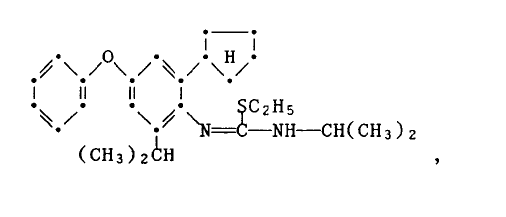

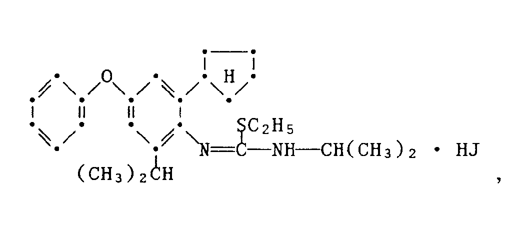

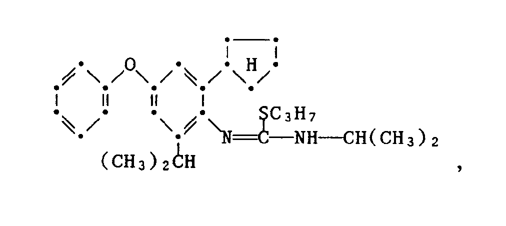

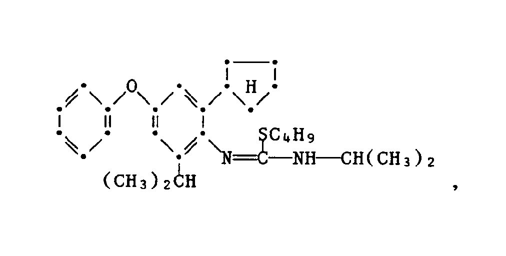

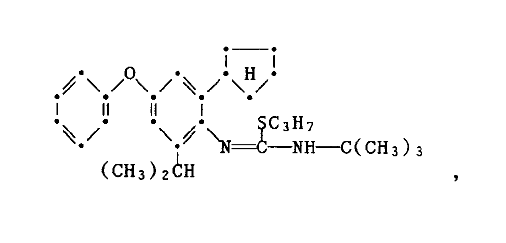

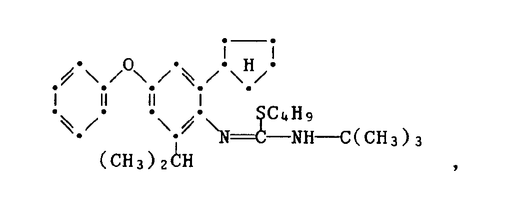

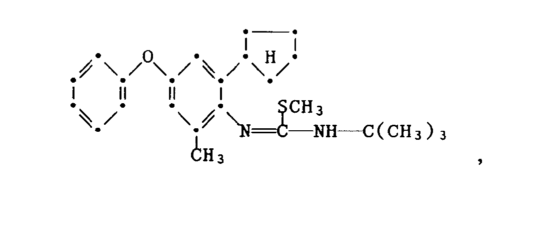

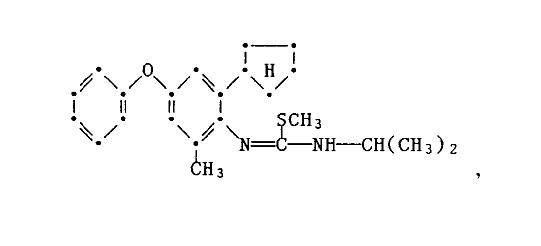

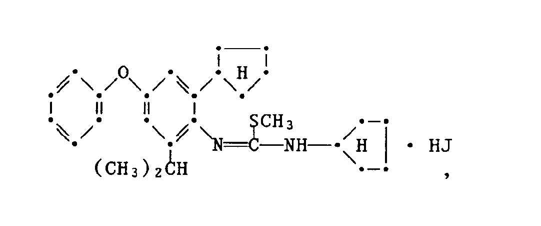

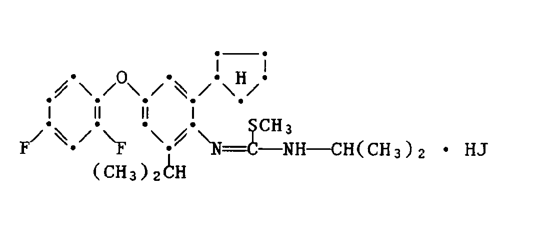

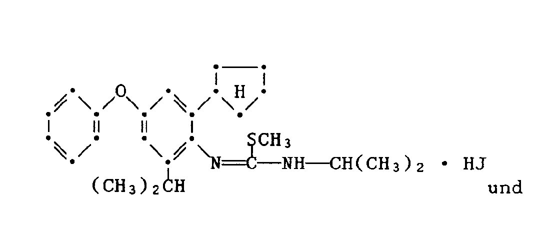

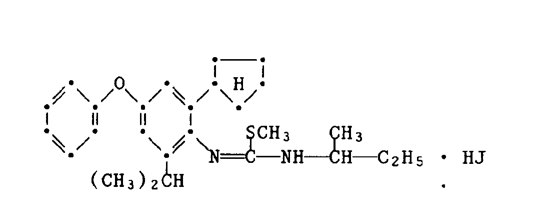

8. Verfahren gemäss einem der Ansprüche 1 oder 5 zur Herstellung der Verbindungen der Formeln 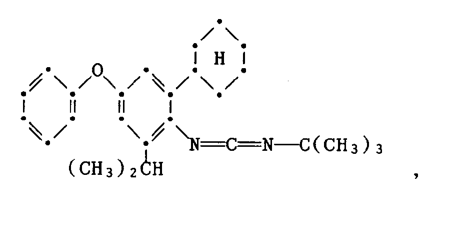 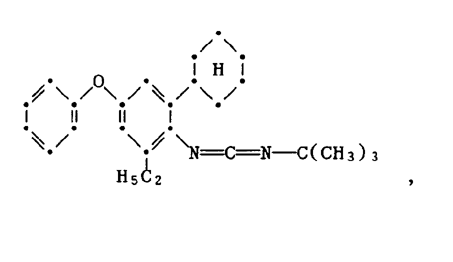 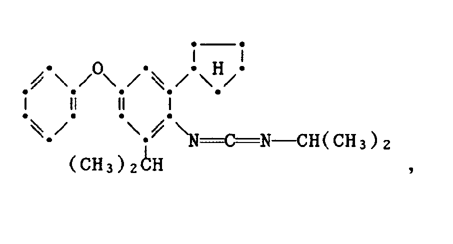 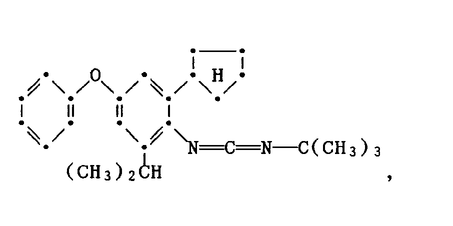 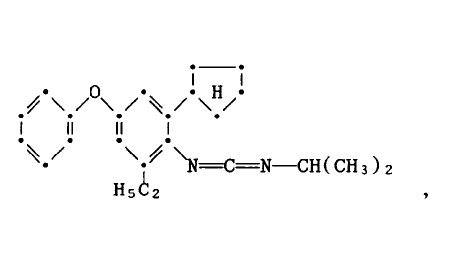 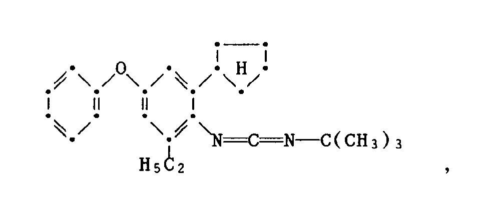 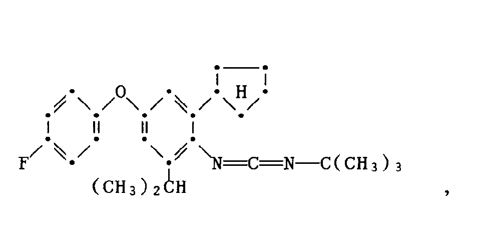 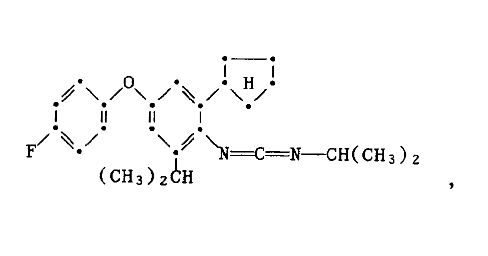 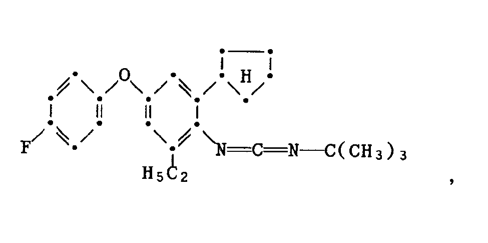 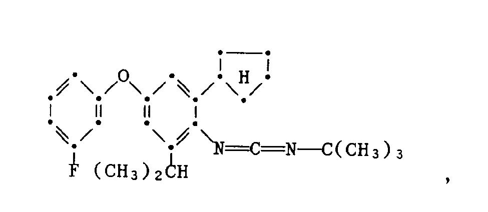 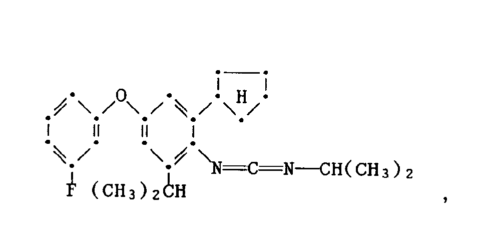 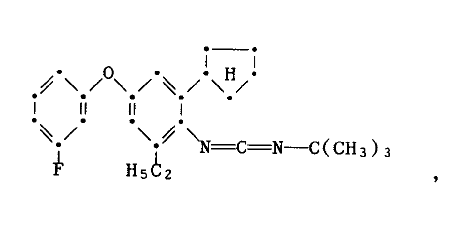 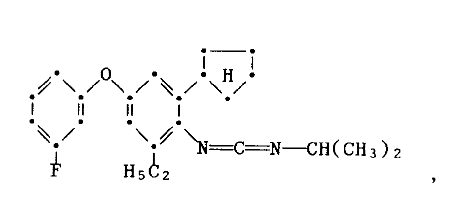 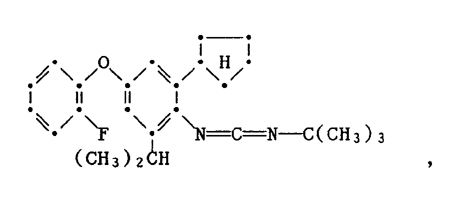 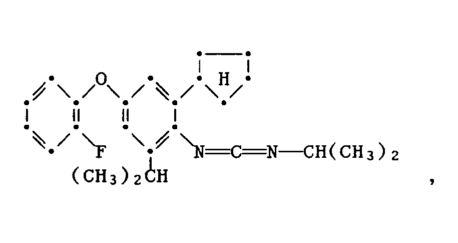 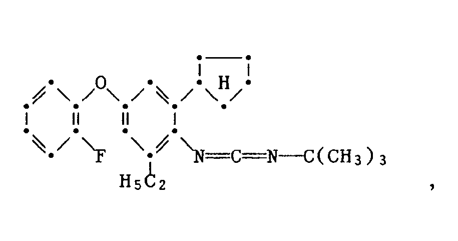 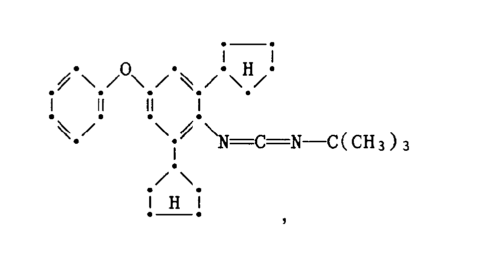 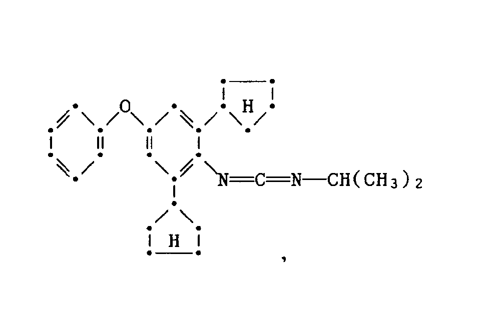 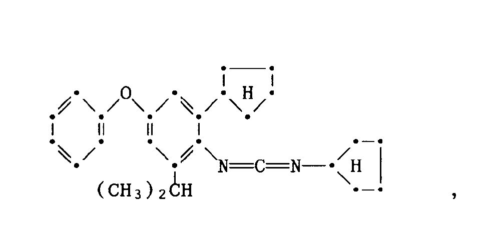 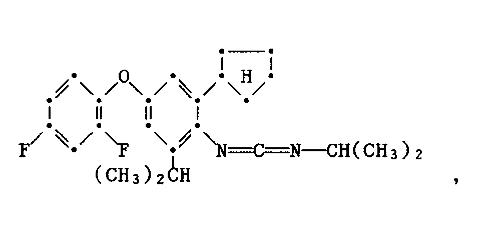 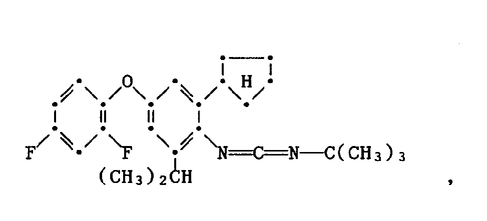 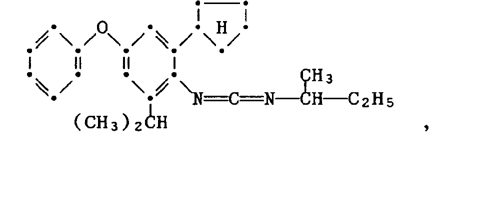 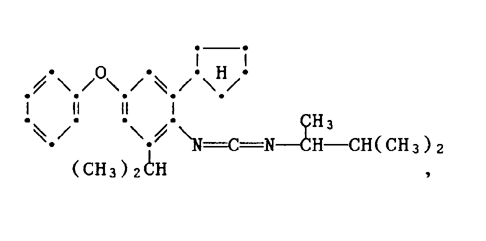 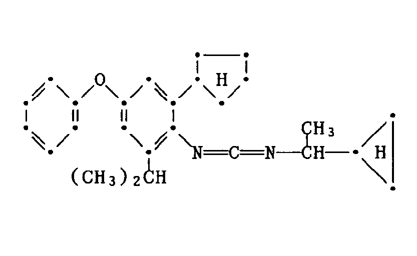 und

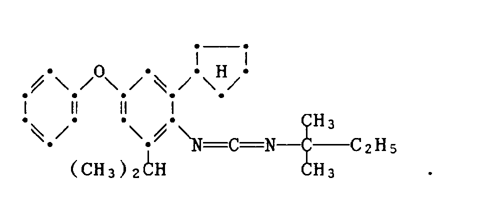

9. Verfahren zur Herstellung einer Verbindung der Formel V 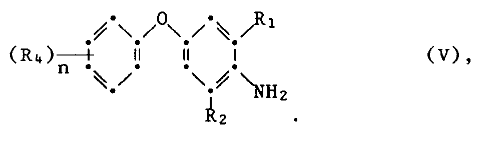
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₄ je für Wasserstoff, Halogen, C₁-C₄-Alkyl, C₁-C₃-Alkoxy oder CF₃ und
n für die Zahlen 1 bis 3 stehen, dadurch gekennzeichnet, dass man ein Phenol der Formel VII 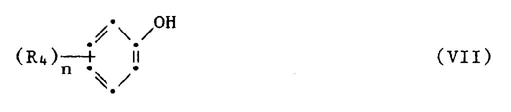 in Gegenwart einer organischen oder anorganischen Base, unter normalem Druck und bei einer Temperatur von 0 bis +100_{°}C in einem inerten Lösungs- oder Verdünnungsmittel mit einem Anilin der Formel VI 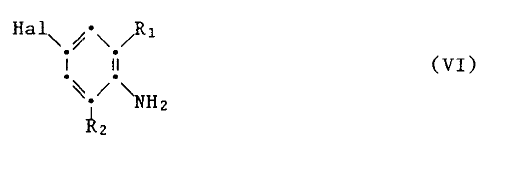 umsetzt, worin R_{1'} R₂, R₄ und n die angegebene Bedeutung haben und Hal für ein Halogenatom steht.

10. Verfahren gemäss Anspruch 9, worin R₁ für C₃-C₇-Cycloalkyl, R₂ für C₁-C₄-Alkyl oder Cyclopentyl, R₄ für Wasserstoff, Halogen oder C₁-C₃-Alkyl und n für die Zahlen 1 oder 2 stehen.

11. Verfahren gemäss Anspruch 10, worin R₁ für C₅-Cₛ-Cycloalkyl, R₂ fürAethyl oder Isopropyl, R₄ für Wasserstoff oder Fluor und n für die Zahlen 1 oder 2 stehen.

12. Verfahren gemäss einem der Ansprüche 10 oder 11 zur Herstellung der Verbindungen der Formeln

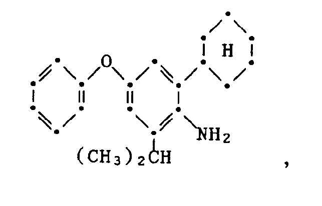

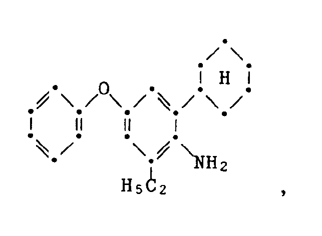

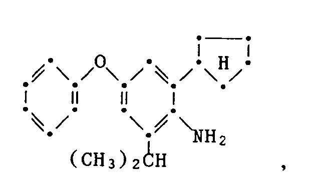

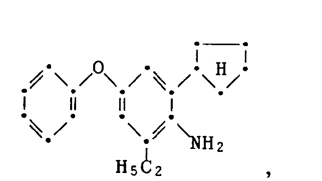

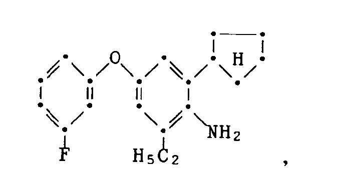

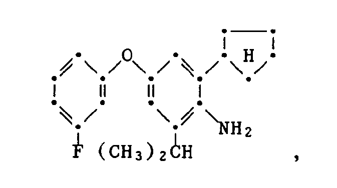

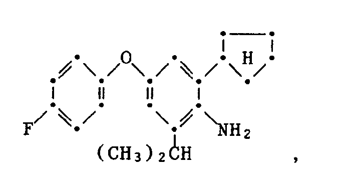

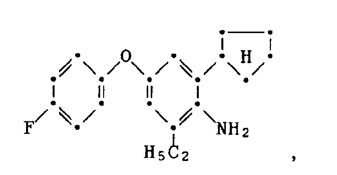

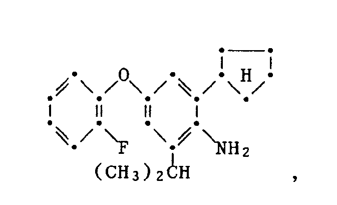

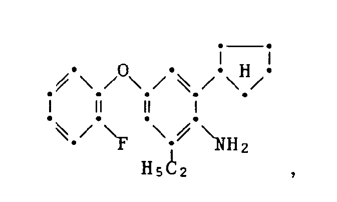

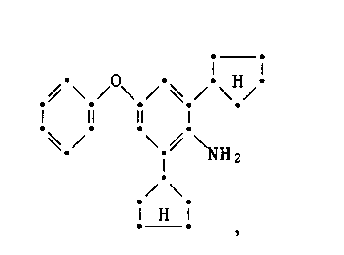

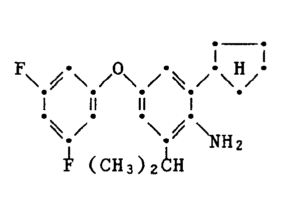

und

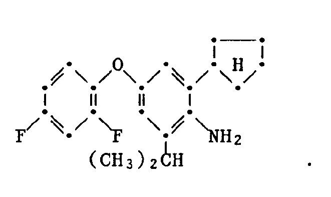

13. Verfahren zur Herstellung einer Verbindung der Formel II 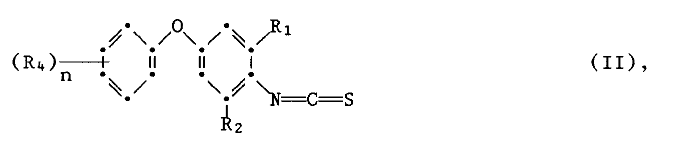
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₄ je für Wasserstoff, Halogen, C₁-C₄-Alkyl, C₁-C₃-Alkoxy oder CF₃ und
n für die Zahlen 1 bis 3 stehen, dadurch gekennzeichnet, dass man ein Phenoxyanilin der Formel V 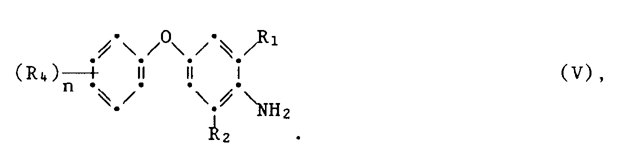 in Gegenwart einer organischen oder anorganischen Base, unter normalem Druck und bei einer Temperatur von 0 bis +100_{°}C in einem inerten Lösungs- oder Verdünnungsmittel mit Thiophosgen umsetzt, worin R_{1'} R₂, R₄ und n die angegebene Bedeutung haben.

14. Verfahren gemäss Anspruch 13, worin R₁ für C₃-C₇-Cycloalkyl, R₂ für C₁-C₄-Alkyl, R₄ für Wasserstoff, Halogen oder C₁-C₃-Alkyl und n für die Zahlen 1 oder 2 stehen.

15. Verfahren gemäss Anspruch 14, worin R₁ für C₅-C₆-Cycloalkyl, R₂ fürAethyl oder Isopropyl, R₄ für Wasserstoff oder Fluor und n für die Zahlen 1 oder 2 stehen.

16. Verfahren gemäss einem der Ansprüche 14 oder 15 zur Herstellung der Verbindungen der Formeln

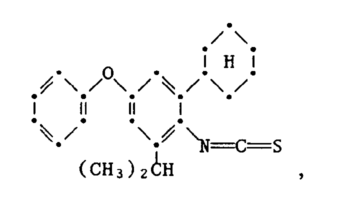

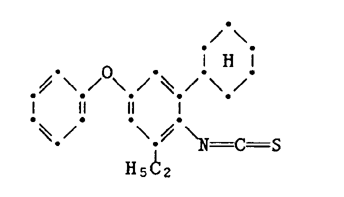

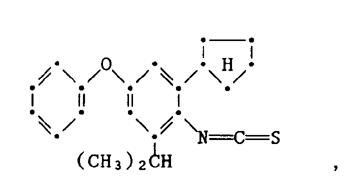

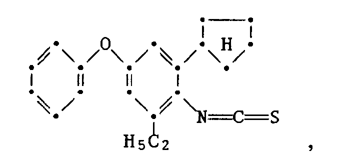

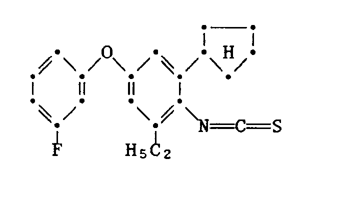

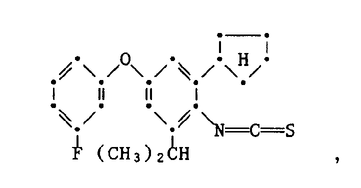

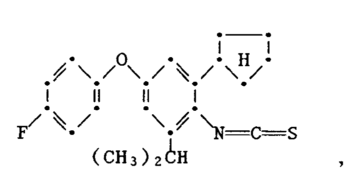

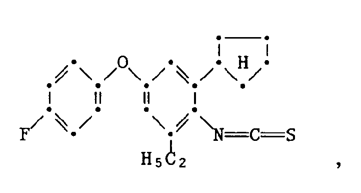

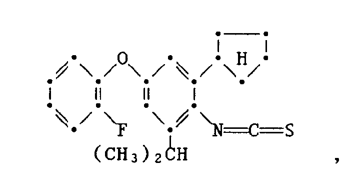

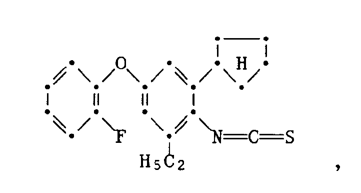

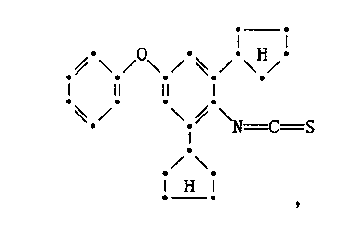

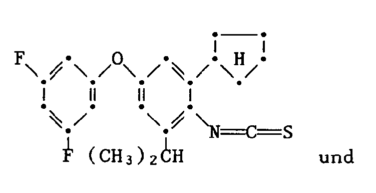

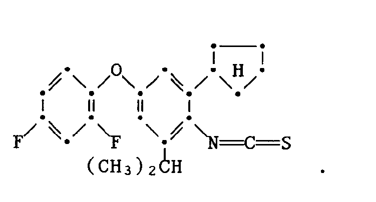

17. Schädlingsbekämpfungsmittel, welches als aktive Komponente mindestens eine Verbindung der Formel I 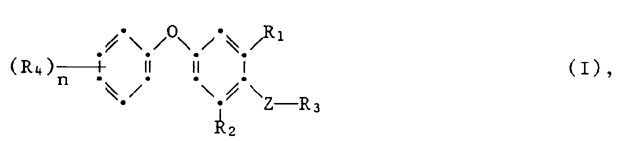
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, oder eines ihrer Salze mit einer organischen oder anorganischen Säure zusammen mit geeigneten Trägern und/oder Zuschlagstoffen enthält.

18. Schädlingsbekämpfungsmittel gemäss Anspruch 17, welches als aktive Komponente mindestens eine Verbindung der Formel enthält, worin R₁ für C₃-C₇-Cycloalkyl, R₂ für C₁-C₄-Alkyl oder Cyclopentyl, R₃ für C₁-C₄-Alkyl oder C₃-C₅-Cycloalkyl, R₄ für Wasserstoff, Halogen oder C₁-C₃-Alkyl, n für die Zahlen 1 oder 2, Z für -NH-CS-NH-, -N=C(SR₅)-NH- oder -N=C=N- und R₅ für C₁-C₃-Alkyl stehen.

19. Schädlingsbekämpfungsmittel gemäss Anspruch 18, welches als aktive Komponente mindestens eine Verbindung der Formel enthält, worin R₁ für C₅-Cₛ-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für -NH-CS-NH- stehen.

20. Schädlingsbekämpfungsmittel gemäss Anspruch 18, welches als aktive Komponente mindestens eine Verbindung der Formel enthält, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2, Z für -N=C(SR₅)-NH- und R₅ für Methyl oder Aethyl stehen.

21. Schädlingsbekämpfungsmittel gemäss Anspruch 18, welches als aktive Komponente mindestens eine Verbindung der Formel enthält, worin R₁ für C₅-C₆-Cycloalkyl, R₂ für Aethyl oder Isopropyl, R₃ für Isopropyl, tert.Butyl oder Cyclopentyl, R₄ für Wasserstoff oder Fluor, n für die Zahlen 1 oder 2 und Z für-N=C=N- stehen.

22. Verwendung einer Verbindung der Formel I 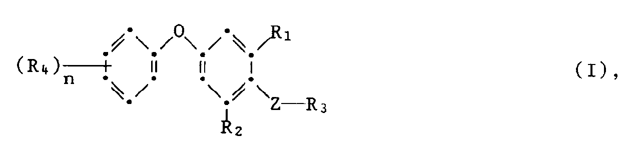
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, oder eines ihrer Salze mit einer organischen oder anorganischen Säure zur Bekämpfung von Schädlingen an Tieren und Pflanzen.

23. Verwendung gemäss Anspruch 22 einer Verbindung der Formel zur Bekämpfung von Insekten und Arachniden.

24. Verfahren zur Bekämpfung von Schädlingen an Tieren und Pflanzen, dadurch gekennzeichnet, dass man die Schädlinge in ihren verschiedenen Entwicklungsstadien mit einer Verbindung der Formel I 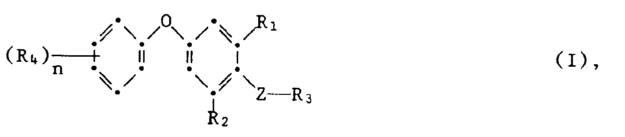
worin
R₁ für C₃-C₇-Cycloalkyl oder C₅-C₆-Cycloalkenyl,
R₂ für C₁-C₆-Alkyl, C₅-Cₛ-Cycloalkyl oder C₅-C₆-Cycloalkenyl,
R₃ für C₁-C₈-Alkyl, C₃-C₆-Cycloalkyl, 1-Cyclopropyl-äthyl oder C₃-C₅-Alkenyl,
R₄ je für Wasserstoff, Halogen, C₁-C₄-Alkyl, C₁-C₃-Alkoxy oder CF₃,
n für die Zahlen 1 bis 3,
Z für -NH-CS-NH-, -N=C(SR₅)-NH- oder -N=C=N- und
R₅ für C₁-C₅-Alkyl oder Allyl
stehen, oder einem ihrer Salze mit einer organischen oder anorganischen Säure in Kontakt bringt.

## Claims (Claims for the following Contracting State(s) : AT, BE, CH, DE, FR, GB, GR, IT, LI, NL.)

1. A compound of formula I 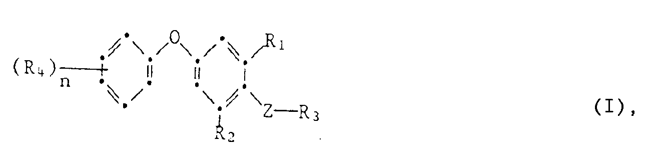
wherein
R₁ is C₃-C₇cycloalkyl or C₅-C₆cycloalkenyl,
R₂ is C₁-C₆alkyl, C₅-C₆cycloalkyl or C₅-C₆cycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl
or a salt thereof with an organic or inorganic acid.

2. A compound of formula I according to claim 1, wherein R₁ is C₃-C₇cycloalkyl, R₂ is C₁-C₄alkyl or cyclopentyl, R₃ is C₁-C₄alkyl or C₃-C₅cycloalkyl, R₄ is hydrogen, halogen or C₁-C₃alkyl, n is the number 1 or 2, Z is -NH-CS-NH-, -N=C(SR₅)-NH- or -N=C=N-, and R₅ is C₁-C₃alkyl.

3. A compound of formula I according to claim 2, wherein R₁ is C₅-Cₛcycloalkyl, R₂ is ethyl or isopropyl, R₃ is isopropyl, tert-butyl or cyclopentyl, R₄ is hydrogen or fluorine, n is the number 1 or 2, and Z is -NH-CS-NH-.

4. A compound of formula I according to claim 2, wherein R₁ is C₅-Cₛcycloalkyl, R₂ is ethyl or isopropyl, R₃ is isopropyl, tert-butyl or cyclopentyl, R₄ is hydrogen or fluorine, n is the number 1 or 2, Z is -N=C(SR₅)-NH-, and R₅ is methyl or ethyl.

5. A compound of formula I according to claim 2, wherein R₁ is C₅-Cₛcycloalkyl, R₂ is ethyl or isopropyl, R₃ is isopropyl, tert-butyl or cyclopentyl, R₄ is hydrogen or fluorine, n is the number 1 or 2, and Z is -N=C=N-.

6. A compound according to either claim 1 or claim 3 of formula

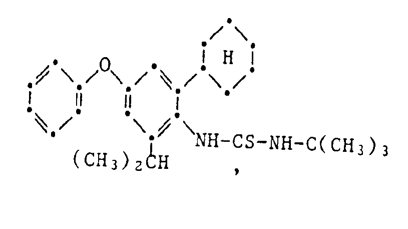

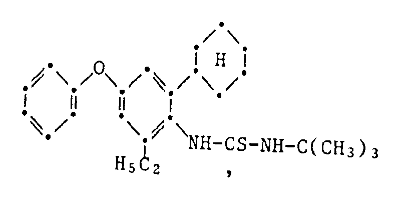

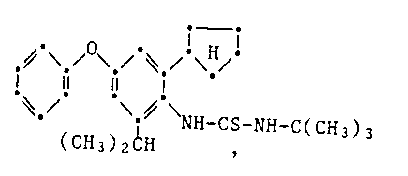

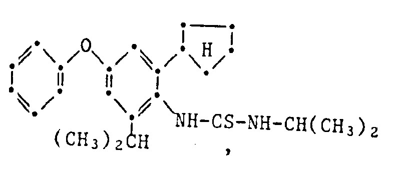

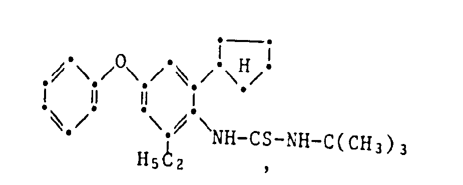

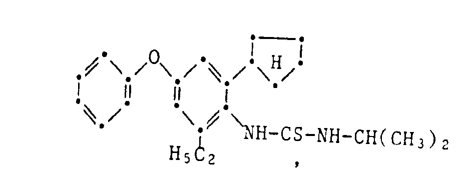

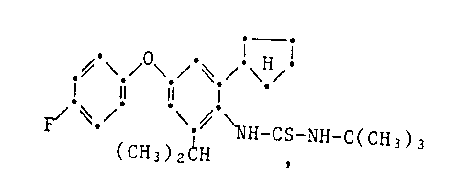

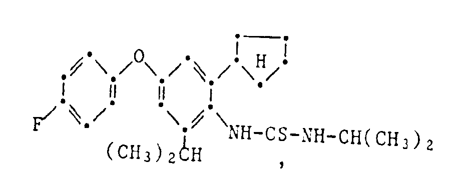

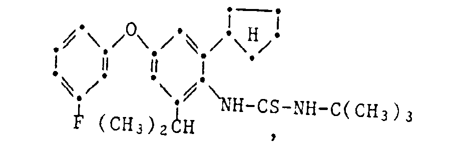

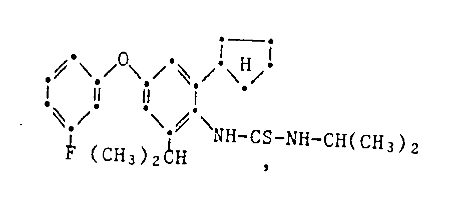

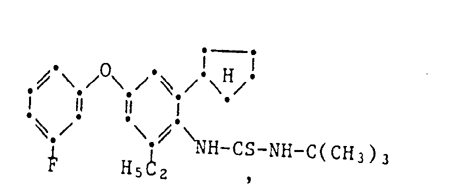

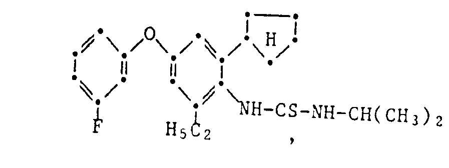

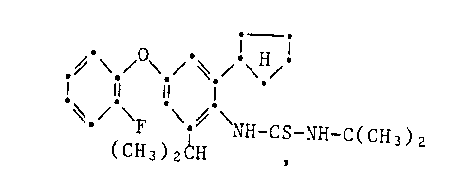

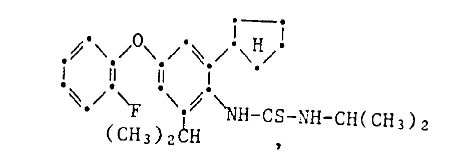

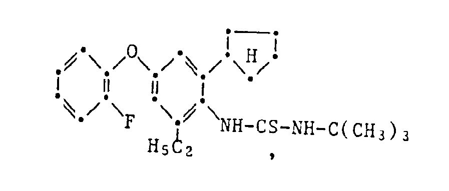

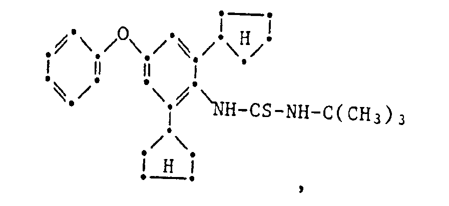

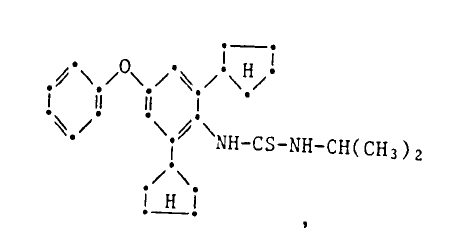

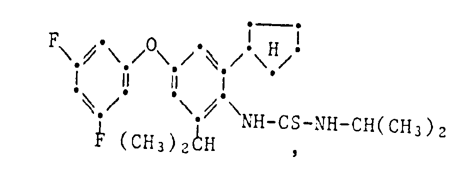

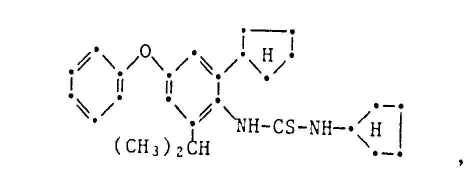

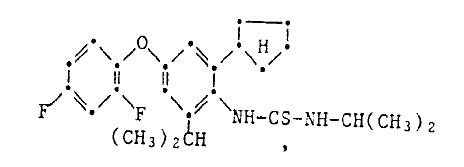

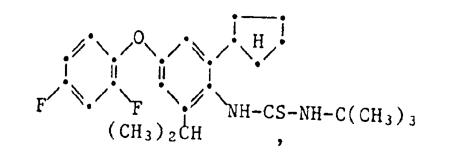

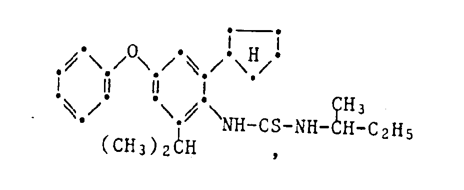

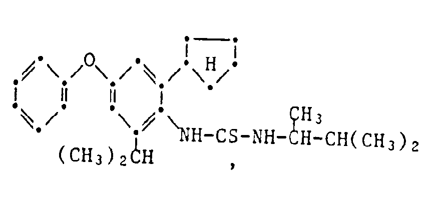

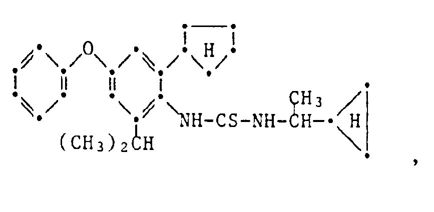

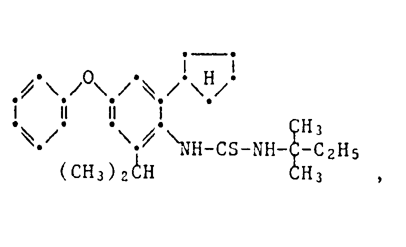

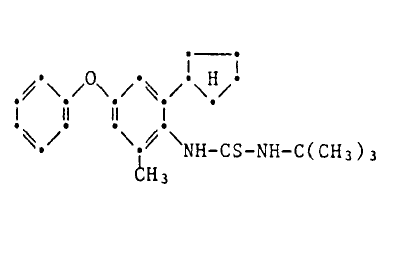

or

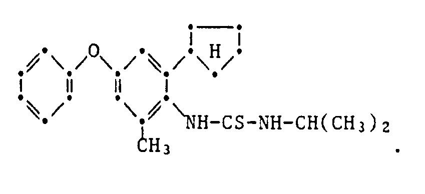

7. A compound according to either claim 1 or claim 4 of formula

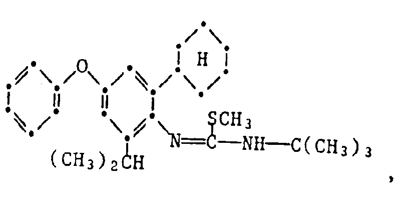

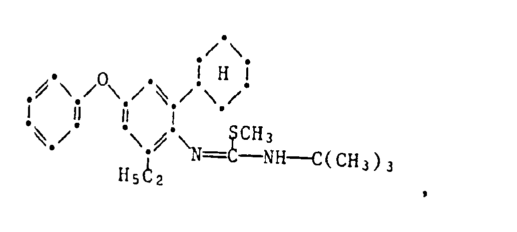

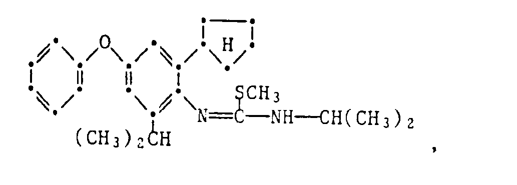

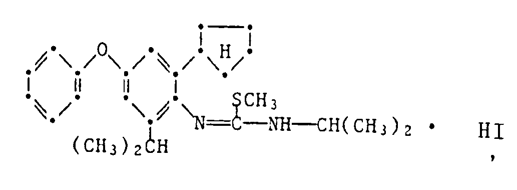

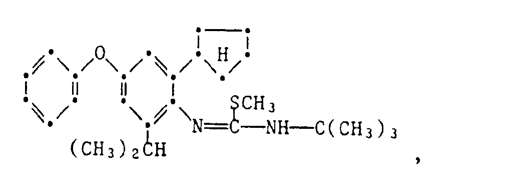

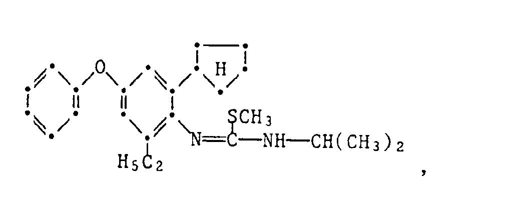

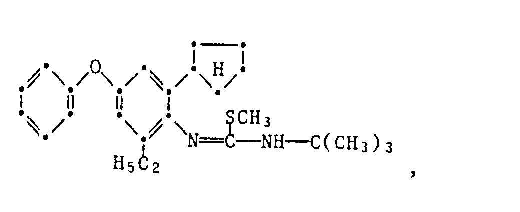

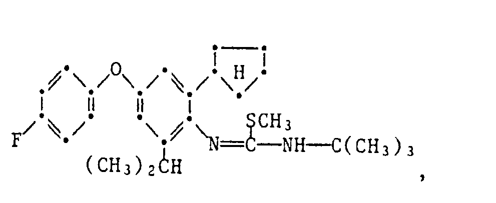

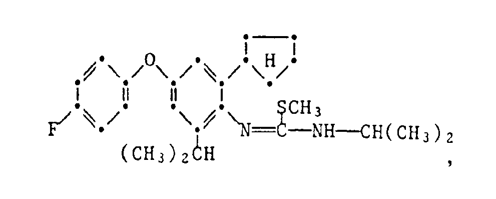

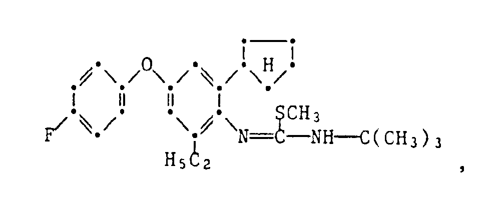

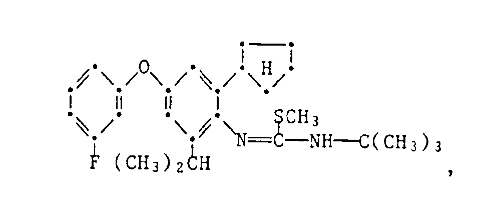

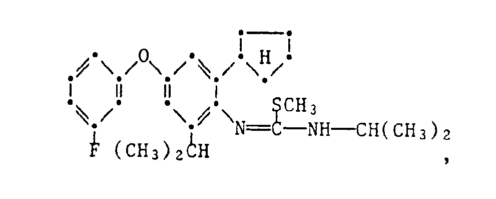

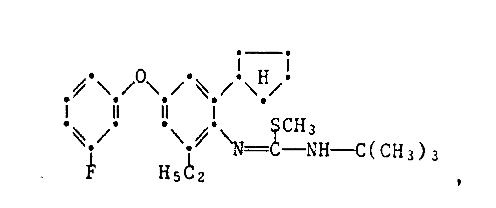

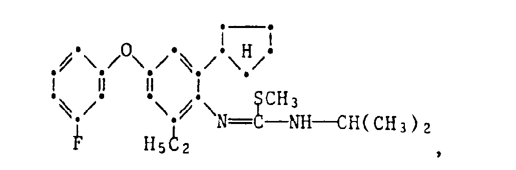

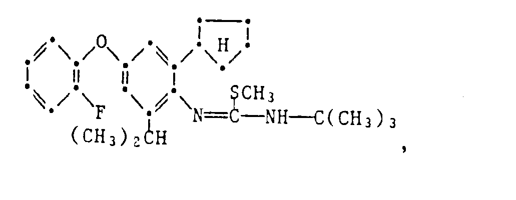

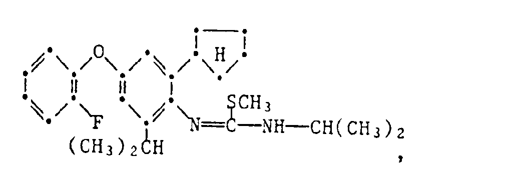

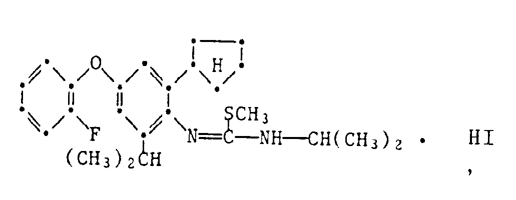

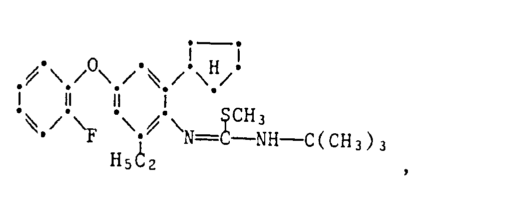

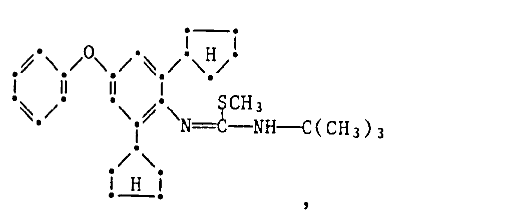

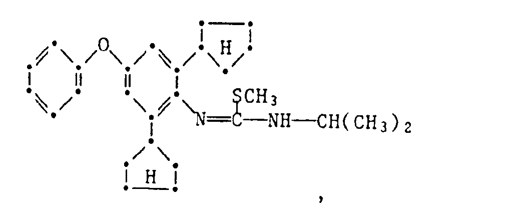

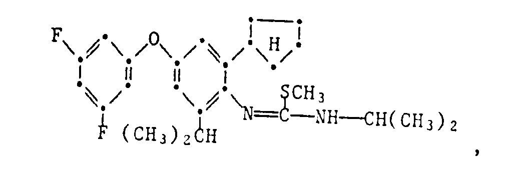

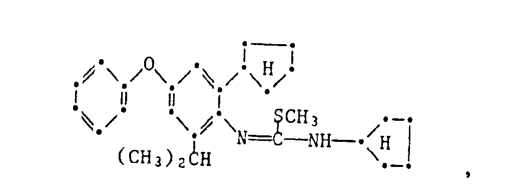

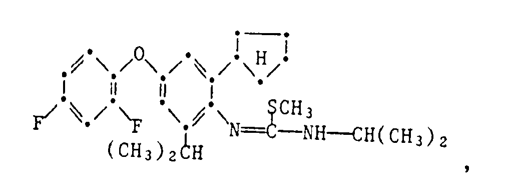

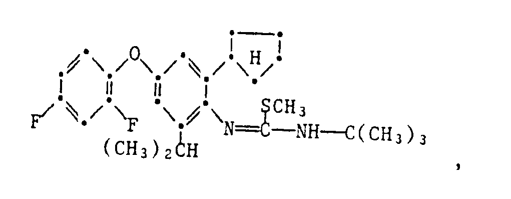

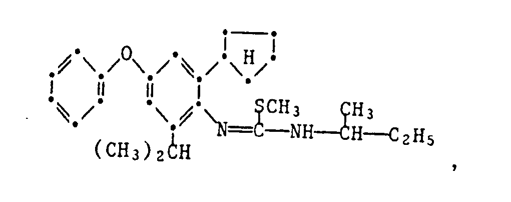

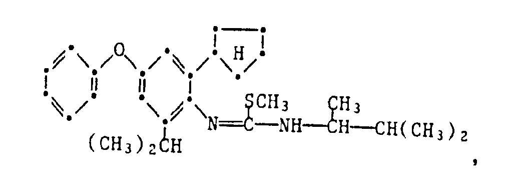

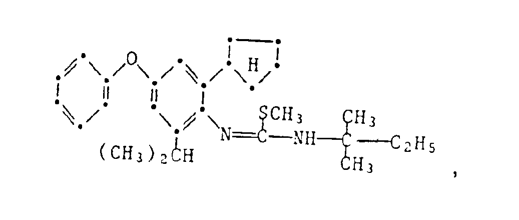

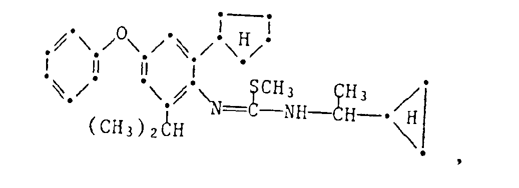

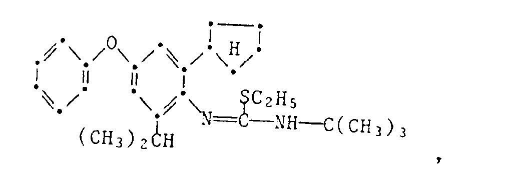

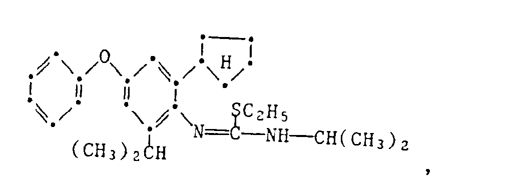

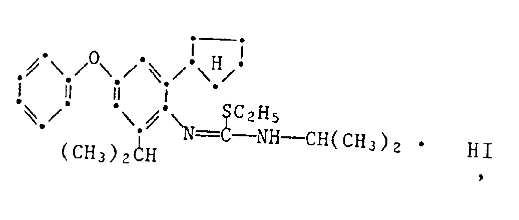

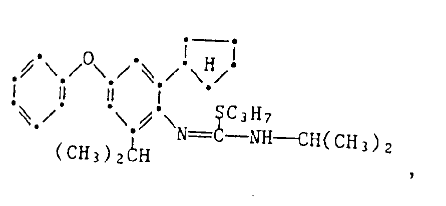

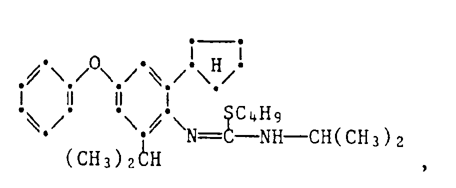

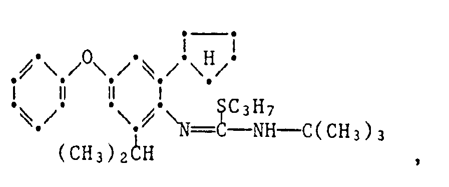

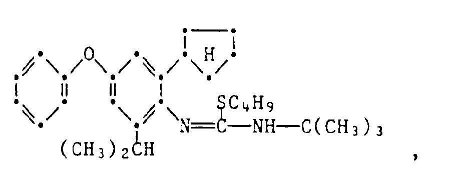

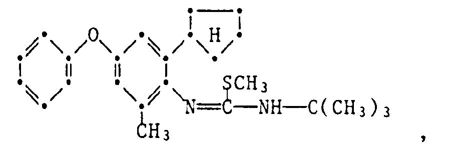

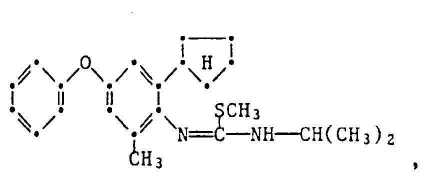

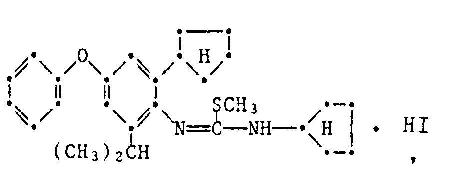

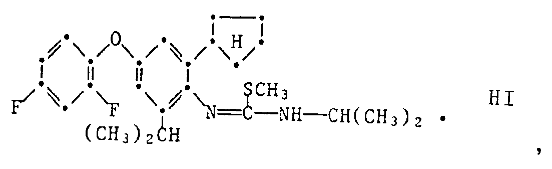

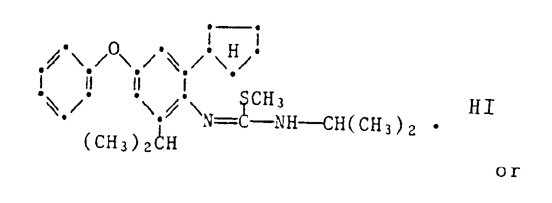

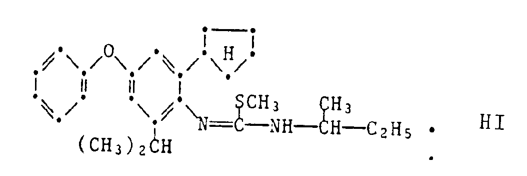

8. The compound according to either claim 1 or claim 5 of formula

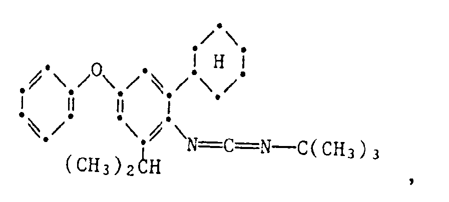

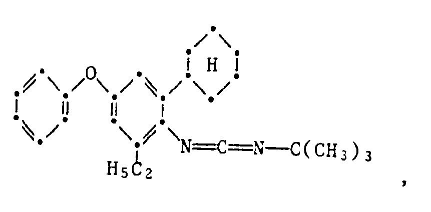

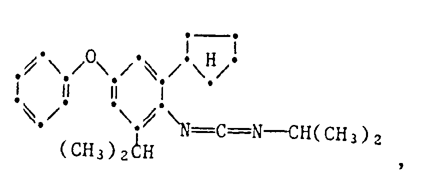

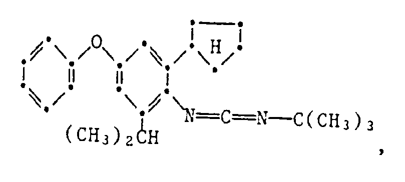

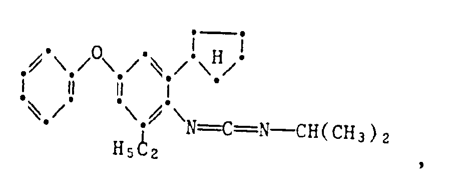

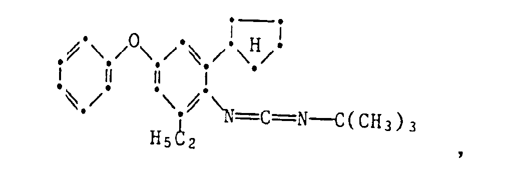

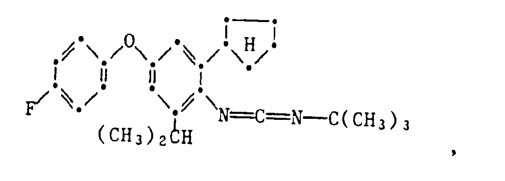

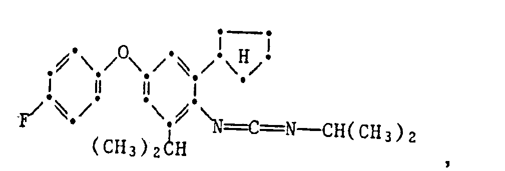

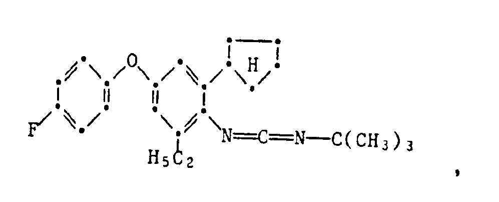

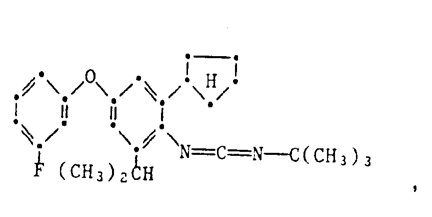

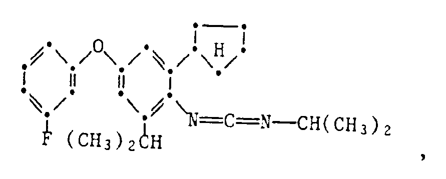

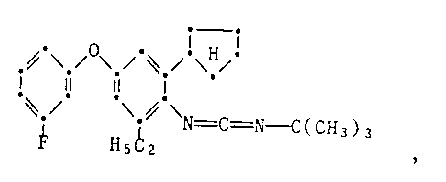

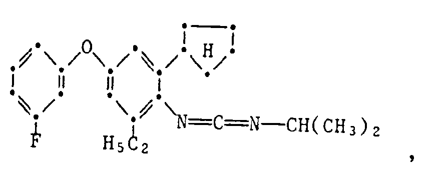

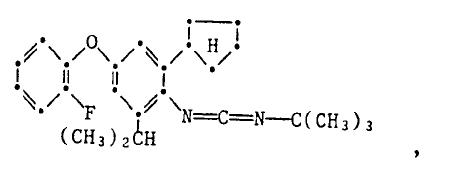

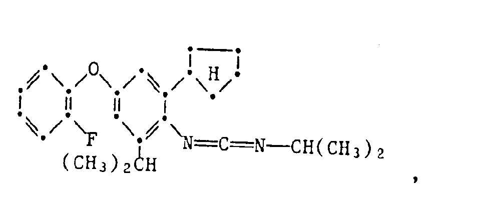

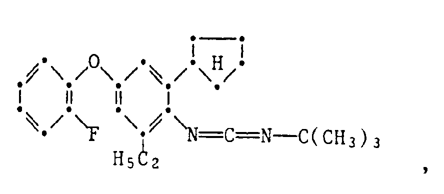

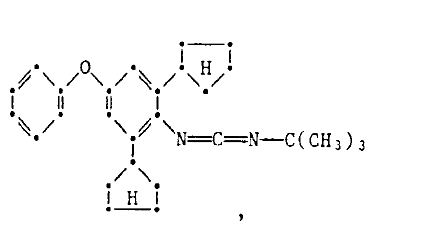

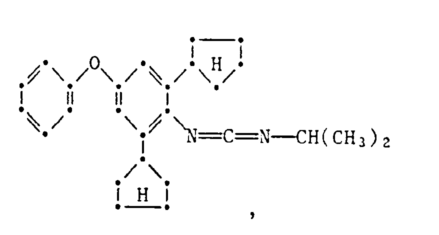

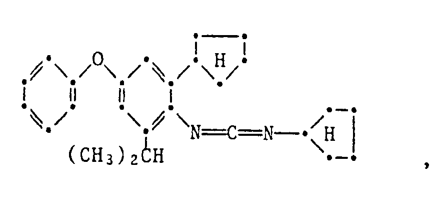

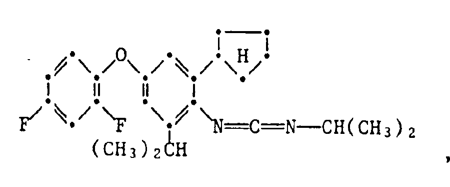

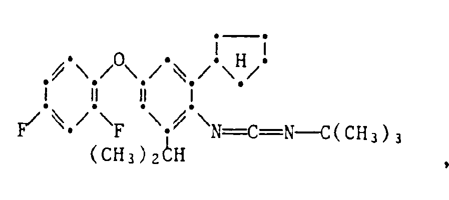

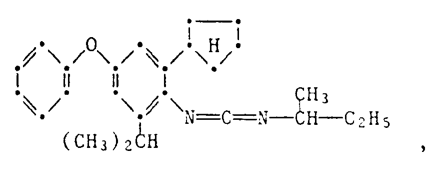

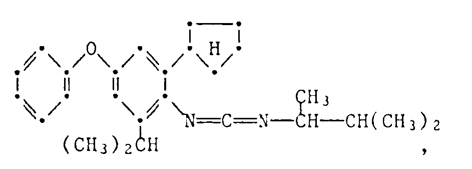

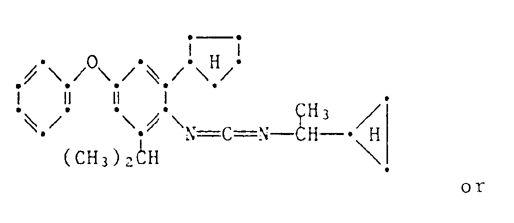

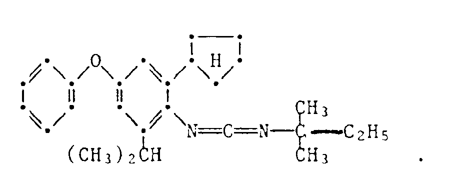

9. A compound of formula V 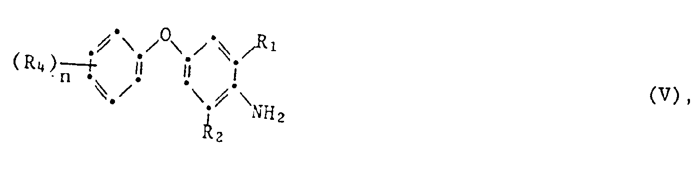
wherein
R₁ is C₃-C₇cycloalkyl or C₅-C₆cycloalkenyl,
R₂ is C₁-C₆alkyl, C₅-C₆cycloalkyl or C₅-C₆cycloalkenyl,
R₄ is in each case hydrogen, halogen, C₁-C₄alkyl, C₁-C₃alkoxy or CF₃, and
n is a number from 1 to 3.

10. A compound of formula V according to claim 9, wherein R₁ is C₃-C₇cycloalkyl, R₂ is C₁-C₄alkyl or cyclopentyl, R₄ is hydrogen, halogen or C₁-C₃alkyl and n is the number 1 or 2.

11. A compound of formula V according to claim 10, wherein R₁ is C₅-C₆cycloalkyl, R₂ is ethyl or isopropyl, R₄ is hydrogen or fluorine and n is the number 1 or 2.

12. The compound according to either claim 10 or claim 11 of formula 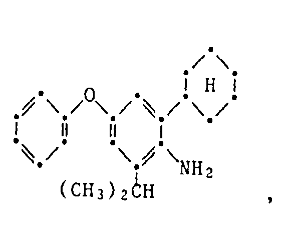 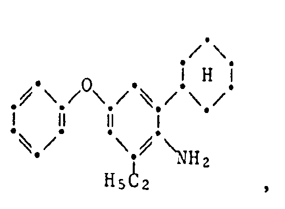 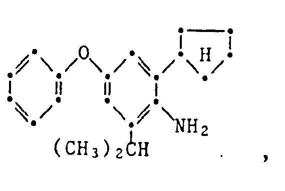 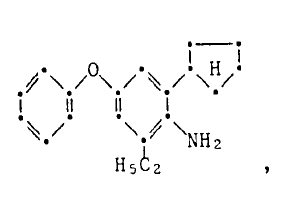 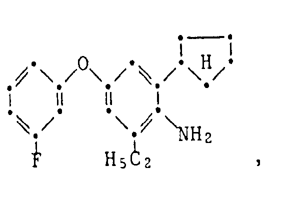 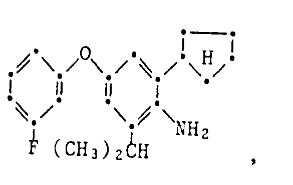 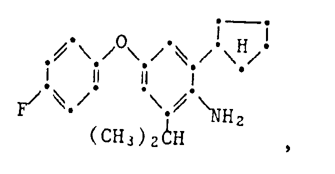 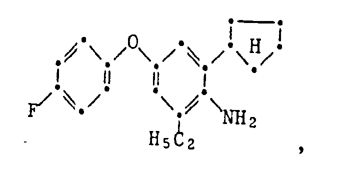 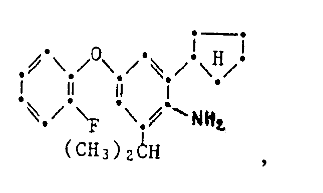 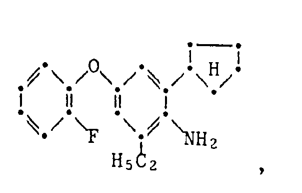 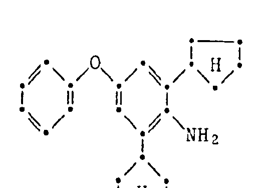 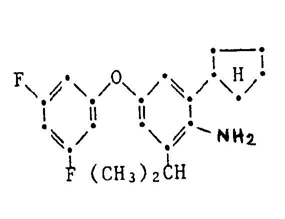 or

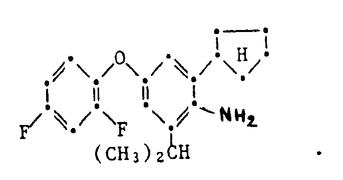

13. A compound of formula II 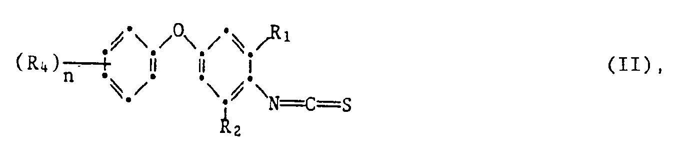
wherein
R₁ is C₃-C₇cycloalkyl or C₅-C₆cycloalkenyl,
R₂ is C₁-C₄alkyl or C₅-C₆cycloalkyl,
R₄ is in each case hydrogen, halogen, C₁-C₄alkyl, C₁-C₃alkoxy or CF₃, and
n is a number from 1 to 3.

14. A compound of formula II according to claim 13, wherein R₁ is C₃-C₇cycloalkyl, R₂ is C₁-C₄alkyl, R₄ is hydrogen, halogen or C₁-C₃alkyl, and n is the number 1 or 2.

15. A compound of formula II according to claim 14, wherein R₁ is C₅-Cₛcycloalkyl, R₂ is ethyl or isopropyl, R₄ is hydrogen or fluorine, and n is the number 1 or 2.

16. The compound according to either claim 14 or claim 15 of formula

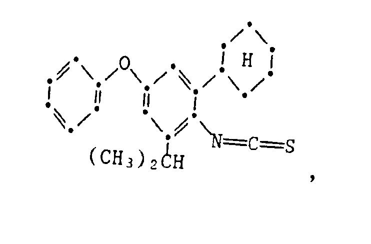

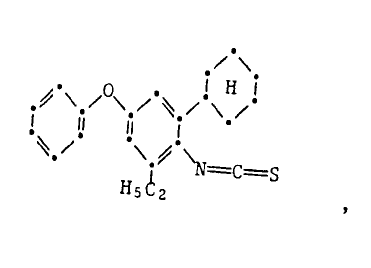

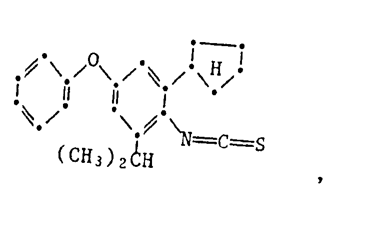

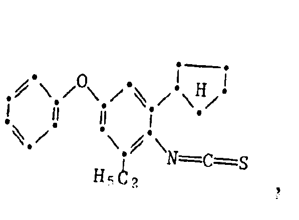

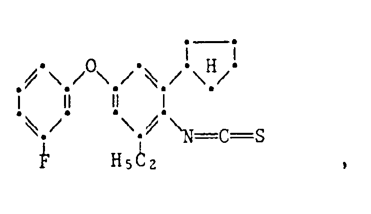

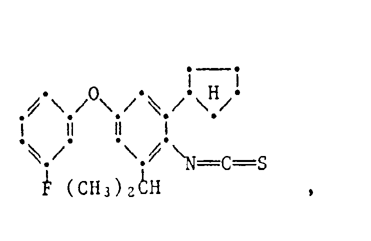

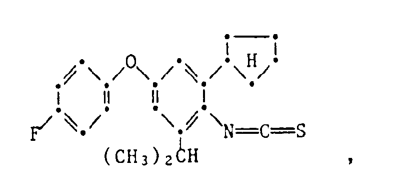

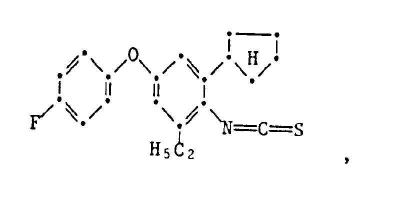

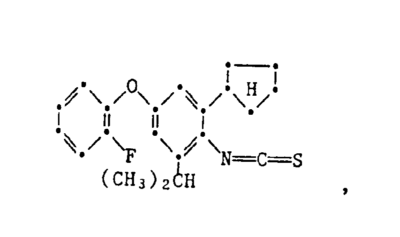

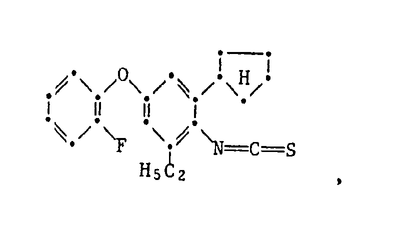

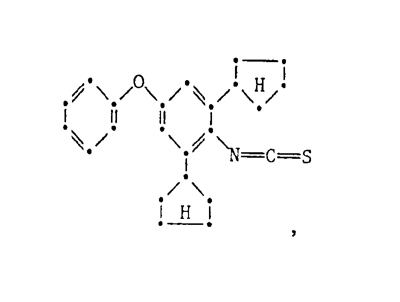

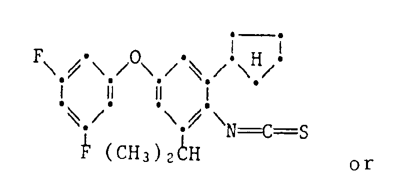

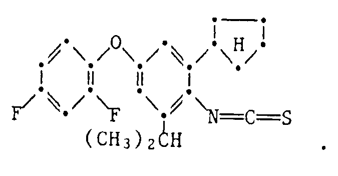

17. A process for the preparation of a compound of formula I 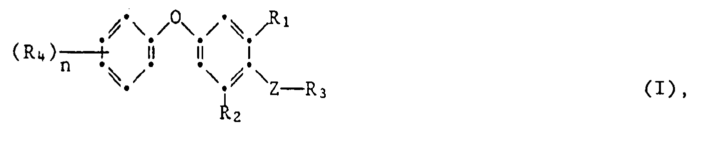
wherein
R₁ is C3-C₇cycloalkyl or C₅-Cₛcycloalkenyl,
R₂ is C₁-C₆alkyl, C₅-C₆cycloalkyl or C₅-Cₛcycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃, n is a number from 1 to 3,
Z is -NH-CS-NH-, -N=C(SR₅)-NH- or -N=C=N-, and
R₅ is C₁-C₅alkyl or allyl,
which process comprises
A) reacting an isothiocyanate of formula II 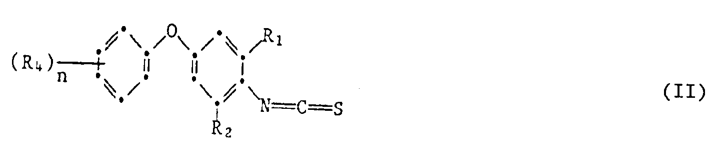 with an amine of formula III 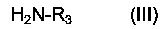
in an organic solvent or diluent under normal pressure and at a temperature from 0 to +150_{°}C, to give the thiourea and, if desired, B) reactina the resultant thiourea with a compound of formula IV 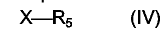
in an inert organic solvent under slightly elevated or normal pressure and at a temperature from +10 to 250°C, to give the isothiourea, or
C) converting the resultant thiourea into the carbodiimide by splitting off hydrogen sulfide in an aprotic organic solvent or diluent under normal pressure and at a temperature from 0 to +150°C, in which R_{1'} R₂, R₃, R₄, R₅ and n have the given meanings and X is a leaving group.

18. A process for the preparation of a compound of formula V 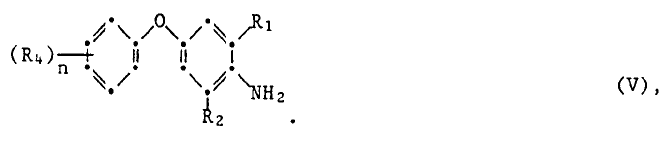
wherein
R₁ is C₃-C₇cycloalkyl or C₅-Cₛcycloalkenyl,
R₂ is C₁-C₆alkyl, C₅-C₆cycloalkyl or C₅-C₆cycloalkenyl,
R₄ is in each case hydrogen, halogen, C₁-C₄alkyl, C₁-C₃alkoxy or CF₃, and n is a number from 1 to 3,
which process comprises reacting a phenol of formula VII 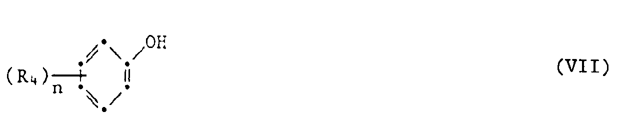
in the presence of an organic or inorganic base, under normal pressure and at a temperature from 0 to +100_{°}C in an inert solvent or diluent, with an aniline of formula VI 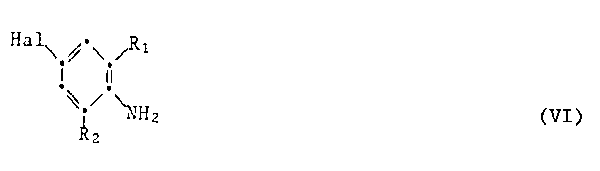
wherein R_{1'} R₂, R₄ and n have the given meanings and Hal is a halogen atom.

19. A process for the preparation of a compound of formula II 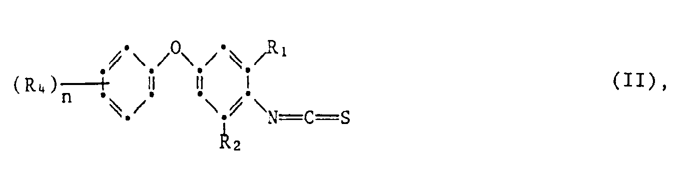
wherein
R₁ is C₃-C₇cycloalkyl or C₅-Cₛcycloalkenyl,
R₂ is C₁-C₆alkyl, C₅-C₆cycloalkyl or C₅-Cₛcycloalkenyl,
R₄ is in each case hydrogen, halogen, C₁-C₄alkyl, C₁-C₃alkoxy or CF₃, and n is a number from 1 to 3,
which process comprises reacting a phenoxyaniline of formula V 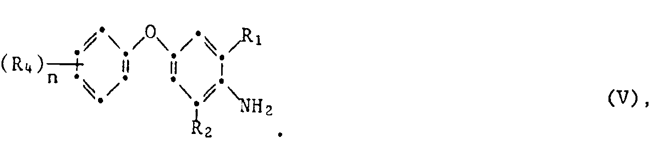 wherein R_{1'} R₂, R₄ and n have the given meanings, with thiophosgene in the presence of an organic or inorganic base, under normal pressure and at a temperature from 0 to +100_{°}C, in an inert solvent or diluent.

20. A pesticidal composition which contains as active component at least one compound of formula I 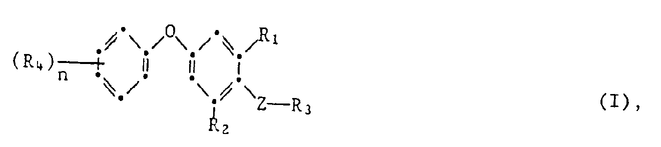
wherein
R₁ is C₃-C₇cycloalkyl or C₅-C₆cycloalkenyl,
R₂ is C₁-C₆alkyl, C₅-C₆cycloalkyl or C₅-C₆cycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl,
or a salt thereof with an organic or inorganic acid, together with suitable carriers and/or adjuvants.

21. A pesticidal composition according to claim 20 which contains as active component at least one compound of formula I, wherein R₁ is C₃-C₇cycloalkyl, R₂ is C₁-C₄alkyl or cyclopentyl, R₃ is C₁-C₄alkyl or C₃-C₅cycloalkyl, R₄ is hydrogen, halogen or C₁-C₃alkyl, n is the number 1 or 2, Z is -NH-CS-NH-, -N-C(SR₅)-NH- or -N=C=N-, and R₅ is C₁-C₃alkyl.

22. A pesticidal composition according to claim 21 which contains as active component at least one compound of formula I, wherein R1 is C₅-Cₛcycloalkyl, R₂ is ethyl or isopropyl, R₃ is isopropyl, tert-butyl or cyclopentyl, R₄ is hydrogen or fluorine, n is the number 1 or 2, and Z is -NH-CS-NH-.

23. A pesticidal composition according to claim 21 which contains as active component at least one compound offormula I, wherein R₁ is C₅-C₆cycloalkyl, R₂ is ethyl or isopropyl, R₃ is isopropyl, tert-butyl or cyclopentyl, R₄ is hydrogen or fluorine, n is the number 1 or 2, Z is -N=C(SR₅)-NH-, and R₅ is methyl or ethyl.

24. A pesticidal composition according to claim 21 which contains as active component at least one compound offormula I, wherein R₁ is C₅-C₆cycloalkyl , R₂ is ethyl or isopropyl, R₃ is isopropyl, tert-butyl or cyclopentyl, R₂ is hydrogen or fluorine, n is the number 1 or 2, and Z is -N=C=N-.

25. The use of a compound of formula I 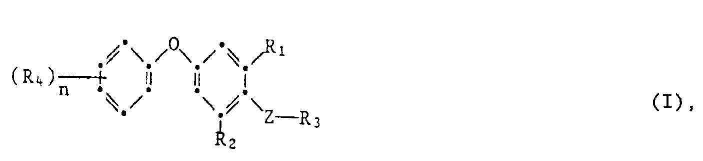
wherein
R₁ is C₃-C₇cycloalkyl or C₅-Cₛcycloalkenyl,
R₂ is C₁-C₆alkyl, C₅-C₆cycloalkyl or C₅-Cₛcycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl,
or a salt thereof with an organic or inorganic acid, for controlling pests on animals and plants.

26. The use according to claim 25 of a compound of formula I for controlling insects and arachnids.

27. A method for controlling pests on animals and plants, which comprises contacting the pests in their different development stages with a compound of formula I 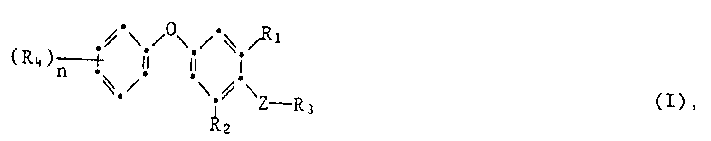
wherein
R₁ is C₃-C₇cycloalkyl or C₅-Cₛcycloalkenyl,
R₂ is C₁-C₆alkyl, C₅-C₆cycloalkyl or C₅-Cₛcycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl,
or with a salt thereof with an organic or inorganic acid.

## Claims (Claims for the following Contracting State(s) : ES)

1. A process for the preparation of a compound of formula I 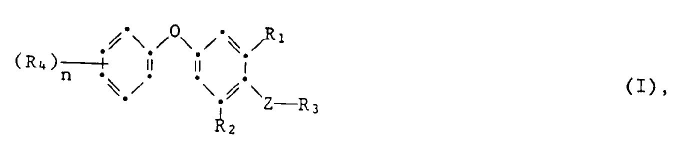
wherein
R₁ is C₃-C₇cycloalkyl or C₅-Cₛcycloalkenyl,
R₂ is C₁-C₆alkyl, C₅-C₆cycloalkyl or C₅-Cₛcycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl,
which process comprises
A) reacting an isothiocyanate of formula II 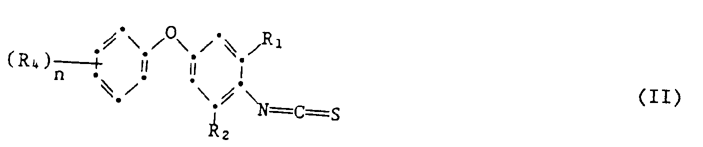 with an amine of formula III 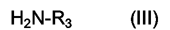
in an organic solvent or diluent under normal pressure and at a temperature from 0 to +150_{°}C, to give the thiourea and, if desired,
B) reacting the resultant thiourea with a compound of formula IV 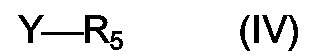
in an inert organic solvent under slightly elevated or normal pressure and at a temperature from +10 to 250°C, to give the isothiourea, or
C) converting the resultant thiourea into the carbodiimide by splitting off hydrogen sulfide in an aprotic organic solvent or diluent under normal pressure and at a temperature from 0 to +150°C, in which R_{1,} R₂, R₃, R₄, R₅ and n have the given meanings and X is a leaving group.

2. A process according to claim 1, wherein R₁ is C₃-C₇cycloalkyl, R₂ is C₁-C₄alkyl or cyclopentyl, R₃ is C₁-C₄alkyl or C₃-C₅cycloalkyl, R₄ is hydrogen, halogen or C₁-C₃alkyl, n is the number 1 or 2, Z is -NH-CS-NH-, -N=C(SR₅)-NH- or-N=C=N-, and R₅ is C₁-C₃alkyl.

3. A process according to claim 2, wherein R₁ is C₅-Cₛcycloalkyl, R₂ is ethyl or isopropyl, R₃ is isopropyl, tert-butyl or cyclopentyl, R₄ is hydrogen or fluorine, n is the number 1 or 2, and Z is -NH-CS-NH-.

4. A process according to claim 2, wherein R₁ is C₅-Cₛcycloalkyl, R₂ is ethyl or isopropyl, R₃ is isopropyl, tert-butyl orcyclopentyl, R₄ is hydrogen orfluorine, n is the number 1 or2, Z is-N=C(SR₅)-NH-, and R₅ is methyl or ethyl.

5. A process according to claim 2, wherein R₁ is C₅-Cₛcycloalkyl, R₂ is ethyl or isopropyl, R₃ is isopropyl, tert-butyl or cyclopentyl, R₄ is hydrogen or fluorine, n is the number 1 or 2, and Z is -N=C=N-.

6. A process according to either claim 1 or claim 3 for the preparation of a compound of formula

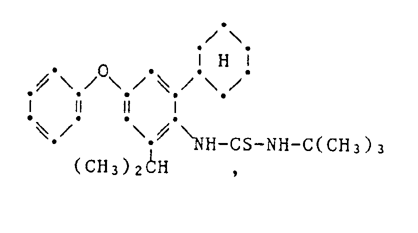

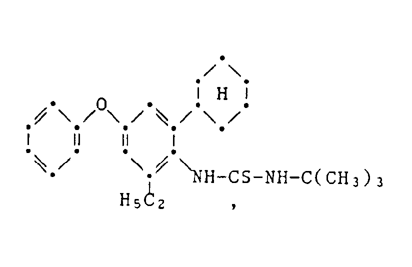

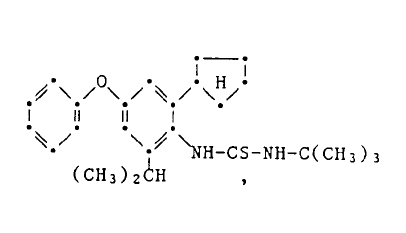

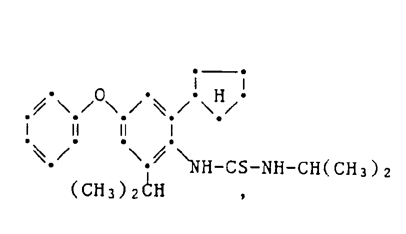

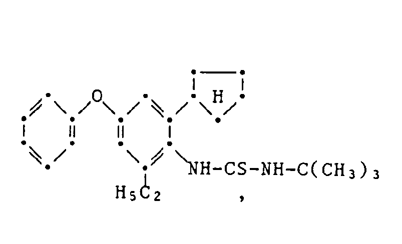

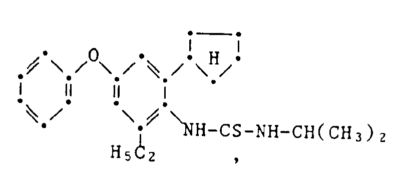

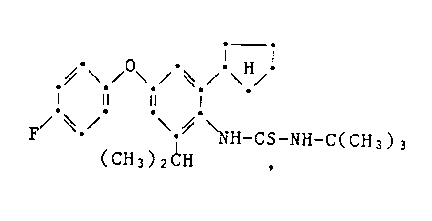

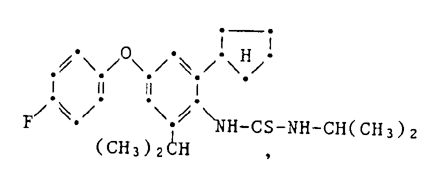

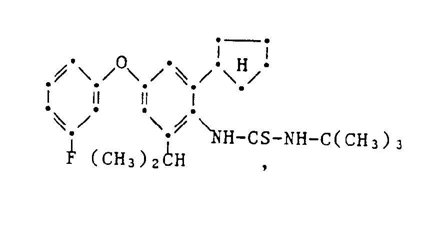

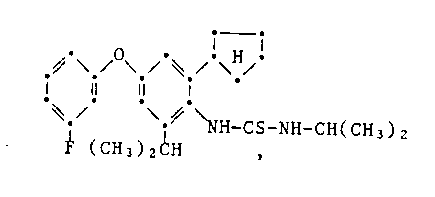

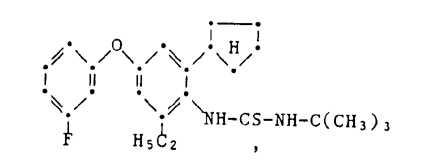

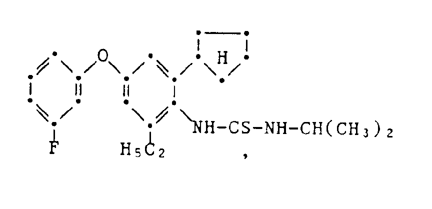

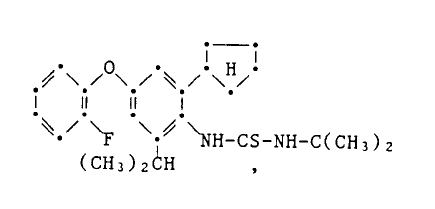

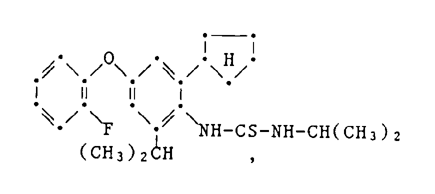

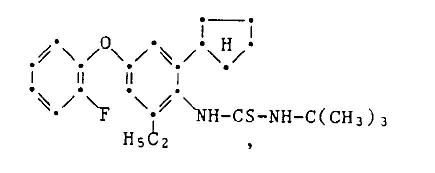

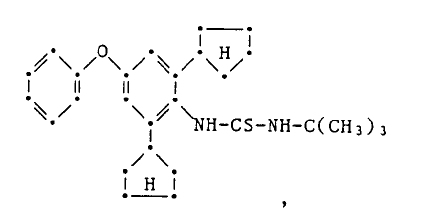

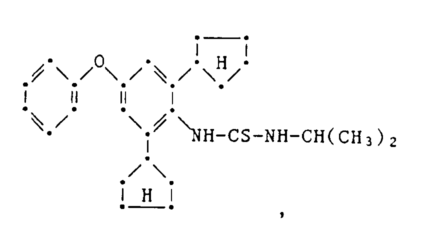

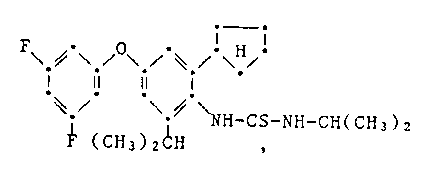

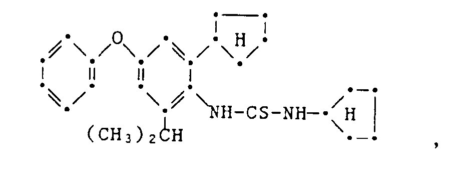

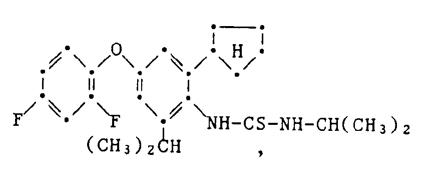

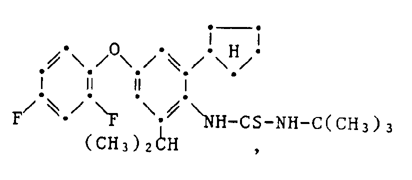

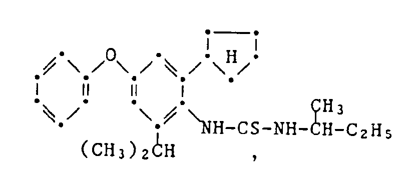

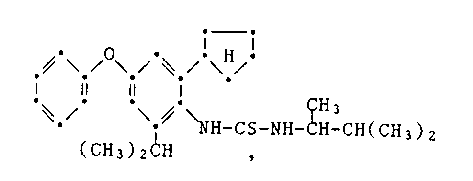

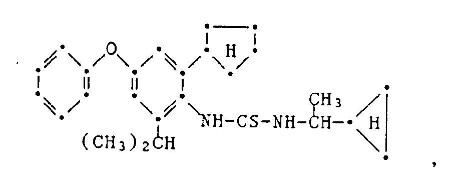

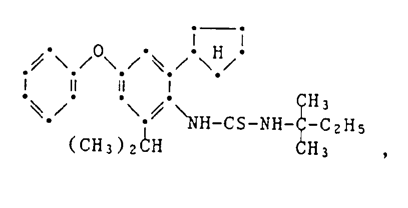

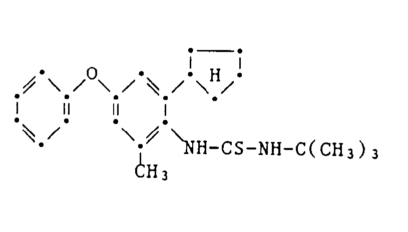

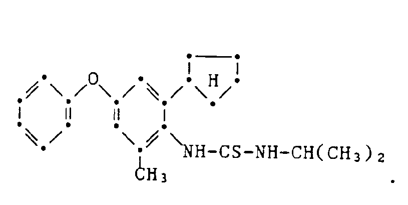

or

7. A process according to either claim 1 or claim 4 for the preparation of a compound of formula

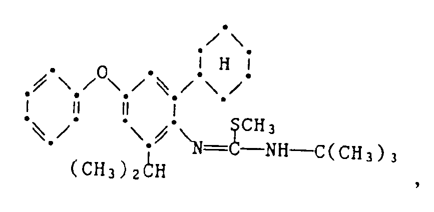

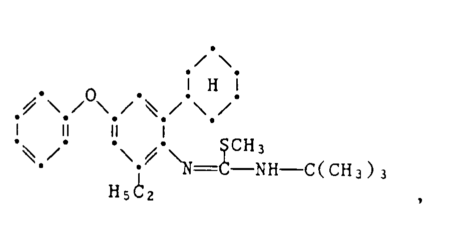

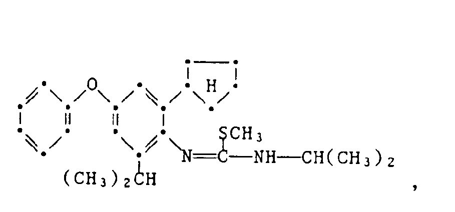

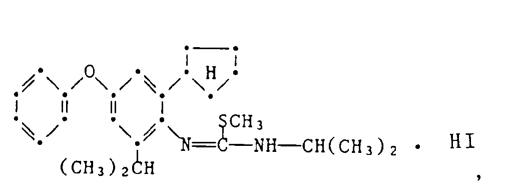

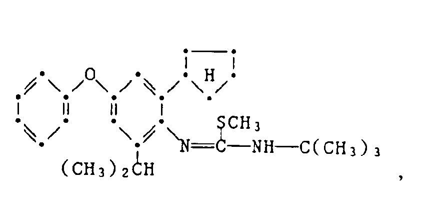

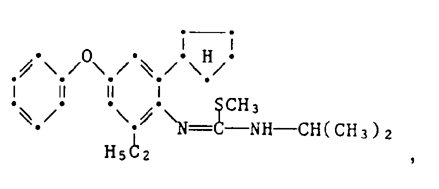

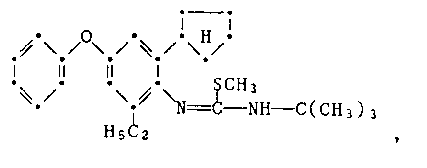

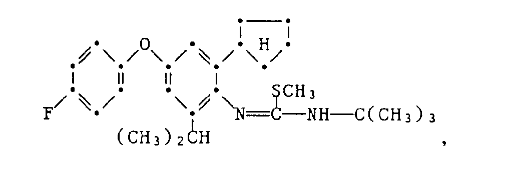

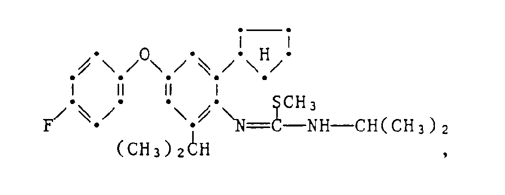

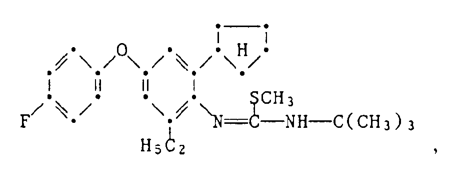

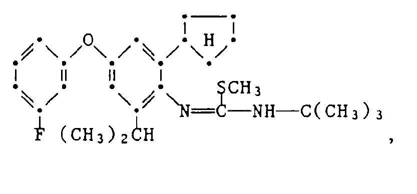

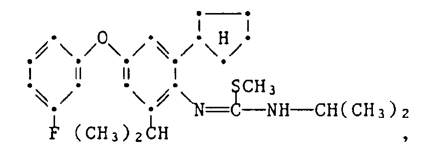

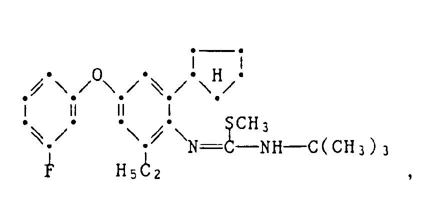

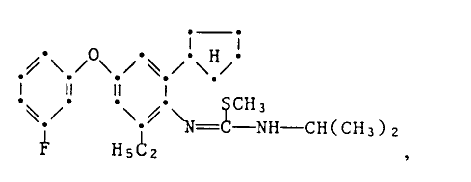

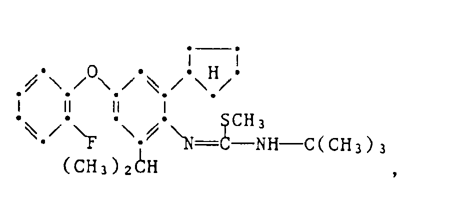

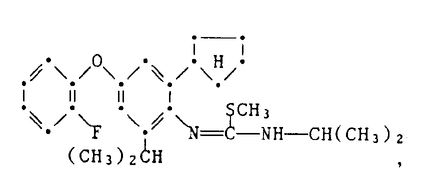

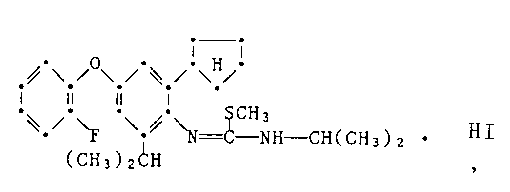

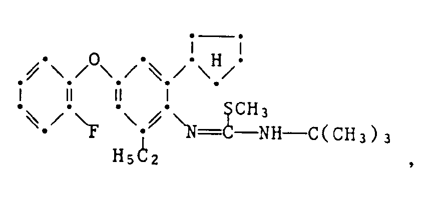

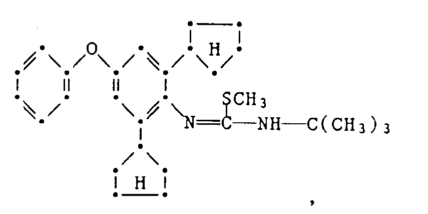

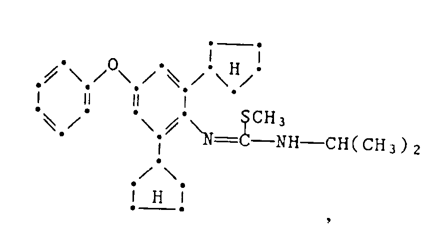

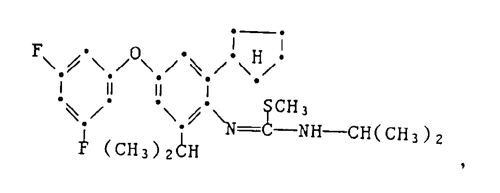

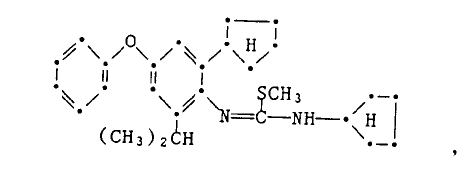

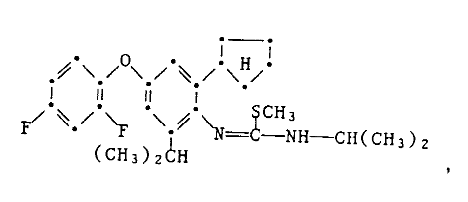

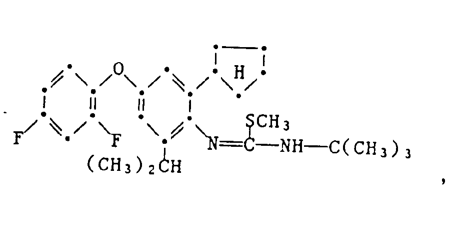

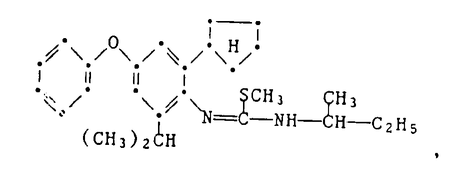

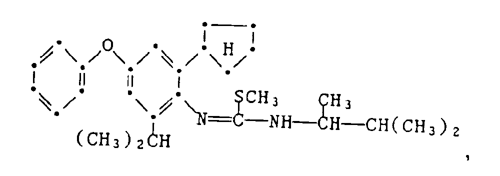

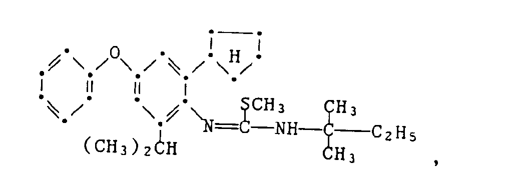

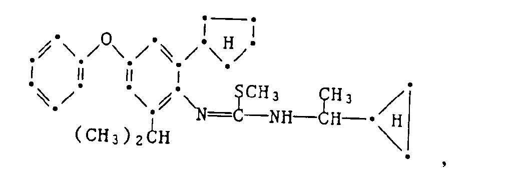

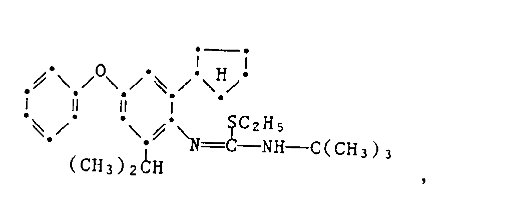

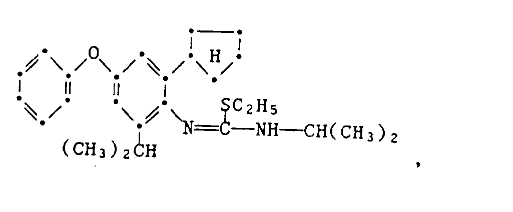

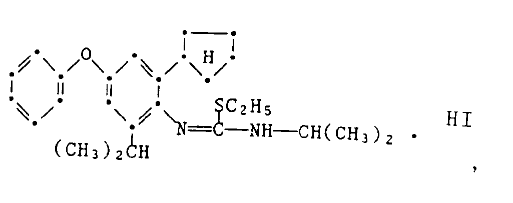

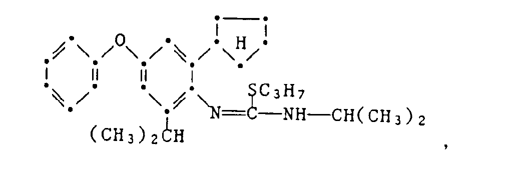

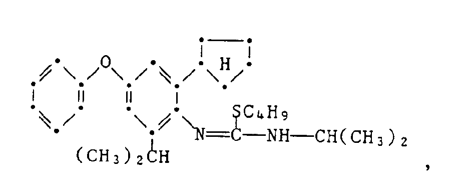

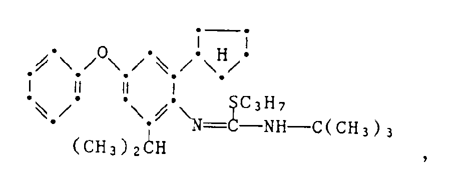

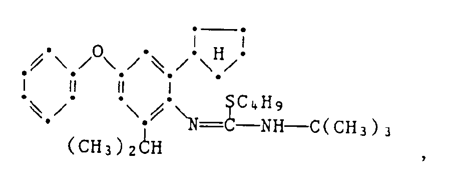

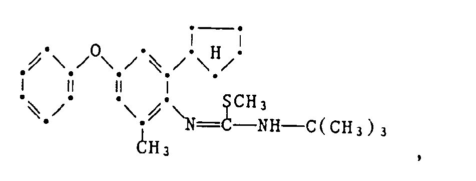

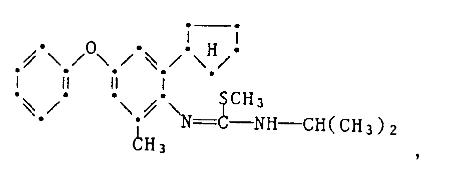

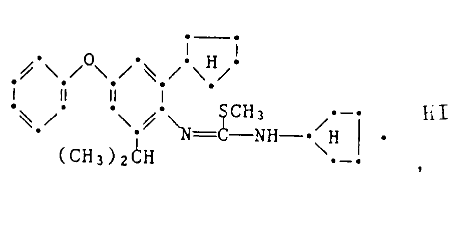

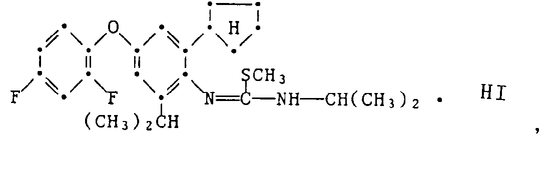

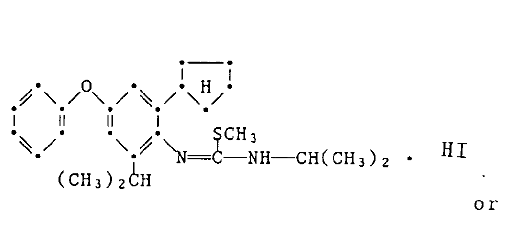

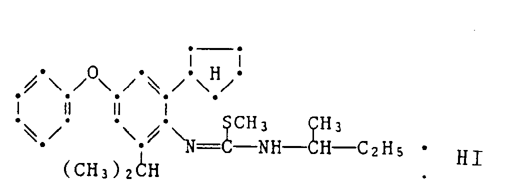

8. A process according to either claim 1 or claim 5 for the preparation of a compound of formula

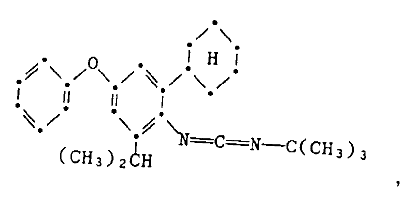

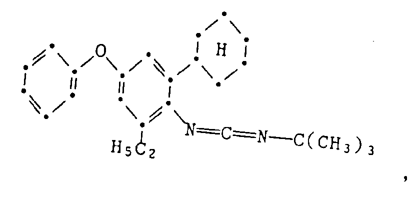

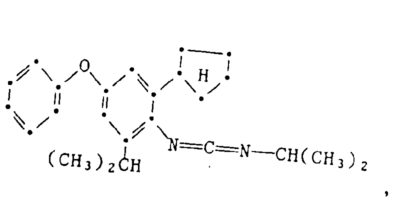

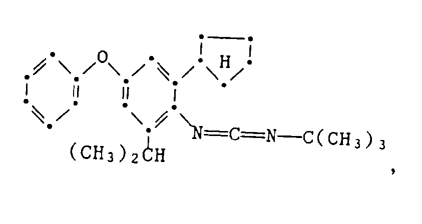

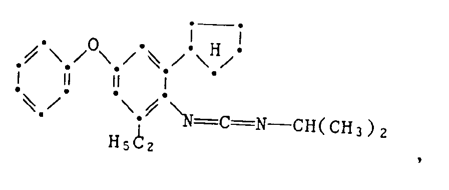

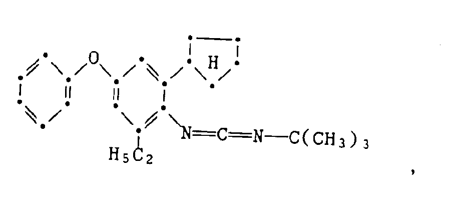

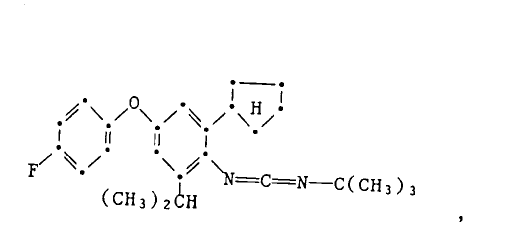

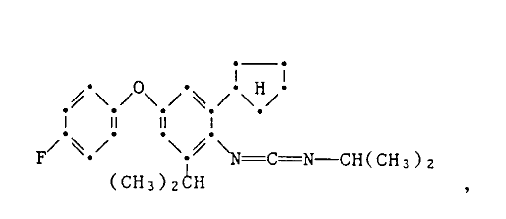

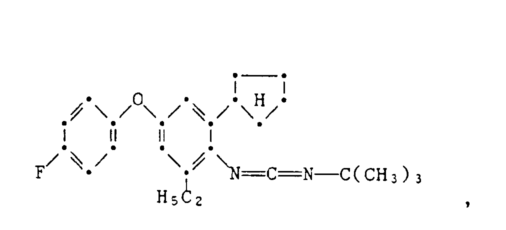

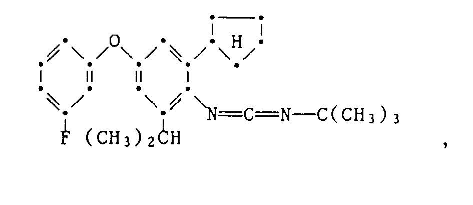

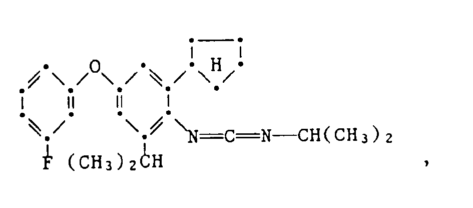

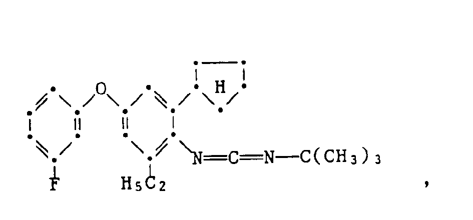

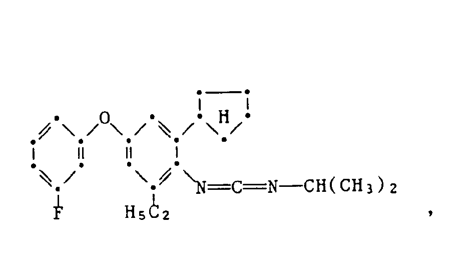

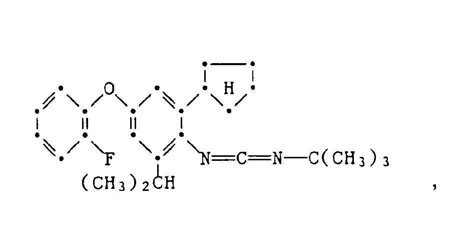

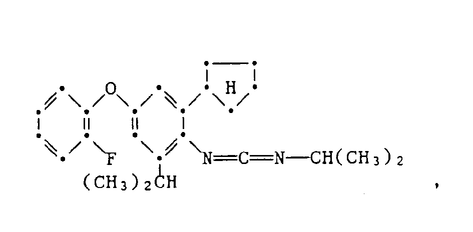

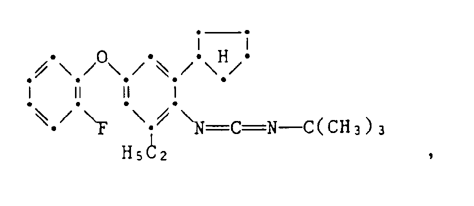

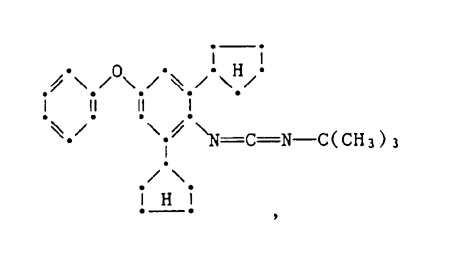

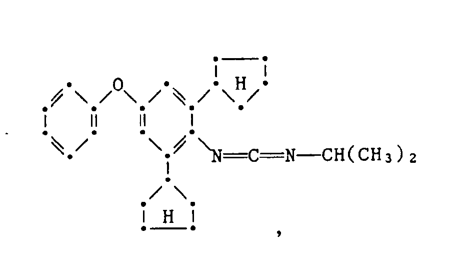

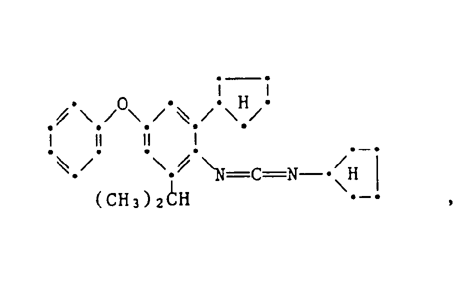

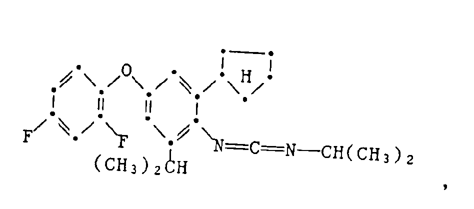

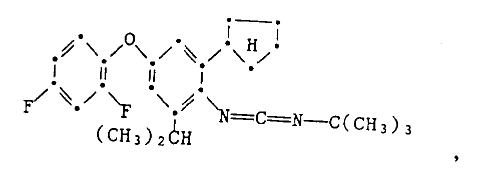

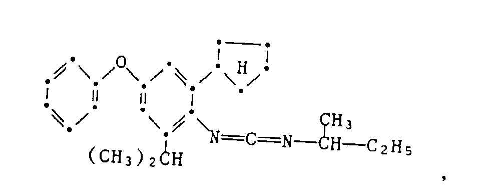

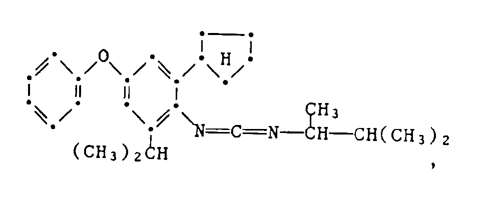

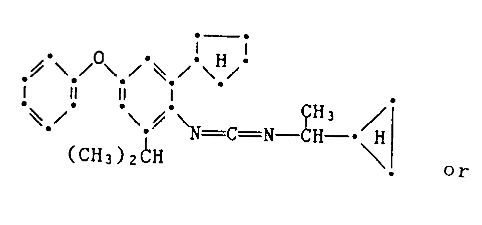

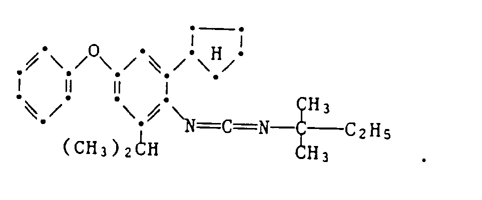

9. A process for the preparation of a compound of formula V 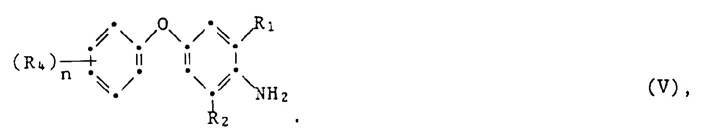
wherein
R₁ is C₃-C₇cycloalkyl or C₅-C₆cycloalkenyl,
R₂ is C₁-C₆alkyl, C₅-C₆cycloalkyl or C₅-C₆cycloalkenyl,
R₄ is in each case hydrogen, halogen, C₁-C₄alkyl, C₁-C₃alkoxy or CF₃, and n is a number from 1 to 3,
which process comprises reacting a phenol of formula VII 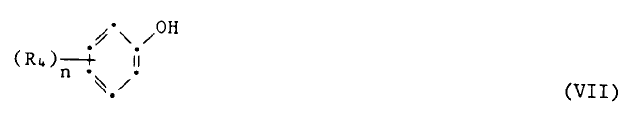
in the presence of an organic or inorganic base, under normal pressure and at a temperature from 0 to +100_{°}C in an inert solvent or diluent, with an aniline of formula VI 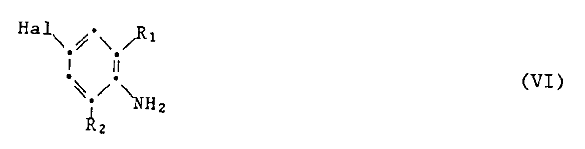 wherein R_{1,} R₂, R₄ and n have the given meanings and Hal is a halogen atom.

10. A process according to claim 9, wherein R₁ is C₃-C₇cycloalkyl, R₂ is C₁-C₄alkyl or cyclopentyl, R₄ is hydrogen, halogen or C₁-C₃alkyl, and n is the number 1 or 2.

11. A process according to claim 10, wherein R₁ is C₅-Cₛcycloalkyl, R₂ is ethyl or isopropyl, R₄ is hydrogen or fluorine and n is the number 1 or 2.

12. A process according to either claim 10 or claim 11 for the preparation of a compound of formula

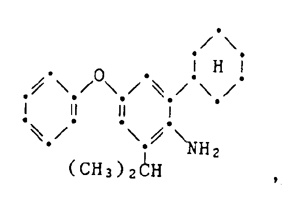

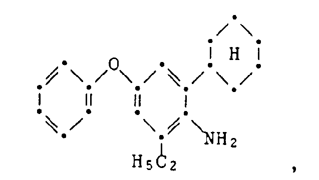

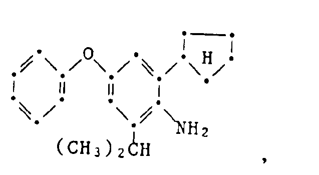

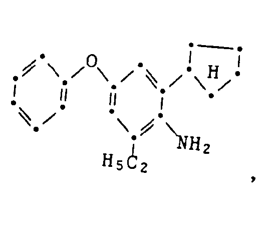

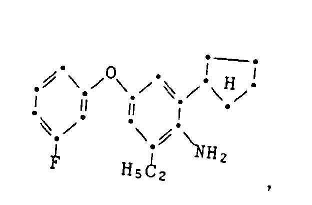

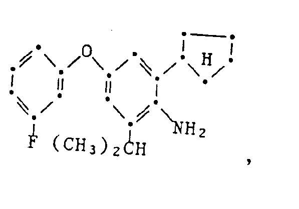

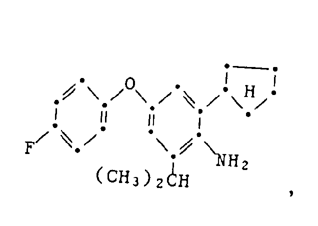

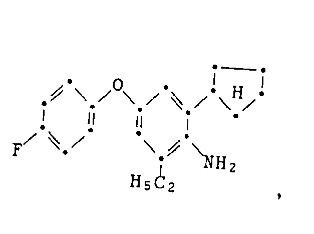

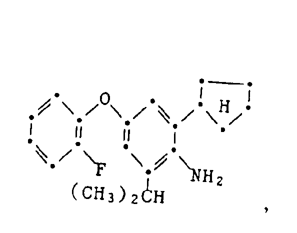

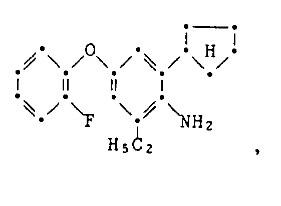

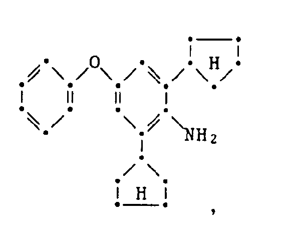

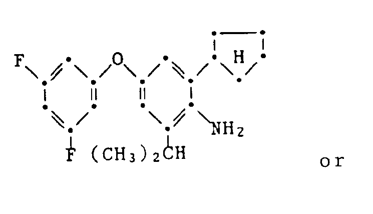

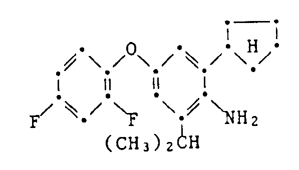

13. A process for the preparation of a compound of formula II 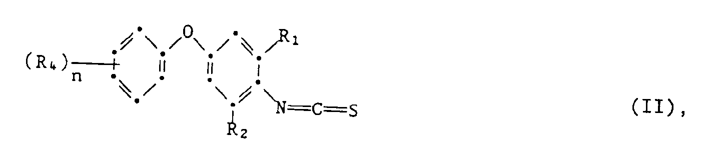
wherein
R₁ is C₃-C₇cycloalkyl or C₅-C₆cycloalkenyl,
R₂ is C₁-C₆alkyl, C₅-C₆cycloalkyl or C₅-C₆cycloalkenyl,
R₄ is in each case hydrogen, halogen, C₁-C₄alkyl, C₁-C₃alkoxy or CF₃, and n is a number from 1 to 3,
which process comprises reacting a phenoxyaniline of formula V 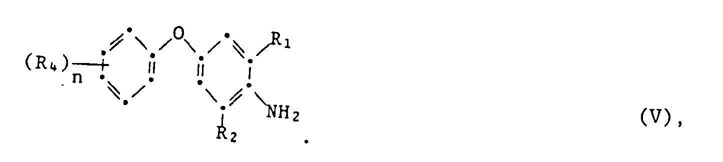 wherein R₁, R₂, R₄ and n have the given meanings, with thiophosgene in the presence of an organic or inorganic base, under normal pressure and at a temperature from 0 to +100_{°}C, in an inert solvent or diluent.

14. A process according to claim 13, wherein R₁ is C₃-C₇cycloalkyl, R₂ is C₁-C₄alkyl, R₄ is hydrogen, halogen or C₁-C₃alkyl, and n is the number 1 or 2.

15. A process according to claim 14, wherein R₁ is C₅-Cₛcycloalkyl, R₂ is ethyl or isopropyl, R₄ is hydrogen or fluorine, and n is the number 1 or 2.

16. A process according to either claim 14 or claim 15 for the preparation of a compound of formula 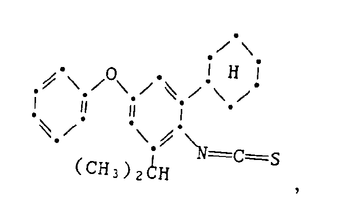 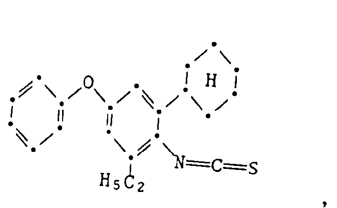 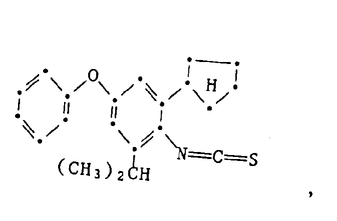 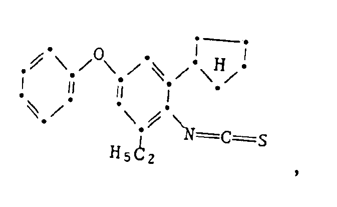 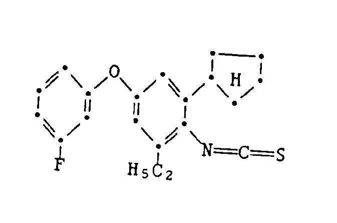 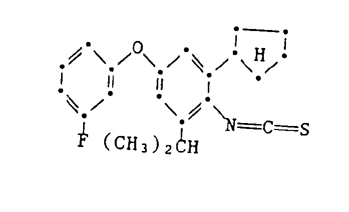 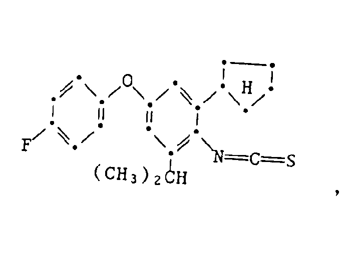 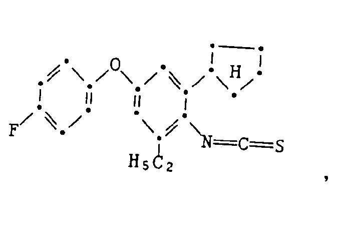 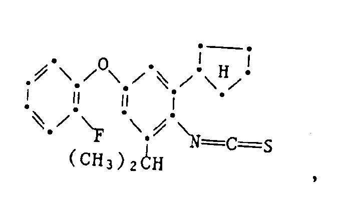 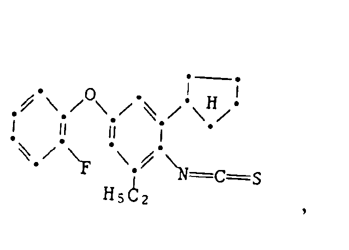 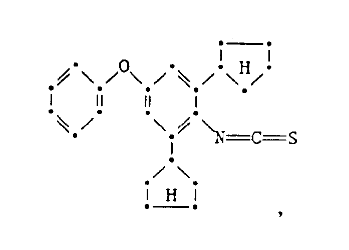 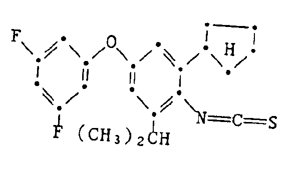 or

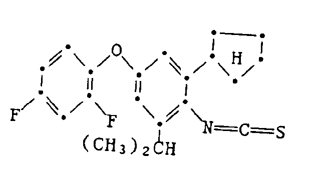

17. A pesticidal composition which contains as active component at least one compound of formula I 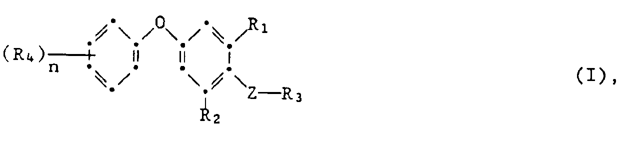
wherein
R₁ is C₃-C₇cycloalkyl or C₅-Cₛcycloalkenyl,
R₂ is C₁-C₆alkyl, C₅-C₆cycloalkyl or C₅-Cₛcycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl,
or a salt thereof with an organic or inorganic acid, together with suitable carriers and/or adjuvants.

18. A pesticidal composition according to claim 17 which contains as active component at least one compound of formula I, wherein R₁ is C₃-C₇cycloalkyl, R₂ is C₁-C₄alkyl or cyclopentyl, R₃ is C₁-C₄alkyl or C₃-C₅cycloalkyl, R₄ is hydrogen, halogen or C₁-C₃alkyl, n is the number 1 or 2, Z is -NH-CS-NH-, -N=C(SR₅)-NH- or -N=C=N-, and R₅ is C₁-C₃alkyl.

19. A pesticidal composition according to claim 18 which contains as active component at least one compound offormula I, wherein R₁ is C₅-C₆cycloalkyl, R₂ is ethyl or isopropyl, R₃ is isopropyl, tert-butyl or cyclopentyl, R₄ is hydrogen or fluorine, n is the number 1 or 2, and Z is -NH-CS-NH-.

20. A pesticidal composition according to claim 18 which contains as active component at least one compound offormula I, wherein R₁ is C₅-C₆cycloalkyl, R₂ is ethyl or isopropyl, R₃ is isopropyl, tert-butyl or cyclopentyl, R₄ is hydrogen or fluorine, n is the number 1 or 2, Z is -N=C(SR₅)-NH-, and R₅ is methyl or ethyl.

21. A pesticidal composition according to claim 18 which contains as active component at least one compound offormula I, wherein R₁ is C₅-C₆cycloalkyl, R₂ is ethyl or isopropyl, R₃ is isopropyl, tert-butyl or cyclopentyl, R₄ is hydrogen or fluorine, n is the number 1 or 2, and Z is -N=C=N.

22. The use of a compound of formula I 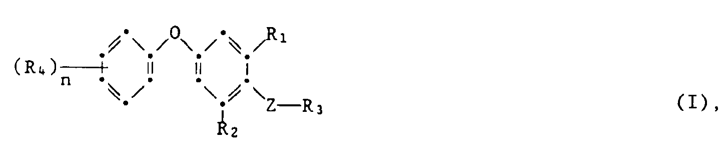
wherein
R₁ is C₃-C₇cycloalkyl or C₅-Cₛcycloalkenyl,
R₂ is C₁-C₆alkyl, C₅-C₅cycloalkyl or C₅-C₆cycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl,
or a salt thereof with an organic or inorganic acid for controlling pests on animals and plants.

23. The use according to claim 22 of a compound of formula I for controlling insects and arachnids.

24. A method for controlling pests on animals and plants, which comprises contacting the pests in their different development stages with a compound of formula I 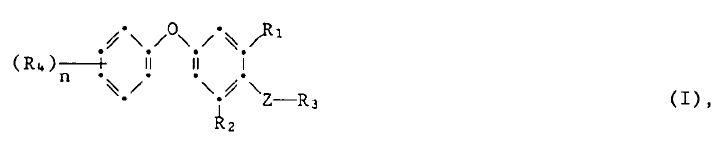
wherein
R₁ is C₃-C₇cycloalkyl or C₅-Cₛcycloalkenyl,
R₂ is C₁-C₆alkyl, C₅-C₆cycloalkyl or C₅-Cₛcycloalkenyl,
R₃ is C₁-C₈alkyl, C₃-C₆cycloalkyl, 1-cyclopropylethyl or C₃-C₅alkenyl,
R₄ is in each case hydrogen, halogen, C₁-C₄alkyl, C₁-C₃alkoxy or CF₃,
n is a number from 1 to 3,
Z is -NH-CS-NH-, -N=C(SR₅)-NH- or -N=C=N-, and
R₅ is C₁-C₅alkyl or allyl,
or with a salt thereof with an organic or inorganic acid.

## Revendications (Revendications pour l'(les) Etat(s) contractant(s) suivant(s) : suivants : AT, BE, CH, DE, FR, GB, GR, IT, LI, NL.)

1. Composés de formule I 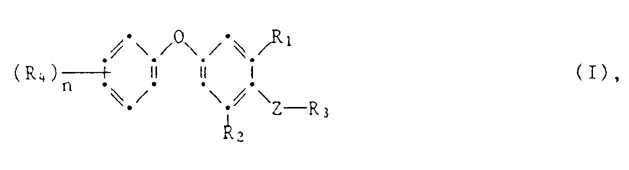
dans laquelle
R₁ représente un groupe cycloalkyle en C₃-C₇ ou cycloalcényle en C₅-C₆,
R₂ représente un groupe alkyle en C₁-C_{6,} cycloalkyle en C₅-C₆ ou cycloalcényle en C₅-C_{6,}
R₃ représente un groupe alkyle en C₁-C₈, cycloalkyle en C₃-C_{6,} 1-cyclopropyléthyle ou alcényle en C₃-C₅, chacun des symboles R₄ représente l'hydrogène, un halogène, un groupe alkyle en C₁-C₄, alcoxy en C₁-C₃ ou CF₃,
n est un nombre allant de 1 à 3,
Z représente -NH-CS-NH-, -N=C(SR₅)-NH- ou -N=C=N-, et
R₅ représente un groupe alkyle en C₁-C₅ ou allyle,
et leurs sels d'acides organiques ou minéraux.

2. Composés de formule I selon revendication 1, dans laquelle R₁ représente un groupe cycloalkyle en C₃-C₇, R₂ représente un groupe alkyle en C₁-C₄ ou cyclopentyie, R₃ un groupe alkyle en C₁-C₄ ou cycloalkyle en C₃-C₅, R₄ l'hydrogène, un halogène ou un groupe alkyle en C₁-C₃, n est égal à 1 ou 2, Z représente -NH-CS-NH-, -N=C(SR₅)-NH- ou -N=C=N- et R₅ représente un groupe alkyle en C₁-C₃.

3. Composés de formule I de la revendication 2, dans laquelle R₁ représente un groupe cycloalkyle en C₅-Cₛ, R₂ représente un groupe éthyle ou isopropyle, R₃ un groupe isopropyle, tert-butyle ou cyclopentyle, R₄ l'hydrogène ou le fluor, n est égal à 1 ou 2 et Z représente -NH-CS-NH-.

4. Composés de formule I de la revendication 2, dans laquelle R₁ représente un groupe cycloalkyle en C₅-C₆, R₂ un groupe éthyle ou isopropyle, R₃ un groupe isopropyle, tert-butyle ou cyclopentyle, R₄ l'hydrogène ou le fluor, n est égal à 1 ou 2, Z représente -N=C(SR₅)NH- et R₅ représente un groupe méthyle ou éthyle.

5. Composés de formule I de la revendication 2, dans laquelle R₁ représente un groupe cycloalkyle en C₅-Cₛ, R₂ un groupe éthyle ou isopropyle, R₃ un groupe isopropyle, tert-butyle ou cyclopentyle, R₄ l'hydrogène ou le fluor, n est égal à 1 ou 2 et Z représente -N=C=N-.

6. Composés selon l'une des revendications 1 ou 3, de formules

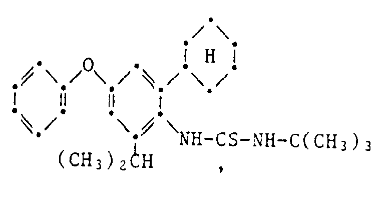

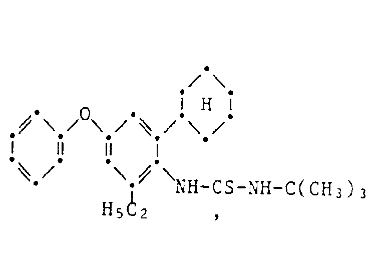

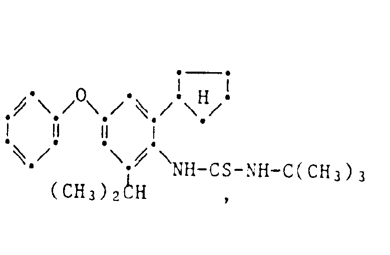

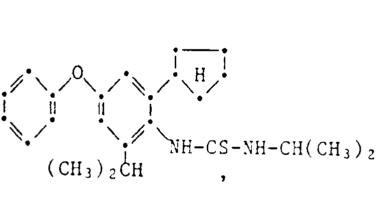

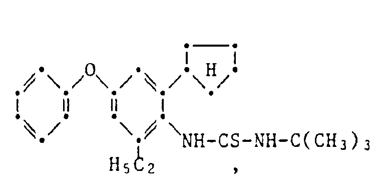

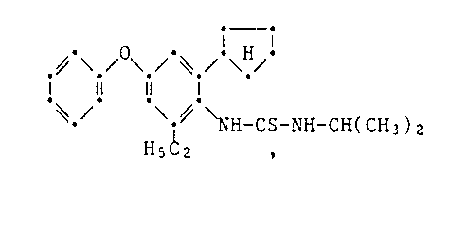

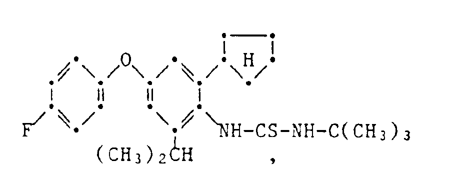

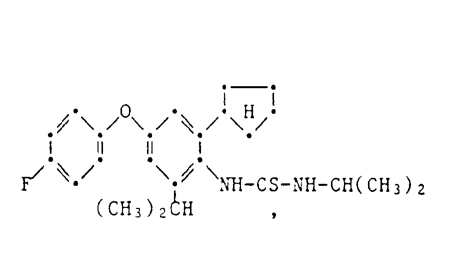

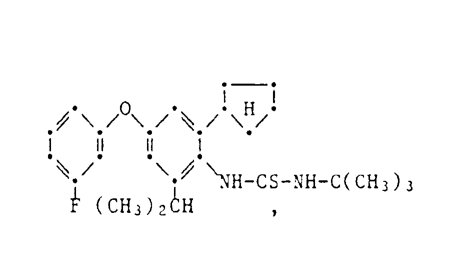

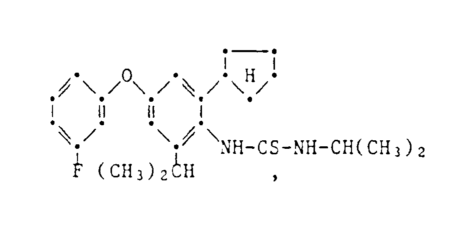

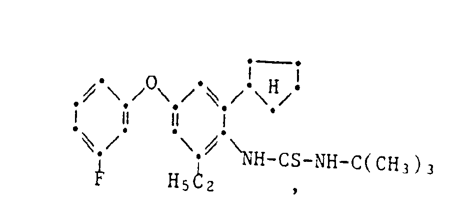

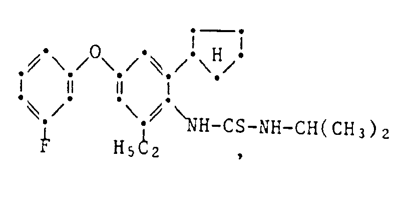

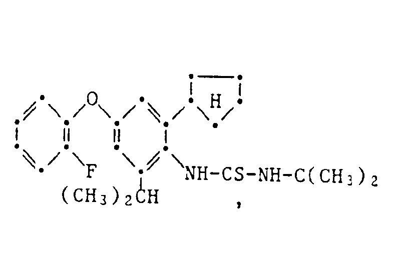

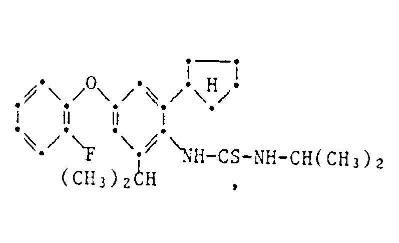

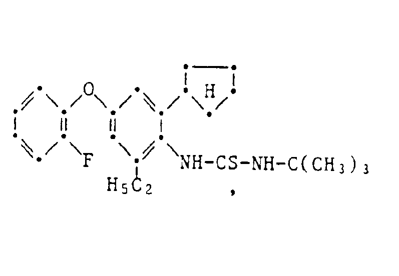

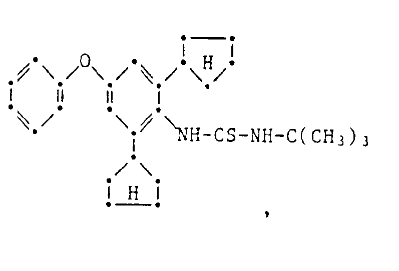

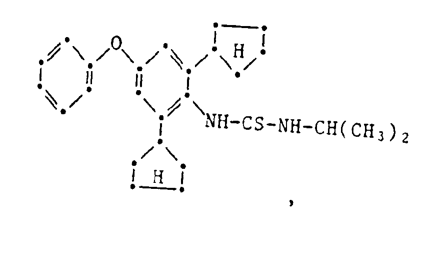

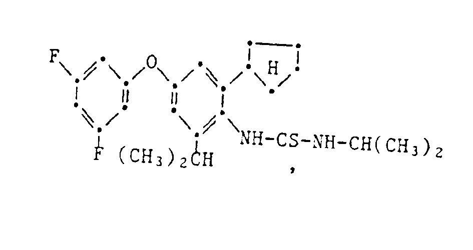

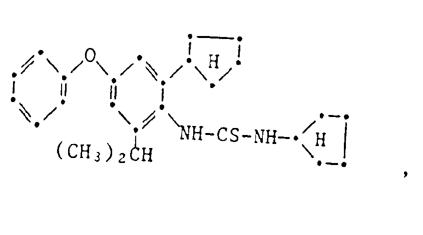

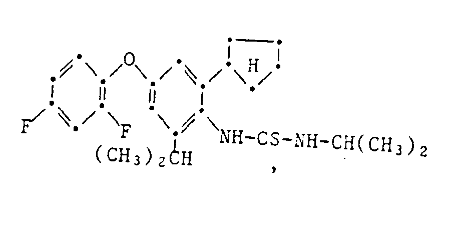

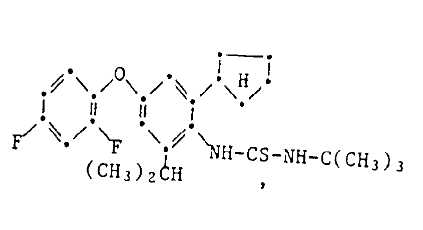

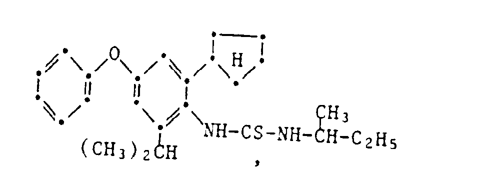

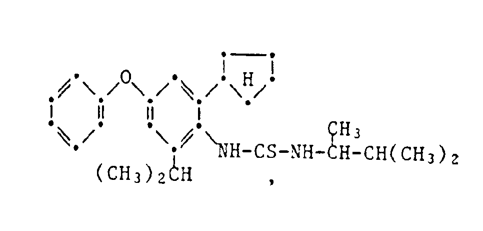

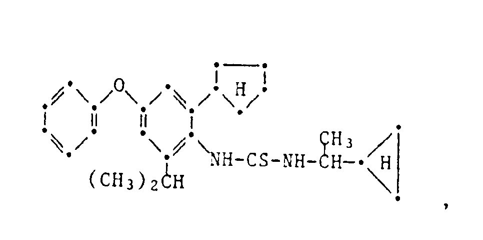

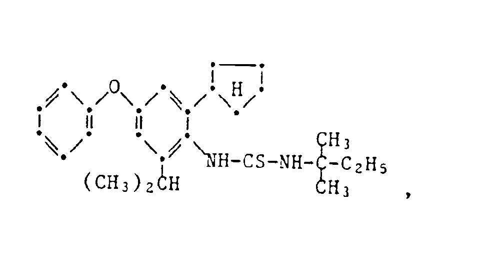

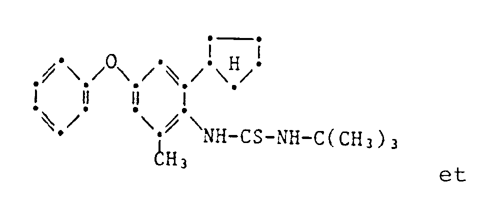

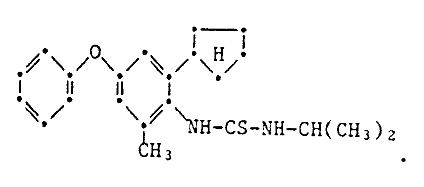

7. Composés selon l'une des revendications 1 ou 4, de formules

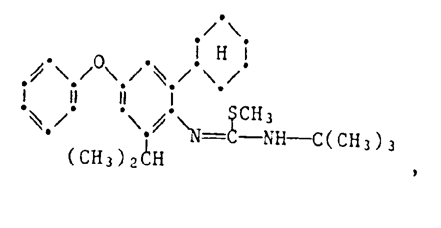

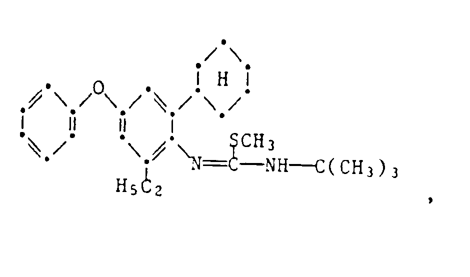

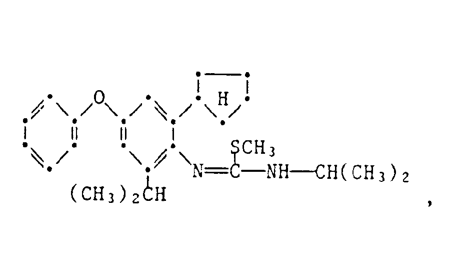

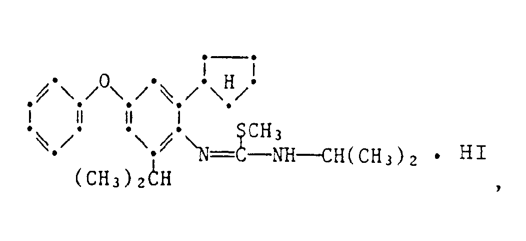

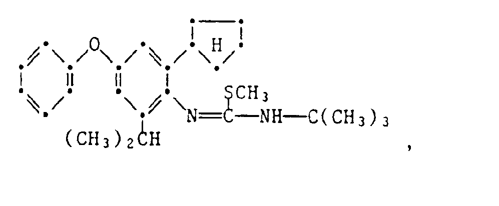

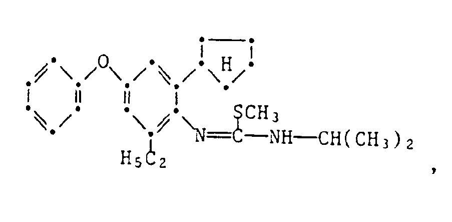

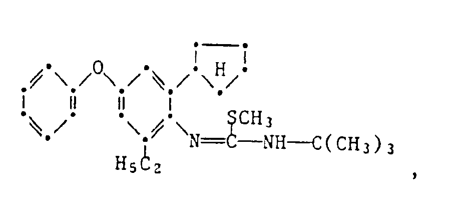

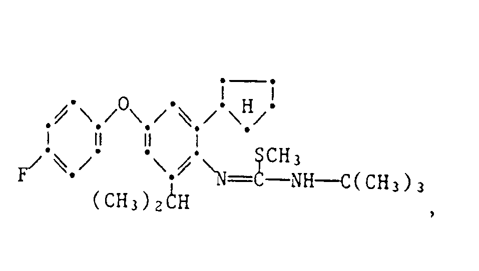

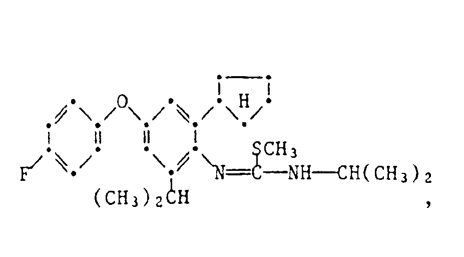

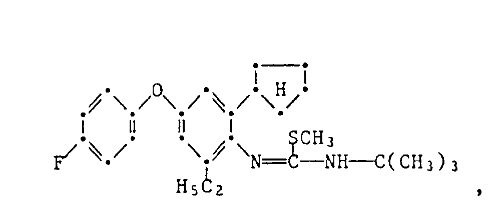

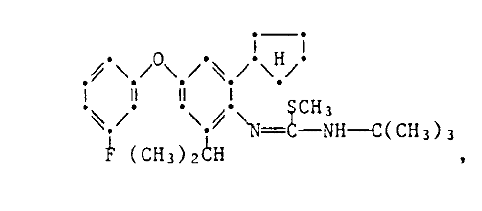

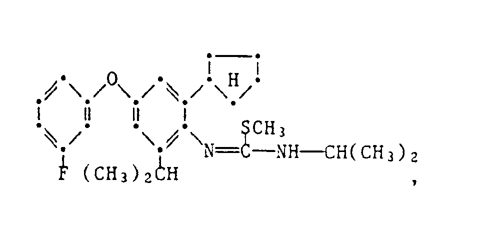

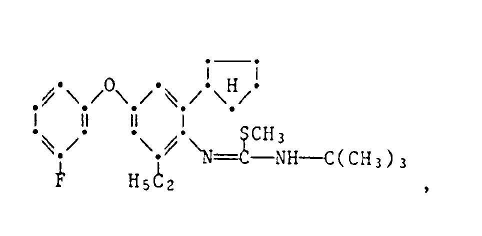

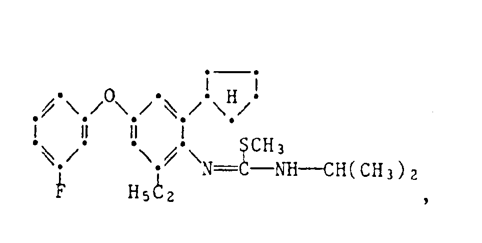

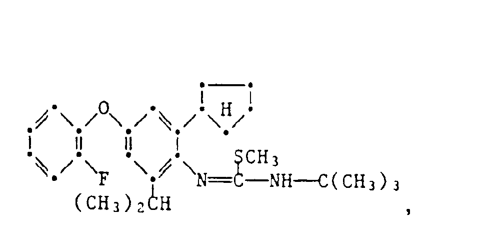

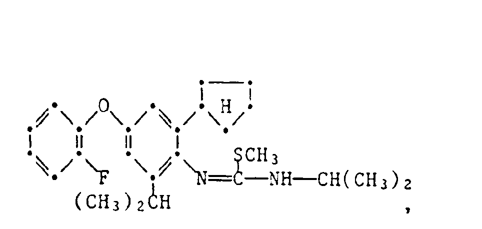

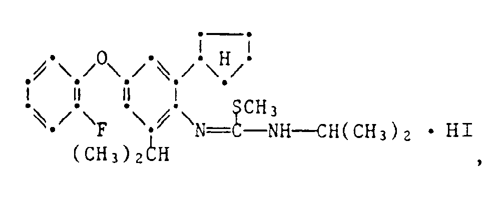

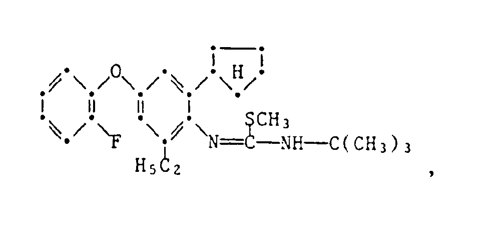

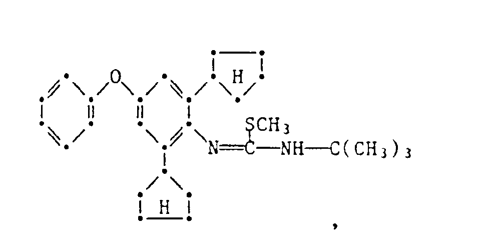

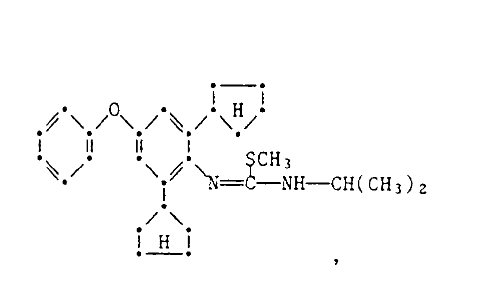

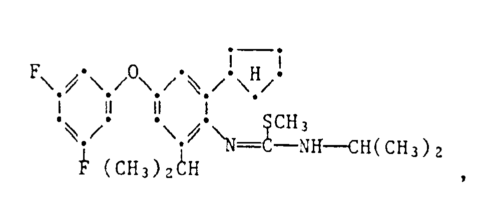

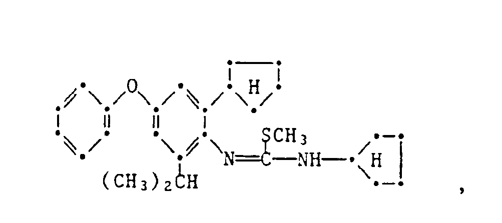

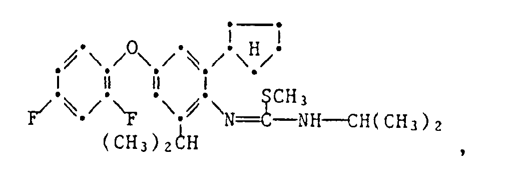

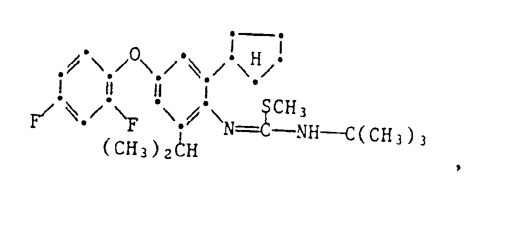

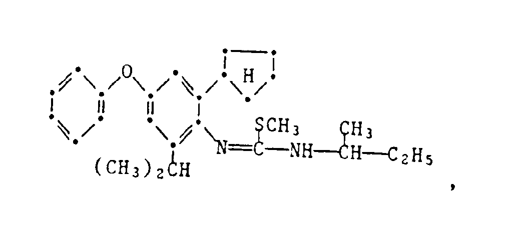

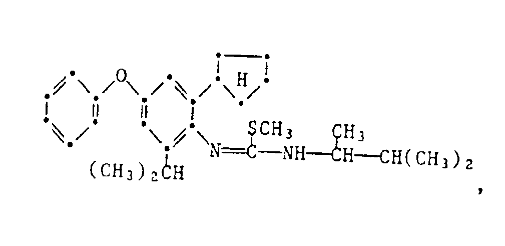

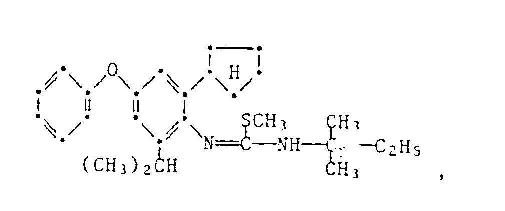

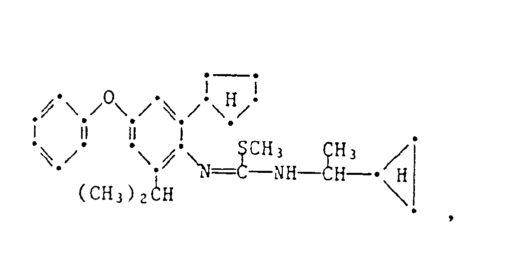

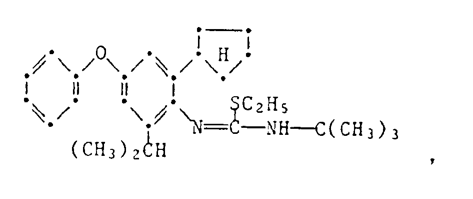

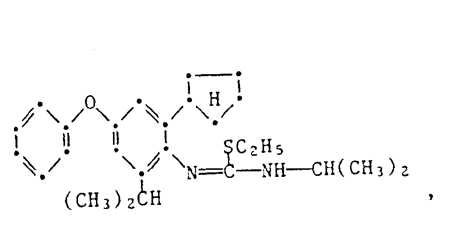

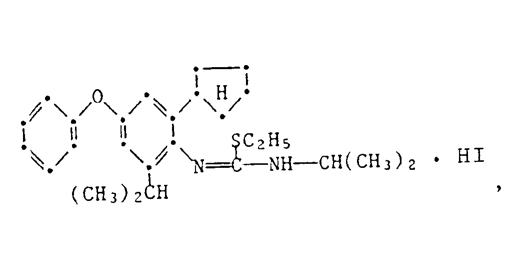

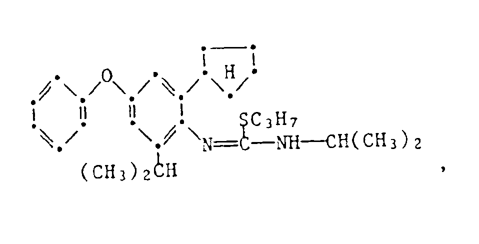

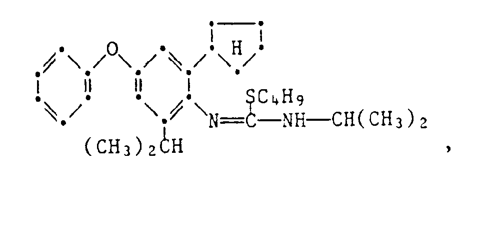

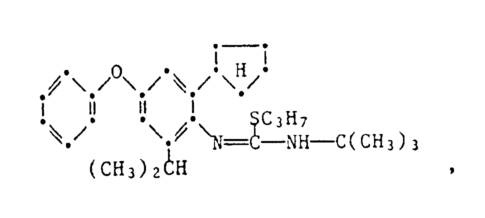

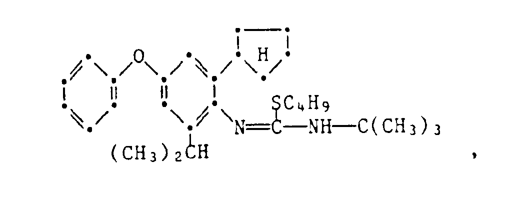

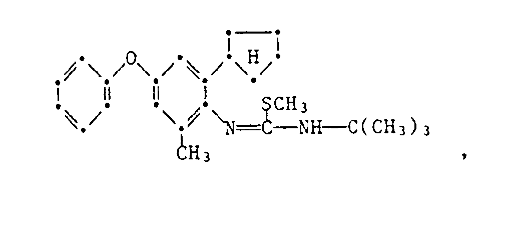

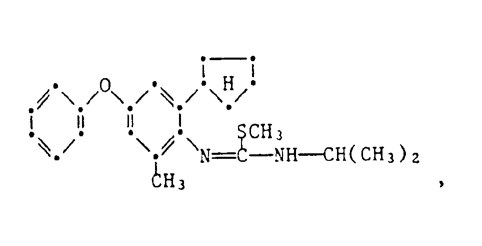

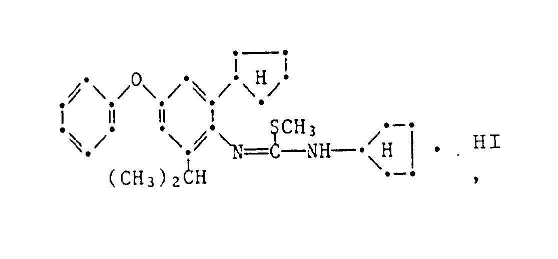

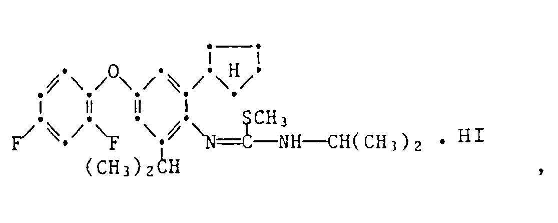

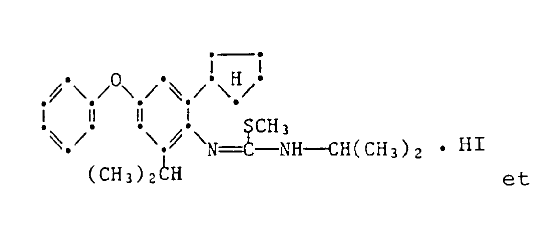

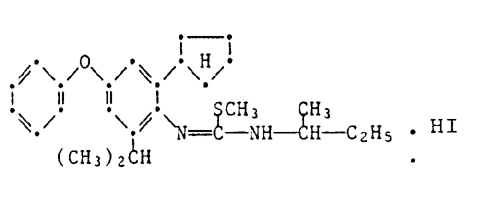

8.- Composés selon l'une des revendications 1 ou 5, de formules 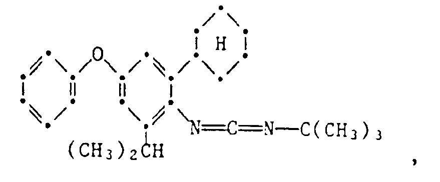 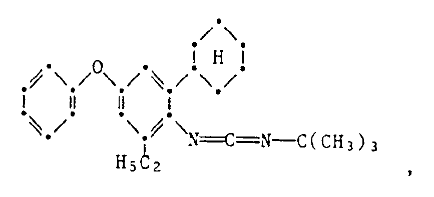 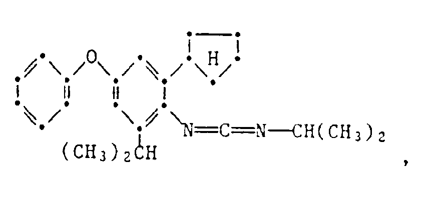 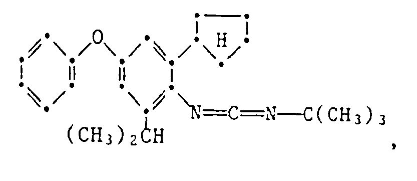 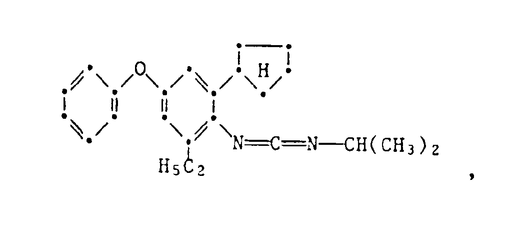 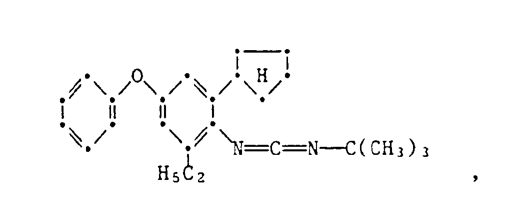 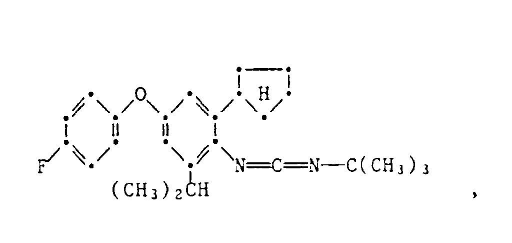 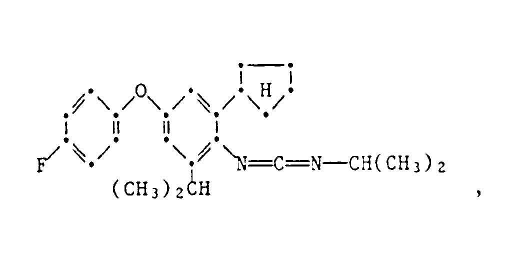 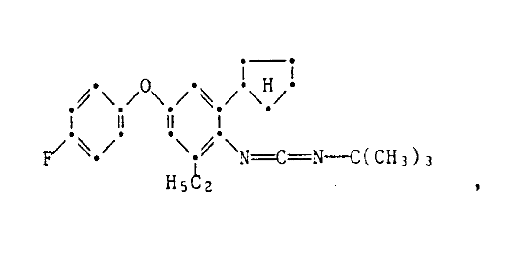 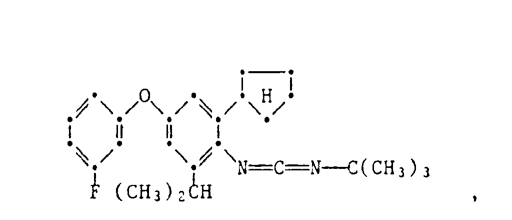 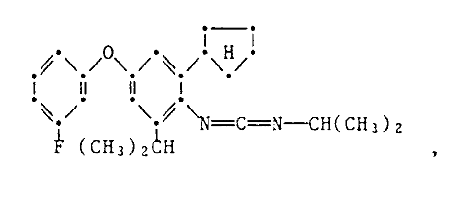 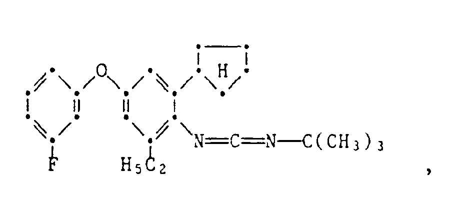 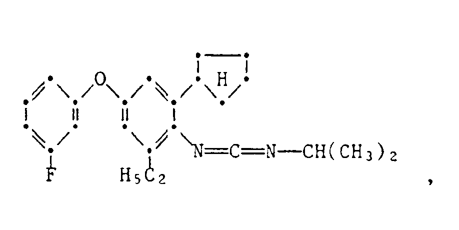 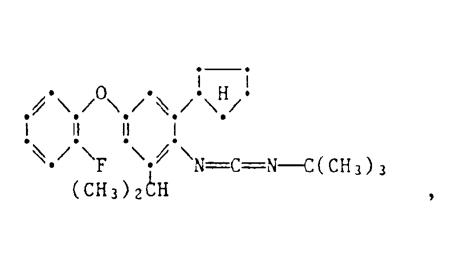 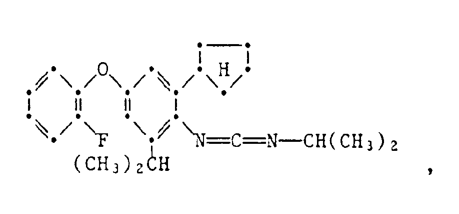 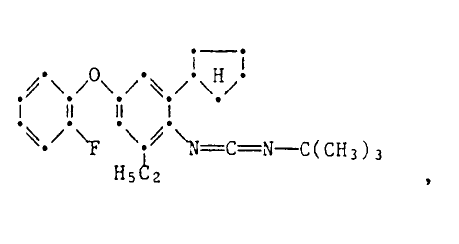 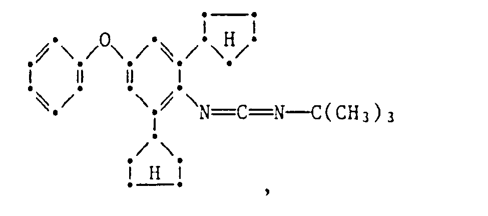 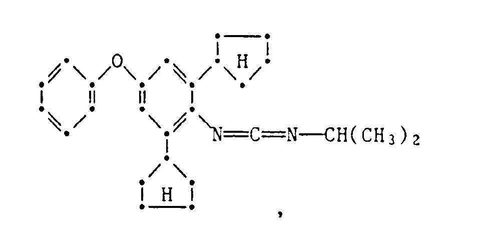 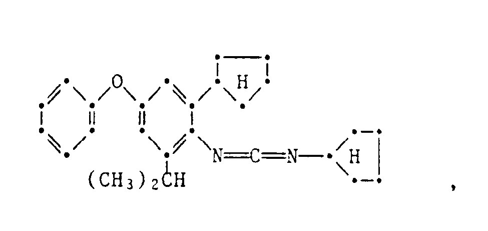 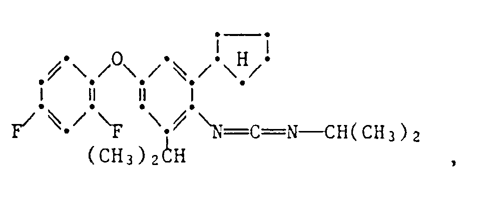 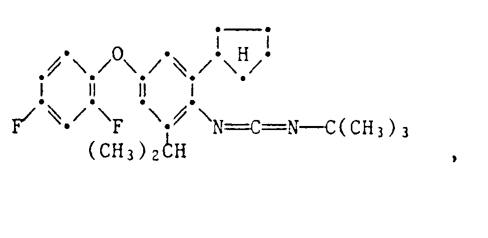 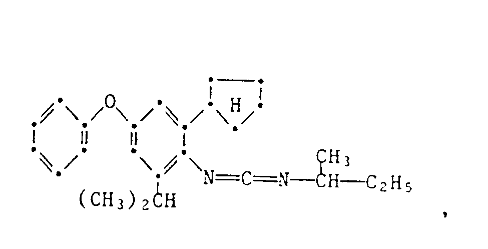 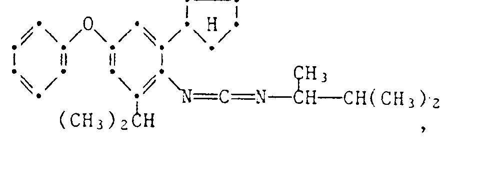 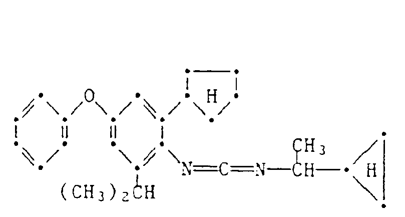 et

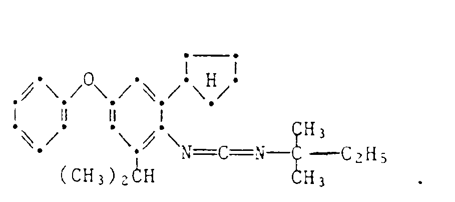

9. Composés de formule V 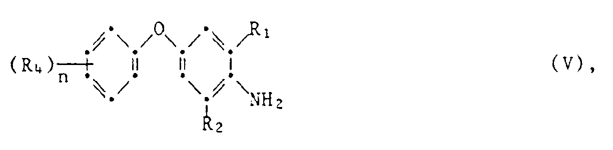
dans laquelle
R₁ représente un groupe cycloalkyle en C₃-C₇ ou cycloalcényle en C₅-C₆,
R₂ représente un groupe alkyle en C₁-C_{6,} cycloalkyle en C₅-C₆ ou cycloalcényle en C₅-C₆, chacun des symboles R₄ représente l'hydrogène, un halogène, un groupe alkyle en C₁-C_{4,}alcoxy en C₁-C₃ ou CF₃, et
n es un nombre allant de 1 à 3.

10. Composés de formule V selon la revendication 9, dans laquelle R₁ représente un groupe cycloalkyle en C₃-C₇, R₂ représente un groupe alkyle en C₁-C₄ ou cyclopentyle, R₄ l'hydrogène, un halogène ou un groupe alkyle en C₁-C₃ et n est égal à 1 ou 2.

11. Composés de formule V selon revendication 10, dans laquelle R₁ représente un groupe cycloalkyle en C₅-C_{6,} R₂ un groupe éthyle ou isopropyle, R₄ l'hydrogène ou le fluor, et n est égal à 1 ou 2.

12. Composés selon l'une des revendications 10 ou 11, de formules

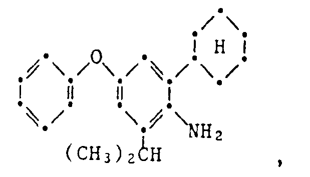

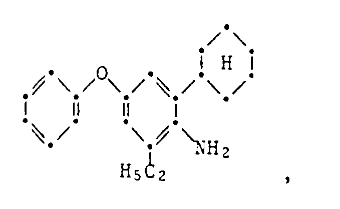

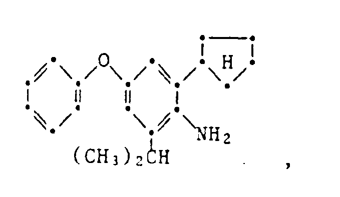

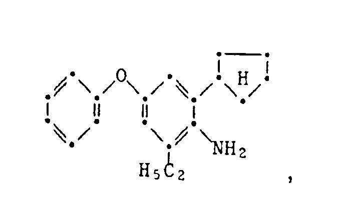

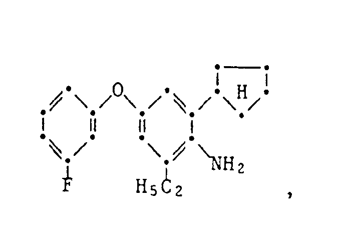

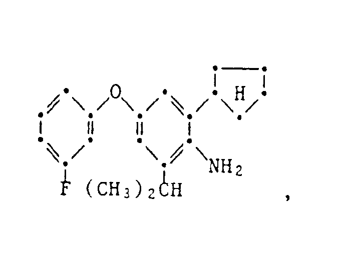

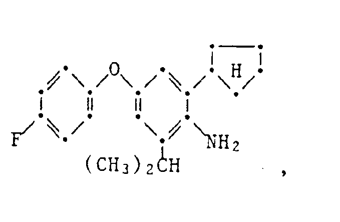

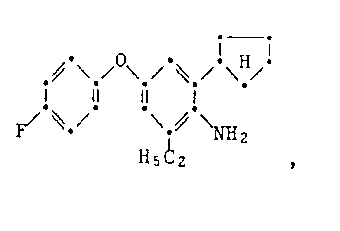

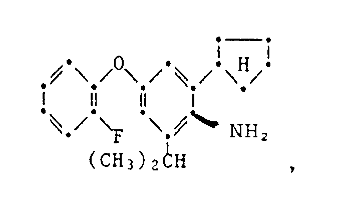

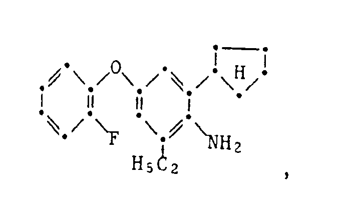

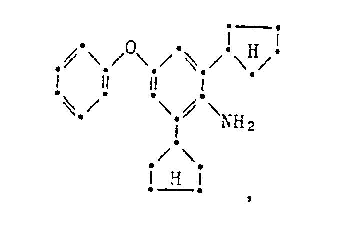

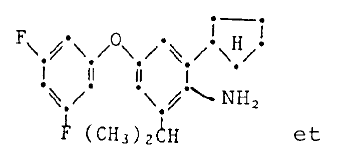

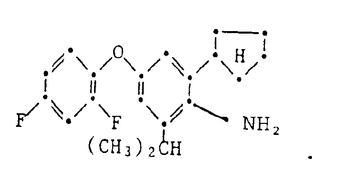

13. Composés de formule II 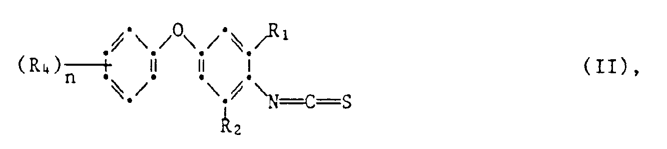
dans laquelle
R₁ représente un groupe cycloalkyle en C₃-C₇ ou cycloalcényle en C₅-C₆,
R₂ représente un groupe alkyle en C₁-C₄ ou cycloalkyle en C₅-C₆, chacun des symboles R₄ représente l'hydrogène, un halogène, un groupe alkyle en C₁-C₄, alcoxy en C₁-C₃ ou CF₃, et
n est un nombre allant de 1 à 3.

14. Composés de formule II selon revendication 13, dans laquelle R₁ représente un groupe cycloalkyle en C₃-C₇, R₂ un groupe alkyle en C₁-C₄, R₄ l'hydrogène, un halogène ou un groupe alkyle en C₁-C₃ et n est égal à 1 ou 2.

15. Composés de formule II selon revendication 14, dans laquelle R₁ représente un groupe cycloalkyle en C₅-C₆, R₂ un groupe éthyle ou isopropyle, R₄ l'hydrogène ou le fluor, et n est égal à 1 ou 2.

16. Composés selon l'une des revendications 14 ou 15, de formules

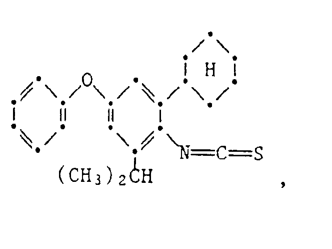

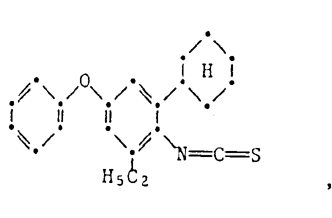

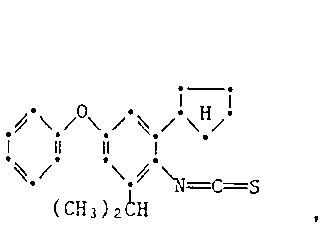

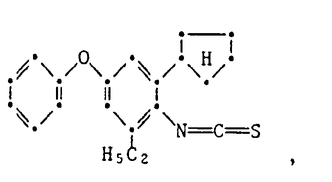

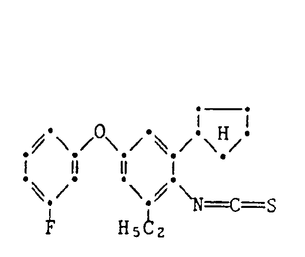

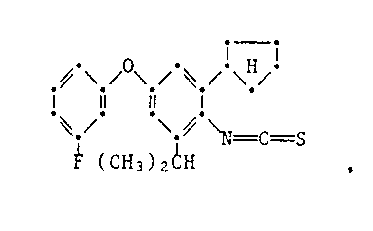

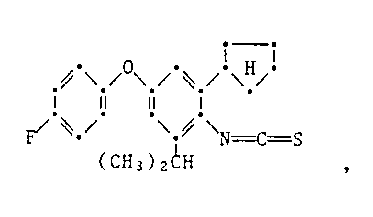

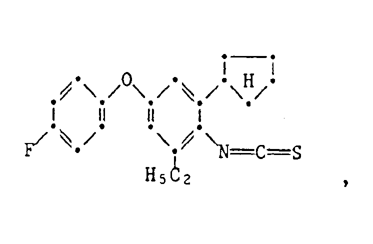

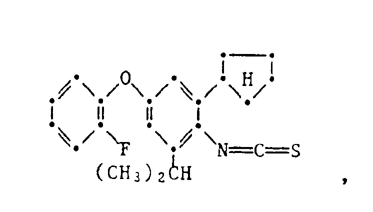

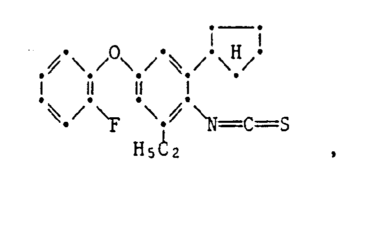

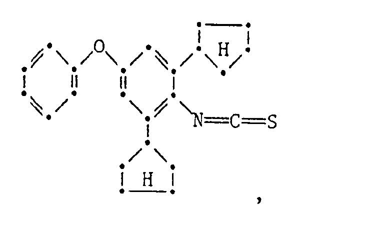

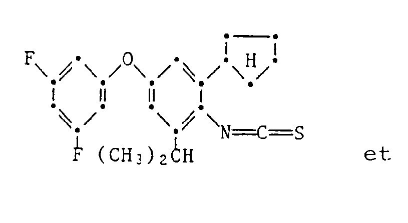

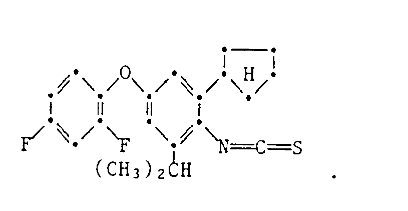

17. Procédé de préparation d'un composé de formule 1 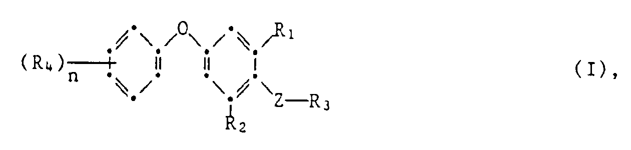
dans laquelle
R₁ représente un groupe cycloalkyle en C₃-C₇ ou cycloalcényle en C₅-C_{6,}
R₂ représente un groupe alkyle en C₁-C_{6,} cycloalkyle en C₅-C₆ ou cycloalcényle e C₅-C₆,
R₃ représente un groupe alkyle en C₁-C_{8,} cycloalkyle en C₃-C₆, 1-cyclopropyléthyle ou alcényle en C₃-C₅, chacun des symboles R₄ représente l'hydrogène, un halogène, un groupe alkyle en C₁-C₄, alcoxy en C₁-C₃ ou CF₃,
n est un nombre allant de 1 à 3,
Z représente -NH-CS-NH-, -N=C(SR₅)-NH- ou -N=C=N-, et
R₅ représente un groupe alkyle en C₁-C₅ ou allyle, caractérisé en ce que :
A) on fait réagir un isothiocyanate de formule II 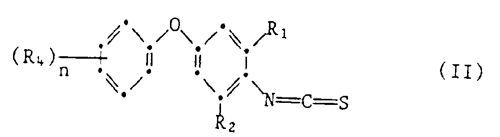 avec une amine de formule III 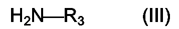
dans un solvant ou diluant organique à pression normale et à une température de 0 à +150_{°}C, la réaction donnant une thiourée, et
B) on fait réagir éventuellement cette thiourée avec un composé de formule IV 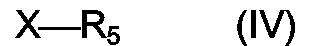 dans un solvant organique inerte à pression normale ou légèrement supérieure à la normale et à une température de +10 à +250_{°}C, la réaction donnant une isothiourée, ou bien
C) on convertit la thiourée, par élimination d'hydrogène sulfuré, dans un solvant ou diluant organique apro- tonique, à pression normale et à une température de 0 à +150°C, en le carbodiimide, les symboles R_{1,} R₂, R₃, R₄, R₅, et n ayant les significations indiquées ci-dessus et X représentant un groupe éliminable.

18. Procédé de préparation d'un composé de formule V 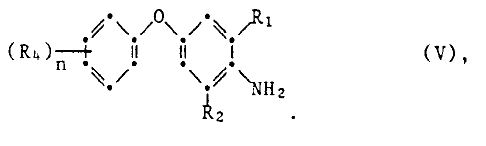
dans laquelle
R₁ représente un groupe cycloalkyle en C₃-C₇ ou cycloalcényle en C₅-C_{6,}
R₂ représente un groupe alkyle en C₁-C_{6,} cycloalkyle en C₅-C₆ ou cycloalcényle en C₅-C₆, chacun des symboles R₄ représente l'hydrogène, un halogène, un groupe alkyle en C₁-C₄, alcoxy en C₁-C₃ ou CF₃, et
n est un nombre allant de 1 à 3,
caractérisé en ce que l'on fait réagir un phénol de formule VII 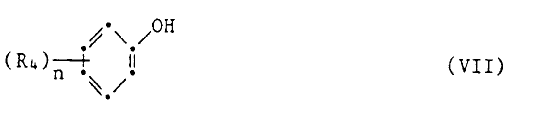
en présence d'une base organique ou minérale, à pression normale et à une température de 0 à +100_{°}C, dans un solvant ou diluant inerte, avec une aniline de formule VI 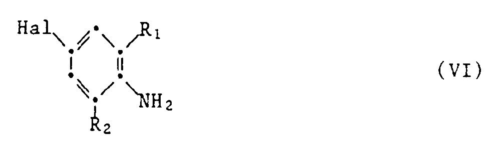
les symboles R_{1,} R₂, R₄ et n ayant les significations indiquées ci-dessus et Hal représentant un atome d'halogène.

19. Procédé de préparation d'un composé de formule II 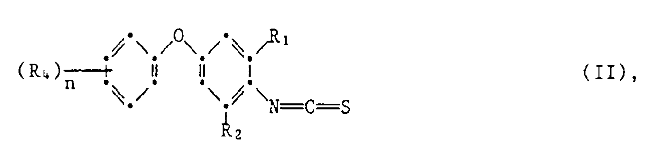
dans laquelle
R₁ représente un groupe cycloalkyle en C₃-C₇ ou cycloalcényle en C₅-C_{6,}
R₂ représente un groupe alkyle en C₁-C_{6,} cycloalkyle en C₅-C₆ ou cycloalcényle en C₅-C_{6,} chacun des symboles R₄ représente l'hydrogène, un halogène, un groupe alkyle en C₁-C_{4,} alcoxy en C₁-C₃ ou CF₃, et
n est un nombre allant de 1 à 3, caractérisé en ce que l'on fait réagir une phénoxyaniline de formule V 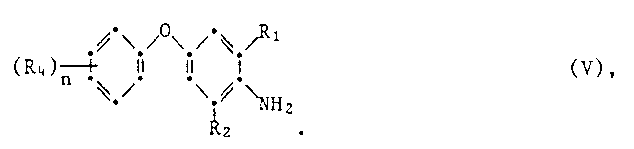 en présence d'une base organique ou minérale, à pression normale et à une température de 0 à +100_{°}C, dans un solvant ou diluant inerte, avec le thiophosgène, les symboles R_{1,} R₂, R₄ et n ayant les significations indiquées ci-dessus.

20. Produit pesticide contenant au moins un composant actif qui consiste en un composé de formule 1 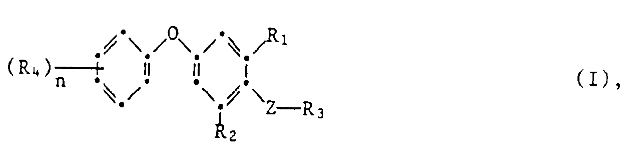
dans laquelle
R₁ représente un groupe cycloalkyle en C₃-C₇ ou cycloalcényle en C₅-C_{6,}
R₂ représente un groupe alkyle en C₁-C₆, cycloalkyle en C₅-C₆ ou cycloalcényle en C₅-C_{6,}
R₃ représente un groupe alkyle en C₁-C_{8,} cycloalkyle en C₃-C_{6,} 1-cyclopropyléthyle ou alcényle en C₃-C₅, chacun des symboles R₄ représente l'hydrogène, un halogène, un groupe alkyle en C₁-C_{4,} alcoxy en C₁-C₃ ou CF₃,
n est un nombre allant de 1 à 3,
Z représente -NH-CS-NH-, -N=C(SR₅)-NH- ou -N=C=N-, et
R₅ représente un groupe alkyle en C₁-C₅ ou allyle, ou l'un de ses sels d'acides organiques ou minéraux avec des véhicules et/ou additifs appropriés.

21. Produit pesticide selon revendication 20, contenant au moins un composant actif qui consiste en un composé de formule I dans laquelle R₁ représente un groupe cycloalkyle en C₃-C₇, R₂ un groupe alkyle en C₁-C₄ ou cyclopentyle, R₃ un groupe alkyle en C₁-C₄ ou cycloalkyle en C₃-C₅, R₄ l'hydrogène, un halogène ou un groupe alkyle en C₁-C_{3,} n est égal à 1 ou 2, Z représente -NH-CS-NH-, -N=C(SR₅)-NH- ou -N=C=N- et R₅ un groupe alkyle en C₁-C₃.

22. Produit pesticide selon revendication 21, contenant au moins un composant actif qui consiste en un composé de formule I dans laquelle R₁ représente un groupe cycloalkyle en C₅-C_{6,} R₂ un groupe éthyle ou isopropyle, R₃ un groupe isopropyle, tert-butyle ou cyclopentyle, R₄ l'hydrogène ou le fluor, n est égal à 1 ou 2 et Z représente -NH-CS-NH-.

23. Produit pesticide selon revendication 21, contenant au moins un composant actif qui consiste en un composé de formule I dans laquelle R₁ représente un groupe cycloalkyle en C₅-C_{6,} R₂ un groupe éthyle ou isopropyle, R₃ un groupe isopropyle, tert-butyle ou cyclopentyle, R₄ l'hydrogène ou le fluor, n est égal à 1 ou 2, Z représente -N=C(SR₅)-NH- et R₅ un groupe méthyle ou éthyle.

24. Produit pesticide selon revendication 21, contenant au moins un composant actif qui consiste en un composé de formule I dans laquelle R₁ représente un groupe cycloalkyle en C₅-C_{6,} R₂ un groupe éthyle ou isopropyle, R₃ un groupe isopropyle, tert-butyle ou cyclopentyle, R₄ l'hydrogène ou le fluor, n est égal à 1 ou 2 et Z représente -N=C=N-.

25. Utilisation d'un composé de formule I 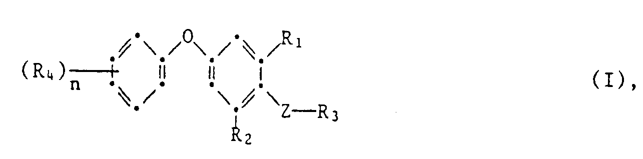
dans laquelle
R₁ représente un groupe cycloalkyle en C₃-C₇ ou cycloalcényle en C₅-C₆,
R₂ représente un groupe alkyle en C₁-C_{6,} cycloalkyle en C₅-C₆ ou cycloalcényle en C₅-C₆,
R₃ représente un groupe alkyle en C₁-C_{8,} cycloalkyle en C₃-C_{6,} 1-cyclopropyléthyle ou alcényle en C₃-C₅, chacun des symboles R₄ représente l'hydrogène, un halogène, un groupe alkyle en C₁-C_{4,} alcoxy en C₁-C₃ ou CF₃,
n est un nombre allant de 1 à 3,
Z représente -NH-CS-NH-, -N=C(SR₅)-NH-, -N=C=N-, et
R₅ représente un groupe alkyle en C₁-C₅ ou allyle, ou de l'un de ses sels d'acide organique ou minéral pour la lutte contre les parasites des animaux et des végétaux.

26. Utilisation selon revendication 25, d'un composé de formule I pour la lutte contre les insectes et les arachnides.

27. Procédé pour combattre les parasites des animaux et des végétaux, caractérisé en ce que l'on fait entrer les parasites, à un stade quelconque du développement, en contact avec un composé de formule I 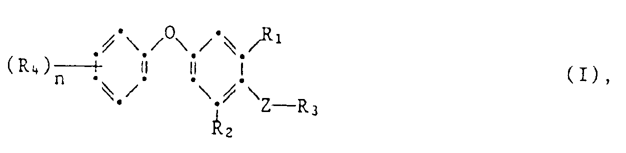
dans laquelle
R₁ représente un groupe cycloalkyle en C₃-C₇ ou cycloalcényle en C₅-C₆,
R₂ représente un groupe alkyle en C₁-C_{6,} cycloalkyle en C₅-C₆ ou cycloalcényle en C₅-C₆,
R₃ représente un groupe alkyle en C₁-C₈, cycloalkyle en C₃-C₆, 1-cyclopropyléthyle ou alcényle en C₃-C₅, chacun des symboles R₄ représente l'hydrogène, un halogène, un groupe alkyle en C₁-C₄, alcoxy en C₁-C₃ ou CF₃,
n est un nombre allant de 1 à 3,
Z représente -NH-CS-NH-, -N=C(SR₅)-NH-, -N=C=N-, et
R₅ représente un groupe alkyle en C₁-C₅ ou allyle, ou l'un de ses sels d'acide organique ou minéral.

## Revendications (Revendications pour l'(les) Etat(s) contractant(s) suivant(s) : suivant : ES)

1. Procédé de préparation d'un composé de formule 1 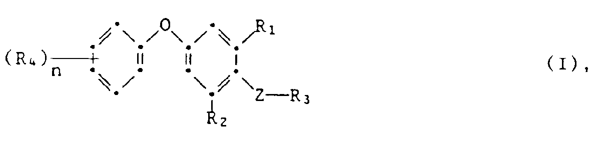
dans laquelle
R₁ représente un groupe cycloalkyle en C₃-C₇ ou cycloalcényle en C₅-C₆,
R₂ représente un groupe alkyle en C₁-C₆, cycloalkyle en C₅-C₆ ou cycloalcényle en C₅-C₆,
R₃ représente un groupe alkyle en C₁-C₈, cycloalkyle en C₃-C₆, 1-cyclopropyléthyle ou alcényle en C₃-C₅, chacun des symboles R₄ représente l'hydrogène, un halogène, un groupe alkyle en C₁-C₄, alcoxy en C₁-C₃ ou CF₃,
n est un nombre allant de 1 à 3,
Z représente -NH-CS-NH-, -N=C(SR₅)-NH-, -N=C=N-, et
R₅ représente un groupe alkyle en C₁-C₅ ou allyle, caractérisé en ce que :
A) on fait réagir un isothiocyanate de formule II 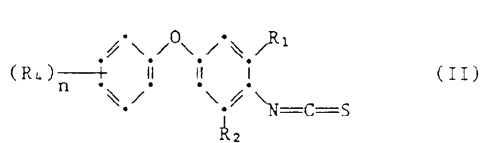 avec une amine de formule III 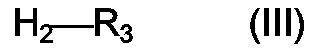
dans un solvant ou diluant organique, à pression normale et à une température de 0 à +150_{°}C, la réaction donnant une thiourée et le cas échéant
B) on fait réagir cette thiourée avec un composé de formule IV 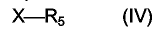
dans un solvant organique inerte, à pression normale ou légèrement supérieure à la normale et à une température de +10 à +250_{°}C, la réaction donnant une isothiourée, ou bien
C) on convertit la thiourée, par scission d'hydrogène sulfuré dans un solvant ou diluant organique aproto- nique, à pression normale et à une température de 0 à +150°C, en un carbodiimide, les symboles R_{1,} R₂, R₃, R₄, R₅ et n ayant les significations indiquées ci-dessus et X représentant un groupe éliminable.

2. Procédé selon la revendication 1, dans lequel R₁ représente un groupe cycloalkyle en C₃-C₇ R₂ représente un groupe alkyle en C₁-C₄ ou cyclopentyle, R₃ représente un groupe alkyle en C₁-C₄ ou cycloalkyle en C₃-C₅, R₄ représente l'hydrogène, un halogène ou un groupe alkyle en C₁-C₃, n est égal à 1 ou 2, Z représente -NH-CS-NH-, -N=C(SR₅)-NH-, -N=C=N- et R₅ représente un groupe alkyle en C₁-C₃.

3. Procédé selon la revendication 2, dans lequel R₁ représente un groupe cycloalkyle en C₅-C₆, R₂ un groupe éthyle ou isopropyle, R₃ un groupe isopropyle, tert-butyle ou cyclopentyle, R₄ l'hydrogène ou le fluor, n est égal à 1 ou 2 et Z représente -NH-CS-NH-.

4. Procédé selon la revendication 2, dans lequel R₁ représente un groupe cycloalkyle en C₅-C₆, R₂ un groupe éthyle ou isopropyle, R₃ un groupe isopropyle, tert-butyle ou cyclopentyle, R₄ l'hydrogène ou le fluor, n est égal à 1 ou 2, Z représente -N=C(SR₅)-NH- et R₅ un groupe méthyle ou éthyle.

5. Procédé selon la revendication 2, dans lequel R₁ représente un groupe cycloalkyle en C₅-C₆, R₂ un groupe éthyle ou isopropyle, R₃ un groupe isopropyle, tert-butyle ou cyclopentyle, R₄ représente l'hydrogène ou le fluor, n est égal à 1 ou 2, et Z représente -N=C=N-.

6. Procédé selon l'une des revendications 1 ou 3, pour la préparation des composés de formules

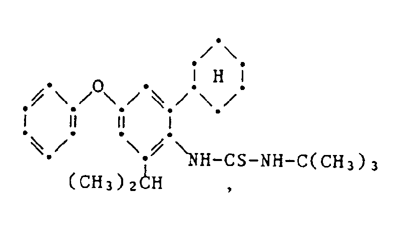

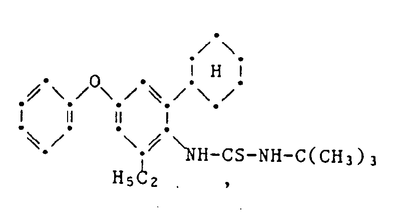

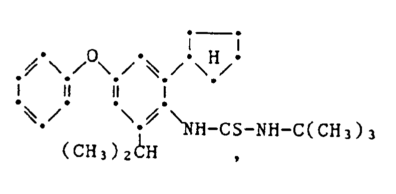

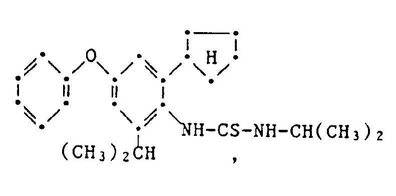

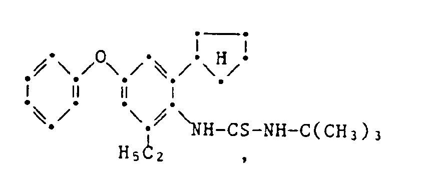

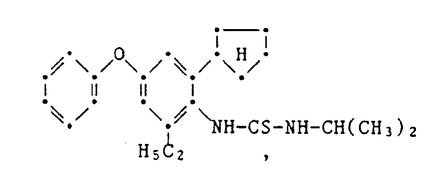

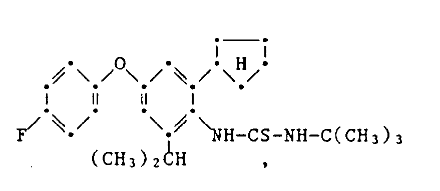

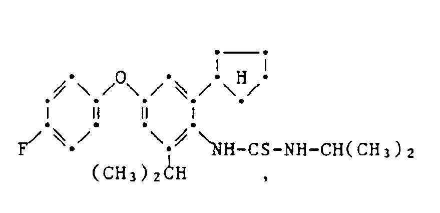

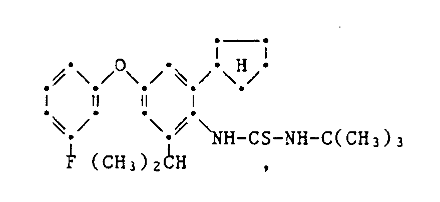

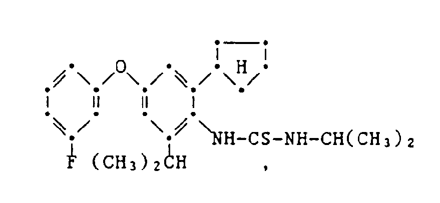

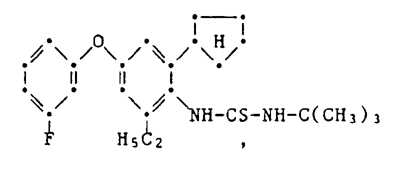

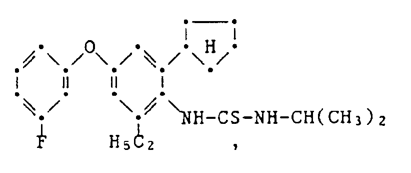

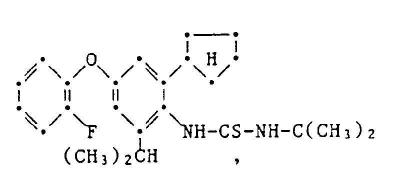

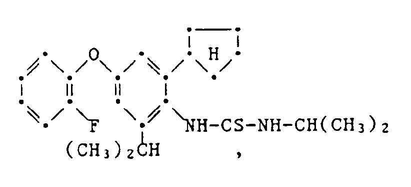

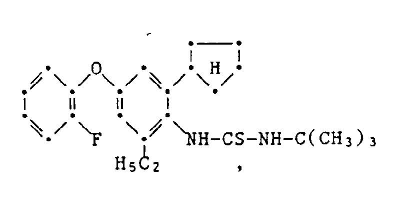

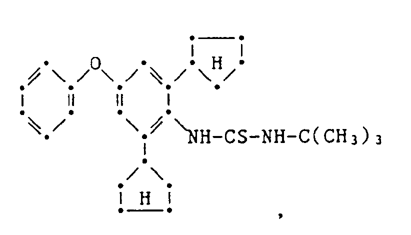

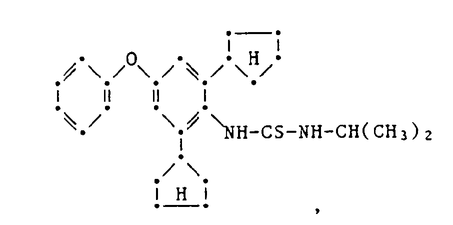

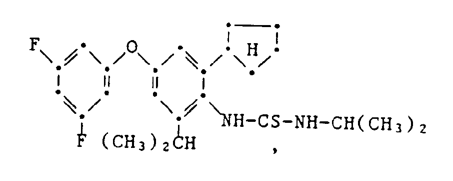

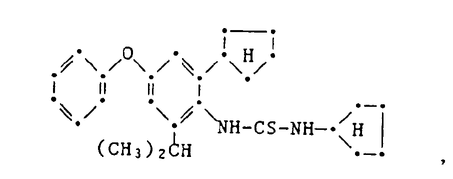

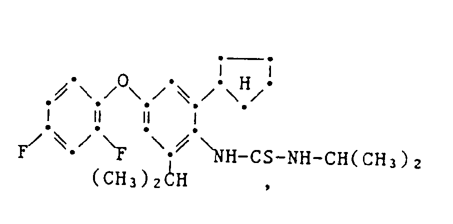

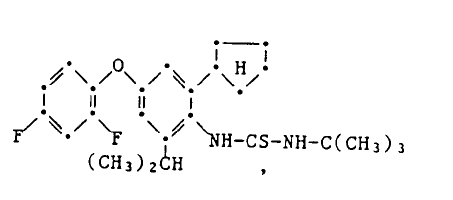

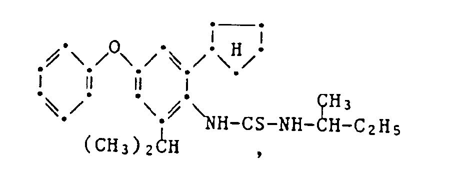

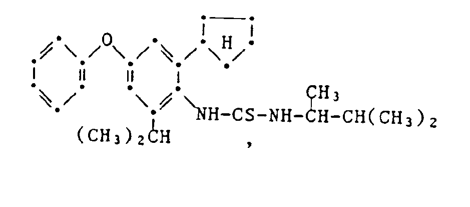

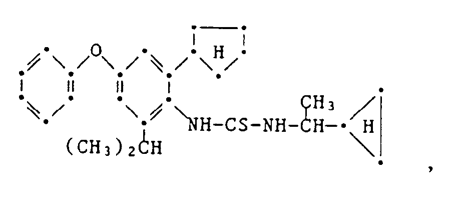

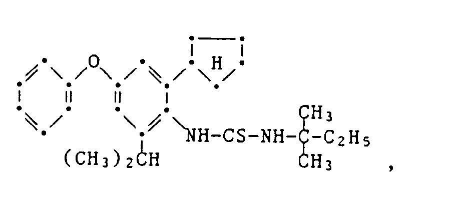

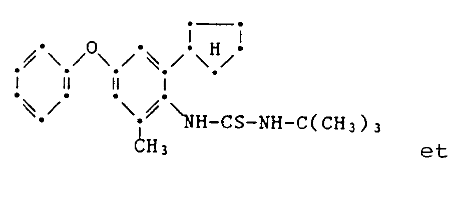

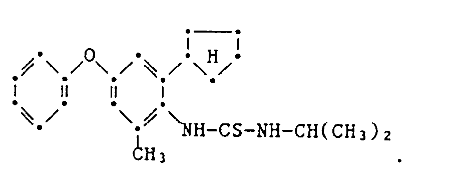

7. Procédé selon l'une des revendications 1 ou 4, pour la préparation des composés de formules

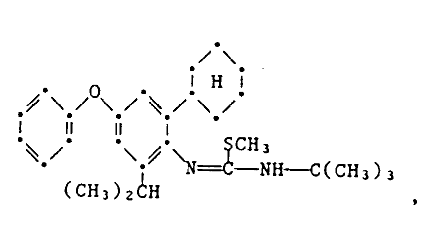

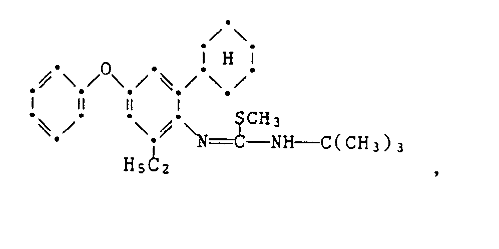

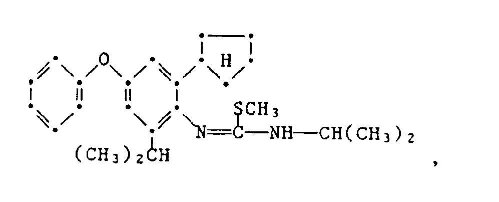

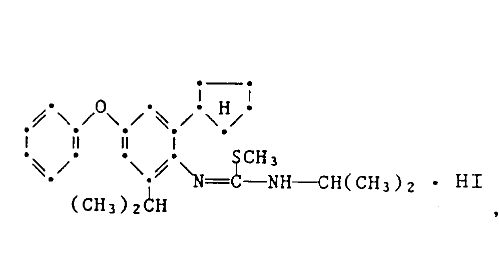

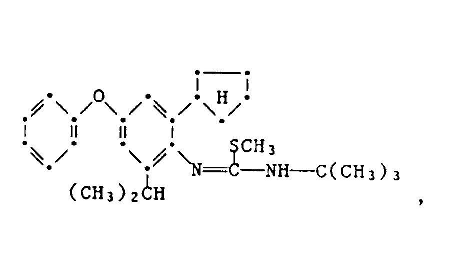

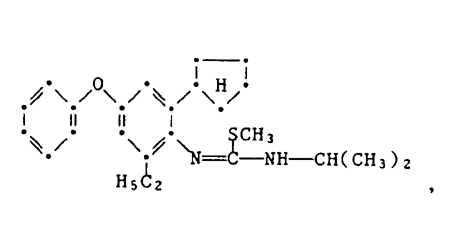

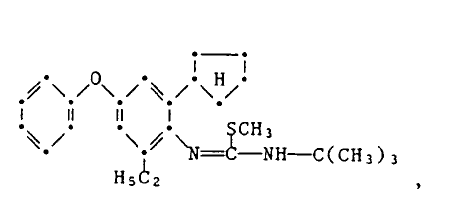

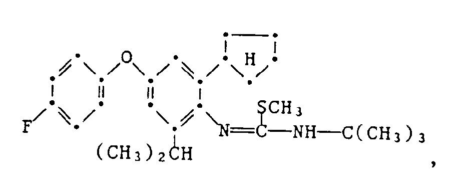

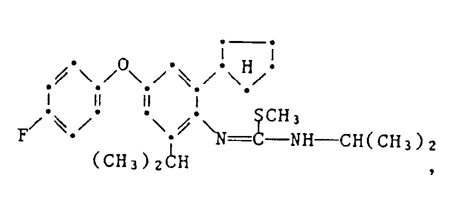

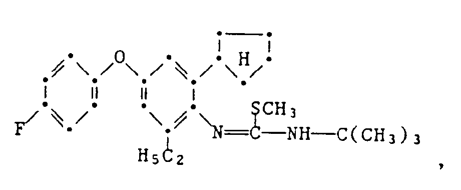

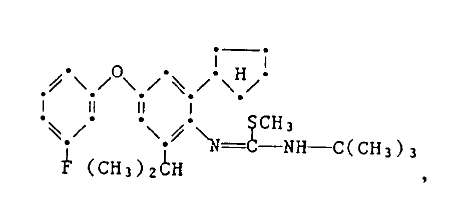

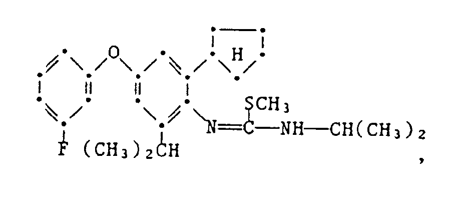

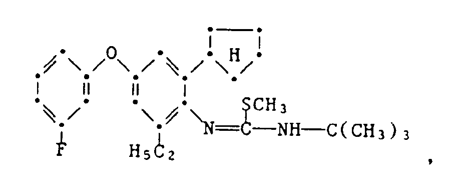

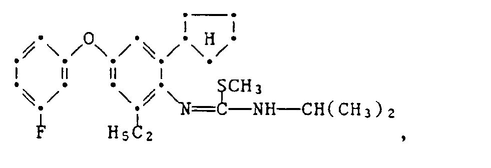

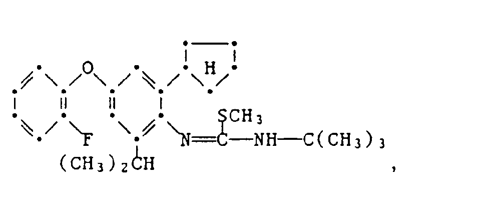

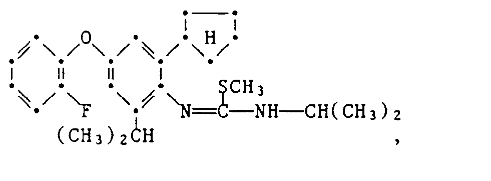

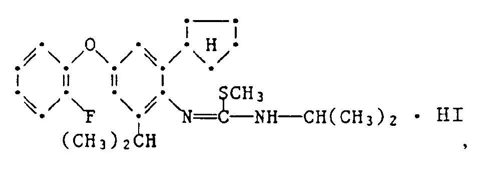

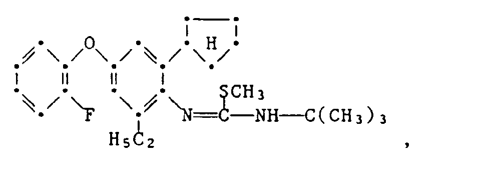

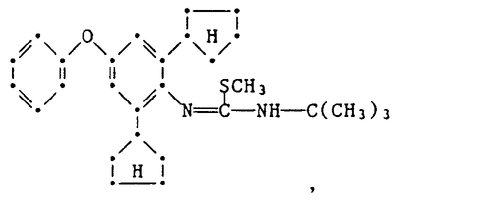

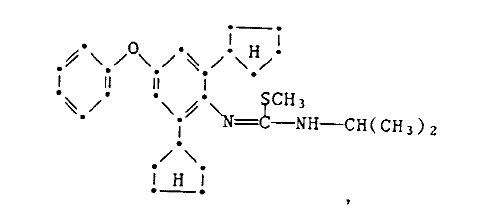

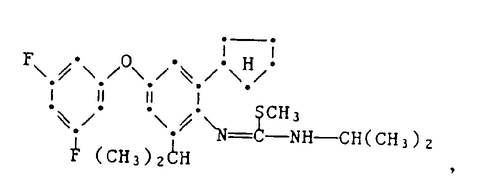

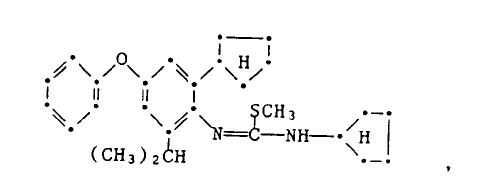

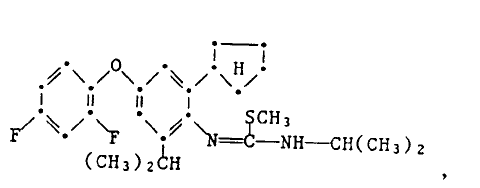

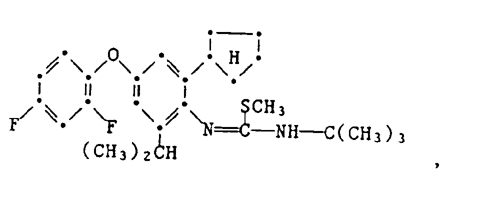

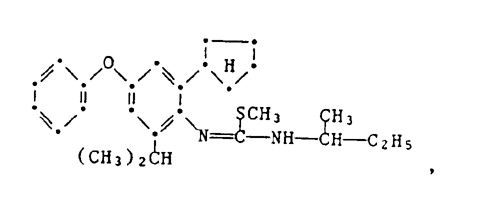

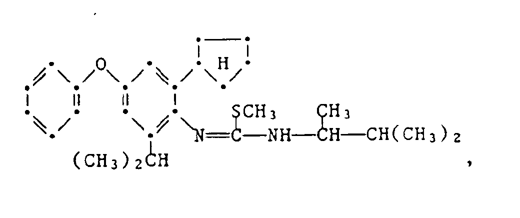

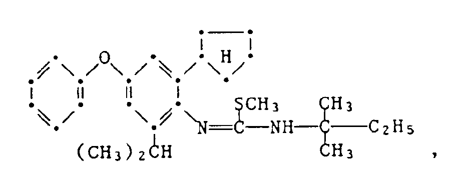

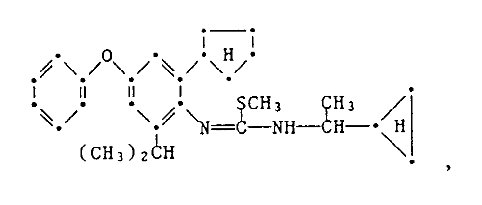

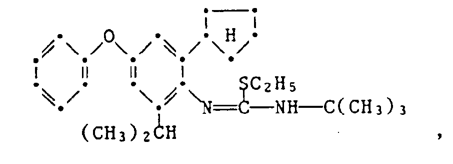

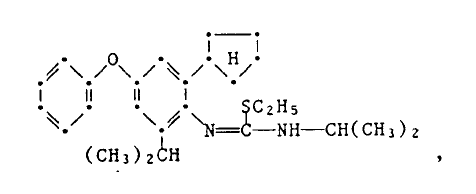

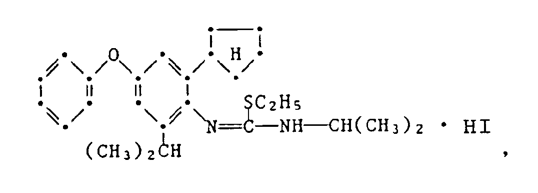

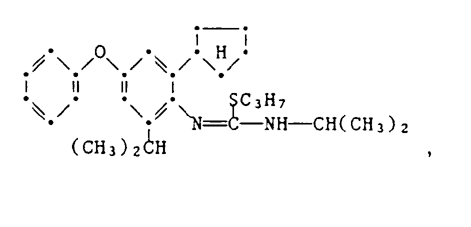

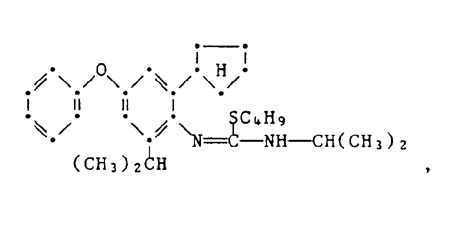

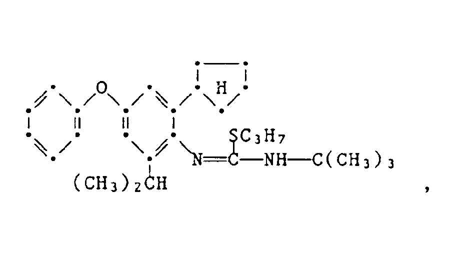

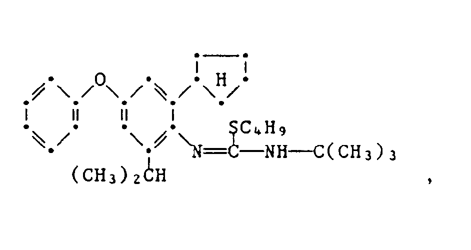

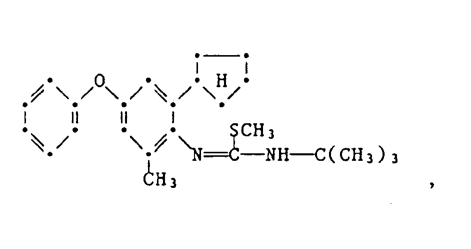

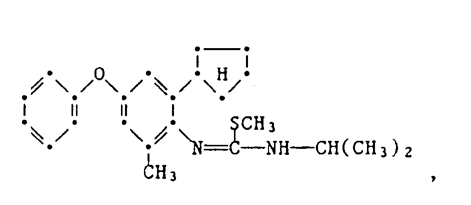

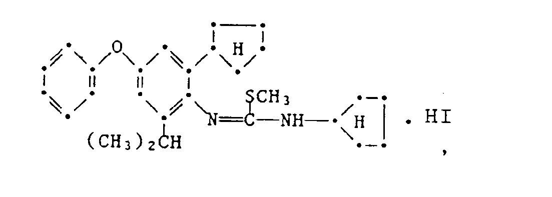

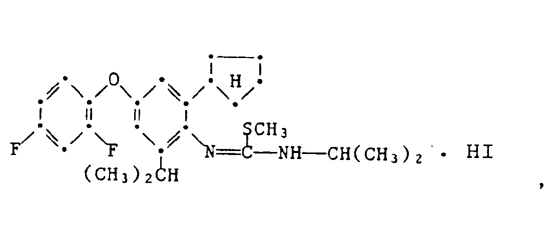

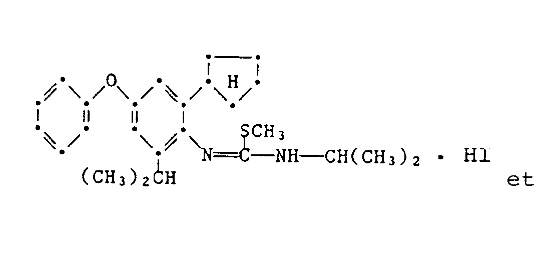

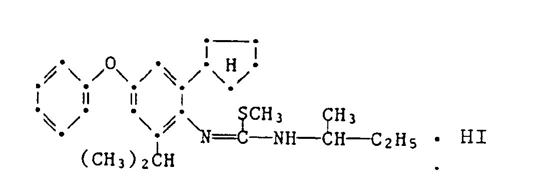

8. Procédé selon l'une des revendications 1 ou 5, pour la préparation des composés de formules

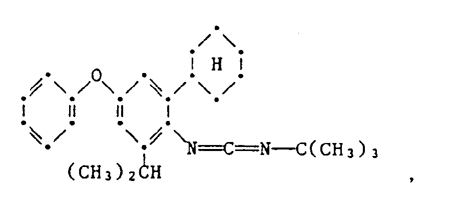

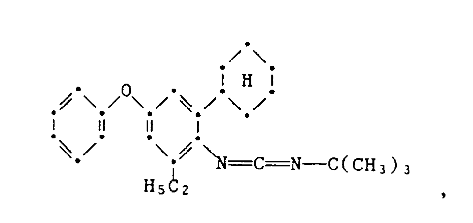

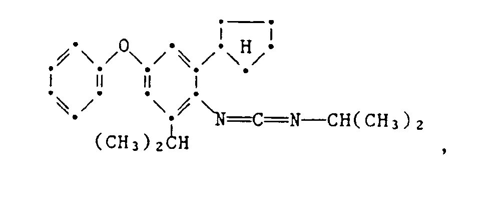

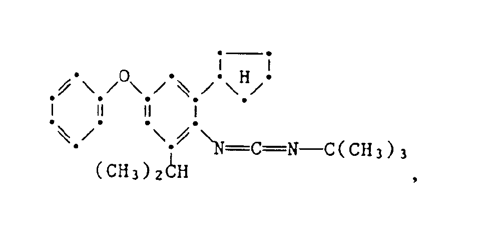

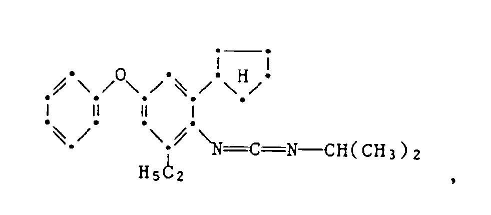

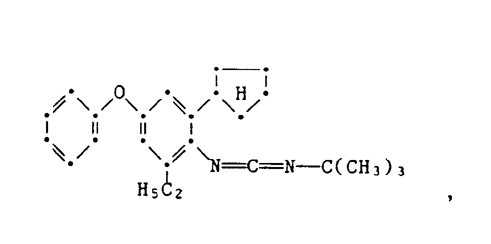

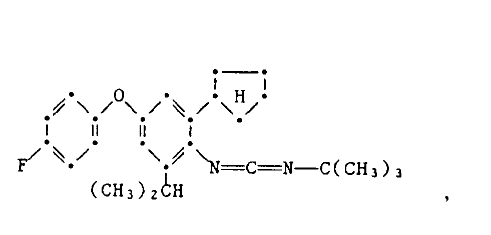

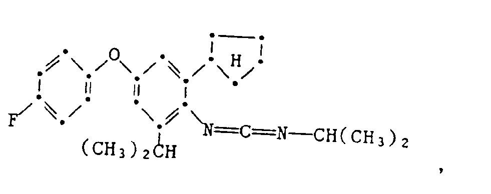

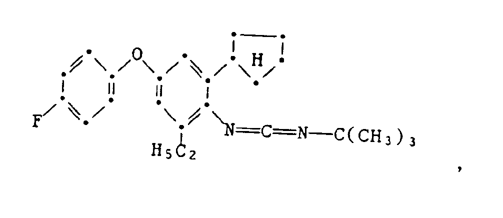

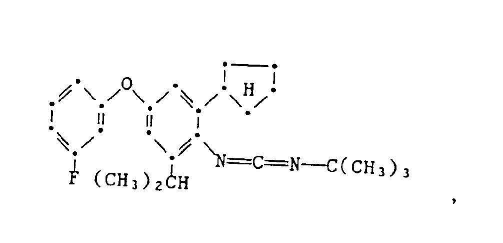

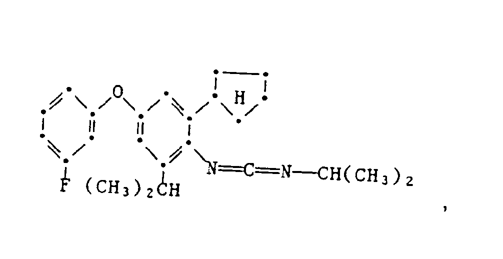

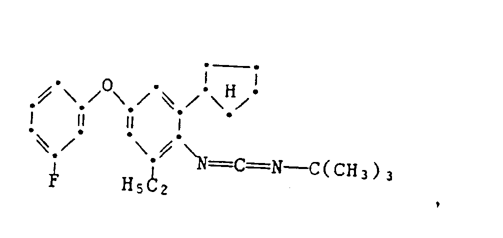

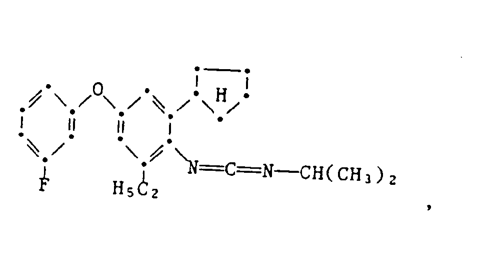

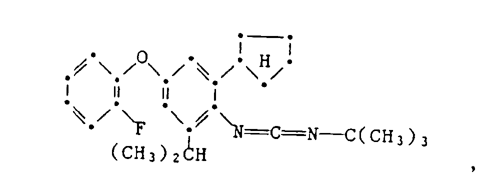

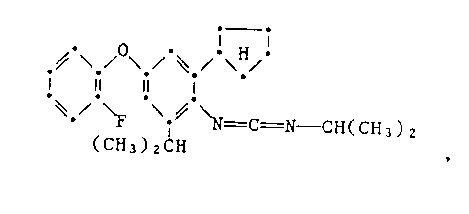

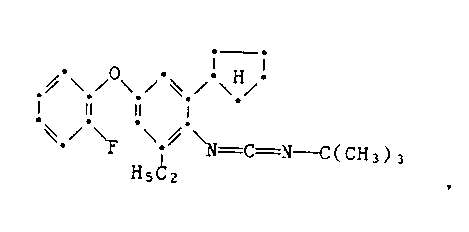

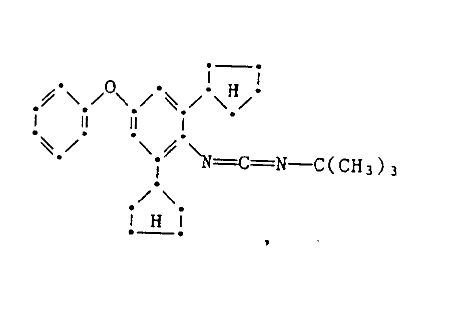

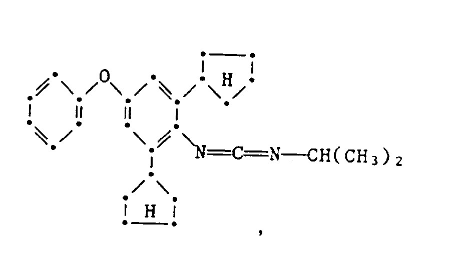

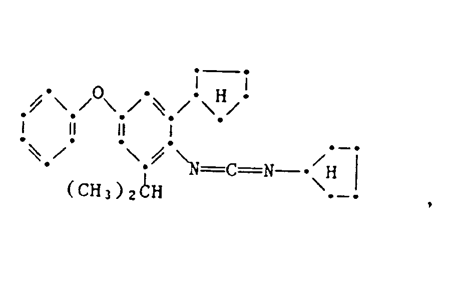

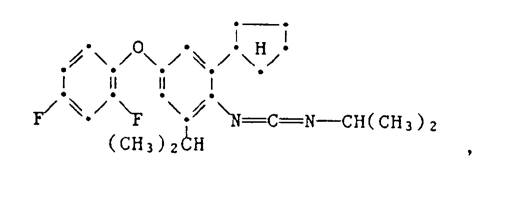

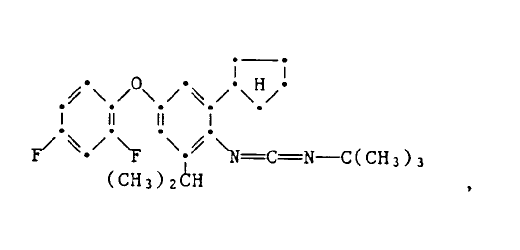

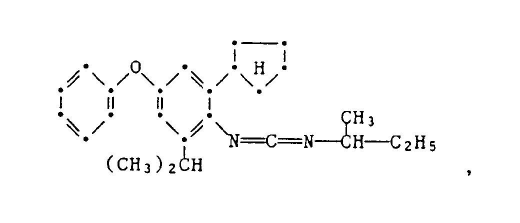

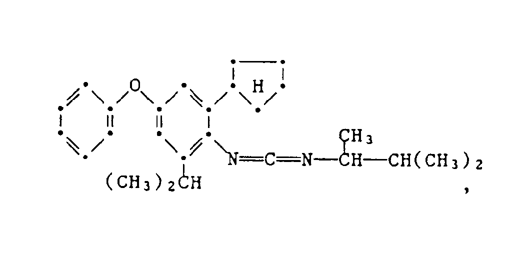

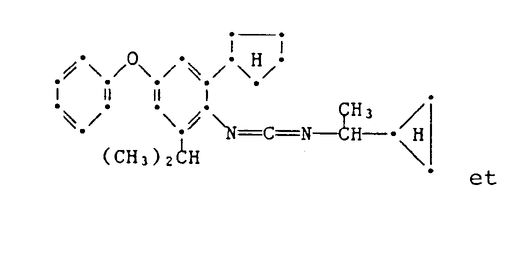

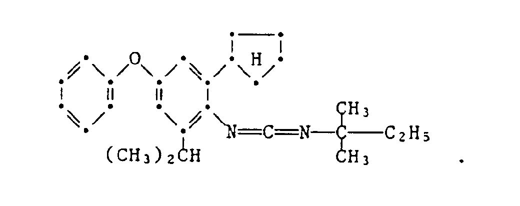

9. Procédé de préparation d'un composé de formule V 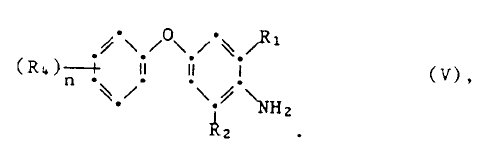
dans laquelle
R₁ représente un groupe cycloalkyle en C₃-C₇ ou cycloalcényle en C₅-C₆,
R₂ représente un groupe alkyle en C₁-C₆, cycloalkyle en C₅-C₆ ou cycloalcényle en C₅-C₆, chacun des symboles R₄ représente l'hydrogène, un halogène, un groupe alkyle en C₁-C₄, alcoxy en C₁-C₃ ou CF₃, et
n est un nombre allant de 1 à 3, caractérisé en ce que l'on fait réagir un phénol de formule VII 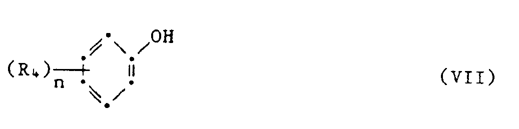
en présence d'une base organique ou minérale, à pression normale et à une température de 0 à +100_{°}C, dans un solvant ou diluant inerte, avec une aniline de formule VI 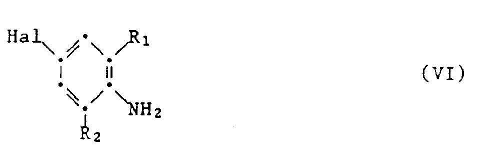
les symboles R_{1'} R₂, R₄ et n ayant les significations indiquées ci-dessus et Hal représentant un atome d'halogène.

10. Procédé selon la revendication 9, dans lequel R₁ représente un groupe cycloalkyle en C₃-C₇, R₂ un groupe alkyle en C₁-C₄ ou cyclopentyle, R₄ l'hydrogène, un halogène ou un groupe alkyle en C₁-C₃ et n est égal à 1 ou 2.

11. Procédé selon la revendication 10, dans lequel R₁ représente un groupe cycloalkyle en C₅-C₆, R₂ un groupe éthyle ou isopropyle, R₄ l'hydrogène ou le fluor et n est égal à 1 ou 2.

12. Procédé selon l'une des revendications 10 ou 11, pour la préparation des composés de formules

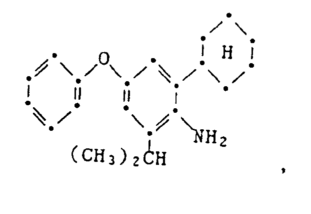

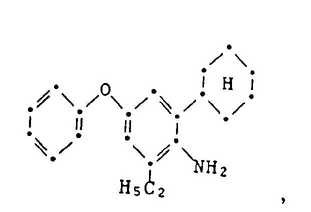

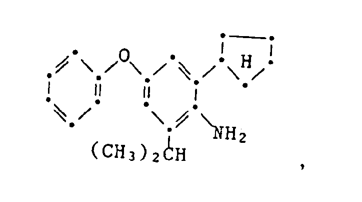

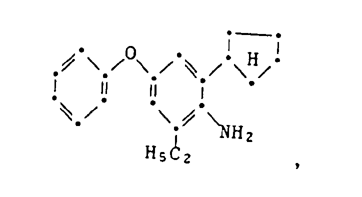

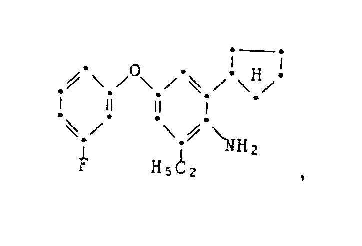

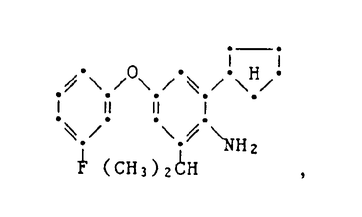

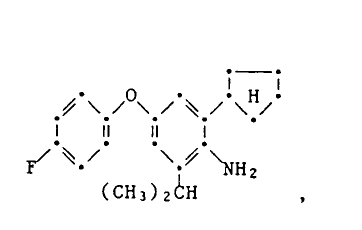

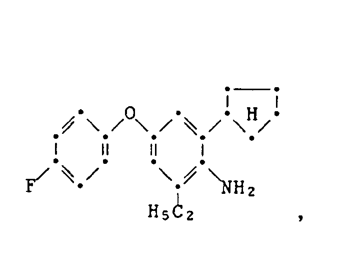

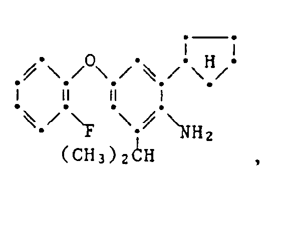

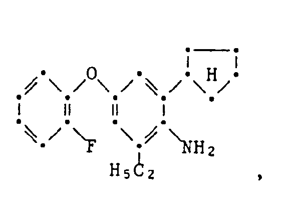

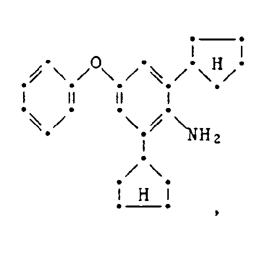

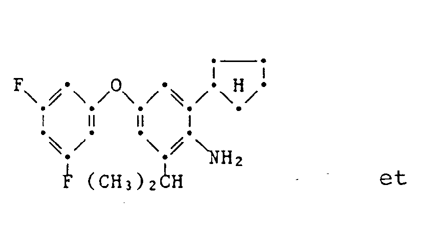

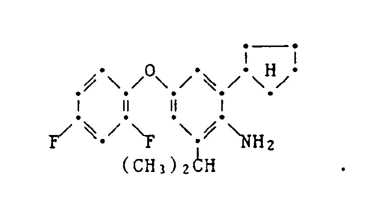

13. Procédé de préparation d'un composé de formule II 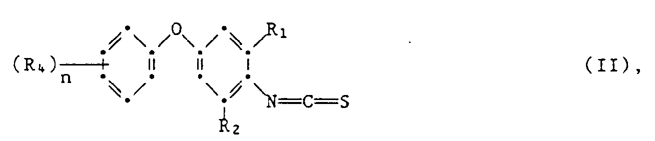
dans laquelle
R₁ représente un groupe cycloalkyle en C₃-C₇ ou cycloalcényle en C₅-C₆,
R₂ représente un groupe alkyle en C₁-C₆, cycloalkyle en C₅-C₆ ou cycloalcényle en C₅-C₆, chacun des symboles R₄ représente l'hydrogène, un halogène, un groupe alkyle en C₁-C₄, alcoxy en C₁-C₃ ou CF₃, et n est un nombre allant de 1 à 3, caractérisé en ce que l'on fait réagir une phénoxyaniline de formule V 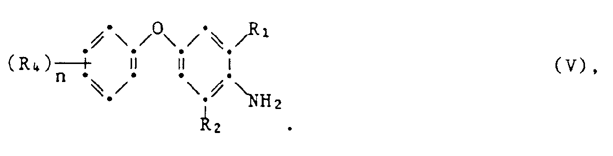 en présence d'une base organique ou minérale, à pression normale et à une température de 0 à 100°C, dans un solvant ou diluant inerte, avec le thiophosgène, R_{1'} R₂, R₄ et n ayant les significations indiquées ci-dessus.

14. Procédé selon la revendication 13, dans lequel R₁ représente un groupe cycloalkyle en C₃-C₇, R₂ un groupe alkyle en C₁-C₄, R₄ l'hydrogène, un halogène ou un groupe alkyle en C₁-C₃ et n est égal à 1 ou 2.

15. Procédé selon la revendication 14, dans lequel R₁ représente un groupe cycloalkyle en C₅-C₆, R₂ un groupe éthyle ou isopropyle, R₄ l'hydrogène ou le fluor et n est égal à 1 ou 2.

16. Procédé selon l'une des revendications 14 ou 15, pour la préparation des composés de formules 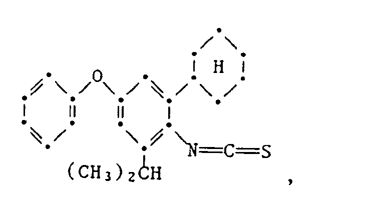 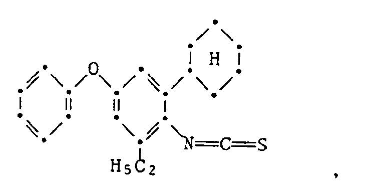 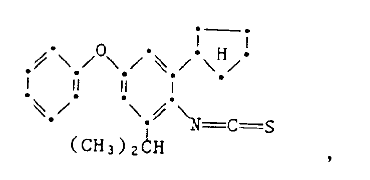 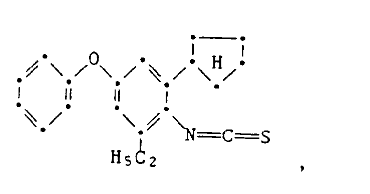 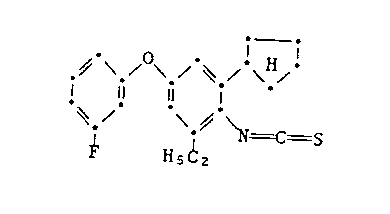 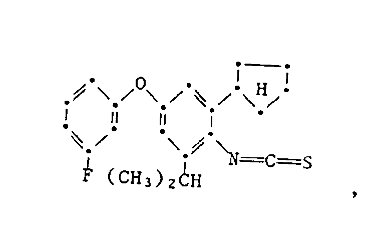 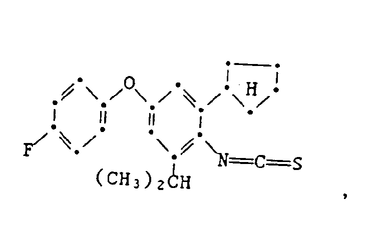 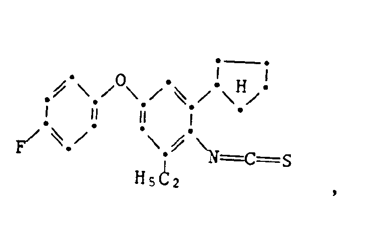 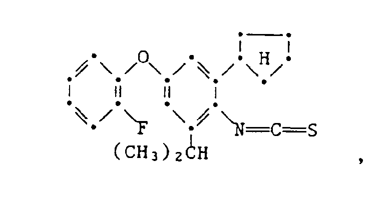 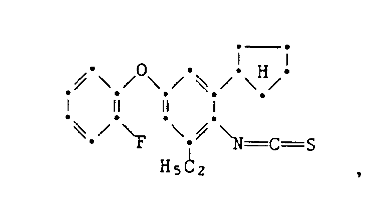 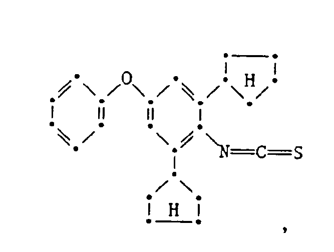 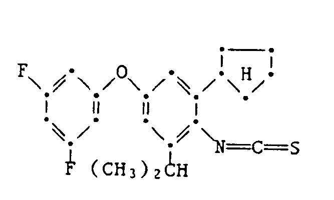 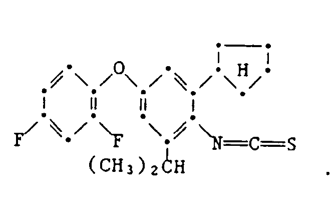 . et

17. Produit pesticide contenant au moins un composant actif qui consiste en un composé de formule I 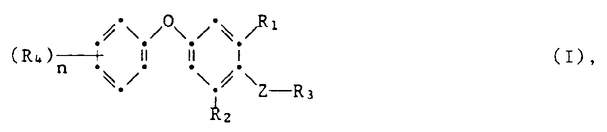
dans laquelle
R₁ représente un groupe cycloalkyle en C₃-C₇ ou cycloalcényle en C₅-C₆,
R₂ représente un groupe alkyle en C₁-C_{6,} cycloalkyle en C₅-C₆ ou cycloalcényle en C₅-C₆,
R₃ représente un groupe en C₁-C₈, cycloalkyle en C₃-C₆, 1-cyclopropyl-éthyle ou alcényle en C₃-C₆, chacun des symboles R₄ représente l'hydrogène, un halogène, un groupe alkyle en C₁-C₄, alcoxy en C₁-C₃ ou CF₃, n est un nombre allant de 1 à 3,
Z représente -NH-CS-NH-, -N=C(SR₅)-NH-, N=C=N-, et
R₅ représente un groupe alkyle en C₁-C₅ ou allyle, ou en l'un de ses sels d'acide organique ou minéral avec des véhicules et/ou additifs appropriés.

18. Produit pesticide selon revendication 17, contenant au moins un composant actif qui consiste en un composé de formule I dans laquelle R₁ représente un groupe cycloalkyle en C₃-C₇, R₂ un groupe alkyle en C₁-C₄ ou cyclopentyle, R₃ un groupe alkyle en C₁-C₄ ou cycloalkyle en C₃-C₅, R₄ l'hydrogène, un halogène ou un groupe alkyle en C₁-C_{3,} n est égal à 1 ou 2, Z représente -NH-CS-NH-, -N=C(SR₅)-NH- ou N=C=N- et R₅ un groupe alkyle en C₁-C₃.

19. Produit pesticide selon revendication 18, contenant au moins un composant actif consistant en un composé de formule I dans laquelle R₁ représente un groupe cycloalkyle en C₅-C₆, R₂ un groupe éthyle ou isopropyle, R₃ un groupe isopropyle, tert-butyle ou cyclopentyle, R₄ l'hydrogène ou le fluor; n est égal à 1 ou 2 et Z représente -NH-CS-NH-.

20. Produit pesticide selon revendication 18, contenant au moins un composant actif consistant en un composé de formule I dans laquelle R₁ représente un groupe cycloalkyle en C₅-C₆, R₂ un groupe éthyle ou isopropyle, R₃ un groupe isopropyle, tert-butyle ou cyclopentyle, R₄ l'hydrogène ou le fluor, n est égal à 1 ou 2, Z représente -N=C(SR₅)-NH- et R₅ un groupe méthyle ou éthyle.

21. Produit pesticide selon revendication 18, contenant au moins un composant actif consistant en un composé de formule I dans laquelle R₁ représente un groupe cycloalkyle en C₅-C₆, R₂ un groupe éthyle ou isopropyle, R₃ un groupe isopropyle, tert-butyle ou cyclopentyle, R₄ l'hydrogène ou le fluor, n est égal à 1 ou 2 et Z représente -N=C=N-.

22. Utilisation d'un composé de formule 1 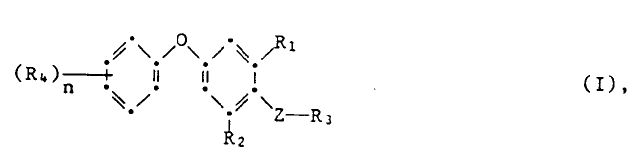
dans laquelle
R₁ représente un groupe cycloalkyle en C₃-C₇ ou cycloalcényle en C₅-C₆,
R₂ représente un groupe alkyle en C₁-C_{6,} cycloalkyle en C₅-C₆ ou cycloalcényle en C₅-C₆,
R₃ représente un groupe alkyle en C₁-C₈, cycloalkyle en C₃-C₆, 1-cyclopropyléthyle ou alcényle en C₃-C₅, chacun des symboles R₄ représente l'hydrogène, un halogène, un groupe alkyle en C₁-C₄, alcoxy en C₁-C₃ ou CF₃,
n est un nombre allant de 1 à 3,
Z représente -NH-CS-NH-, -N=C(SR₅)-NH-, -N=C=N-, et
R₅ représente un groupe alkyle en C₁-C₅ ou allyle, ou en l'un de ses sels d'acide organique ou minéral, pour la lutte contre les parasites des animaux et des végétaux.

23. Utilisation selon revendication 22 d'un composé de formule I pour combattre les insectes et les arachnides.

24. Procédé pour combattre les parasites des animaux et des végétaux, caractérisé en ce que l'on met les parasites, à un stade de développement quelconque, en contact avec un composé de formule I 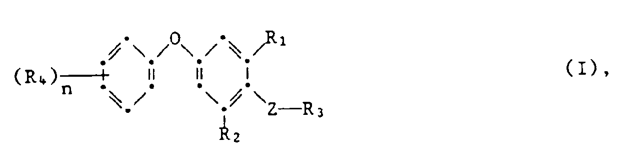
dans laquelle
R₁ représente un groupe cycloalkyle en C₃-C₇ ou cycloalcényle en C₅-C₆,
R₂ représente un groupe alkyle en C₁-C₆, cycloalkyle cycloalkyle en C₅-C₆ ou cycloalcényle en C₅-C₆,
R₃ représente un groupe alkyle en C₁-C_{8,} cycloalkyle en C₃-C₆, 1-cyclopropyléthyle ou alcényle en C₃-C₅, chacun des symboles R₄ représente l'hydrogène, un halogène, un groupe alkyle en C₁-C_{4,} alcoxy en C₁-C₃ ou CF₃, n est un nombre allant de 1 à 3,
Z représente -NH-CS-NH-, -NH=C(SR₅)-NH-, -N=C=N-, et
R₅ représente un groupe alkyle en C₁-C₅ ou allyle, ou l'un de ses sels d'acide organique ou minéral.
